(19) Europäisches Patentamt
European Patent Office
Office européen des brevets

(11) EP 4 596 676 A1

(12) # EUROPEAN PATENT APPLICATION
published in accordance with Art. 153(4) EPC

(43) Date of publication:
**06.08.2025 Bulletin 2025/32**

(21) Application number: **23871764.9**

(22) Date of filing: **05.09.2023**

(51) International Patent Classification (IPC):
***C12N 5/071*** *(2010.01)* ***B05C 1/02*** *(2006.01)*
***B05C 5/00*** *(2006.01)* ***B05C 11/10*** *(2006.01)*
***B05D 1/26*** *(2006.01)* ***B05D 1/36*** *(2006.01)*
***B05D 3/00*** *(2006.01)* ***B05D 7/00*** *(2006.01)*
***C12N 5/077*** *(2010.01)* ***C12N 5/10*** *(2006.01)*
***C12N 15/09*** *(2006.01)*

(52) Cooperative Patent Classification (CPC):
**B05C 1/02; B05C 5/00; B05C 11/10; B05D 1/26; B05D 1/36; B05D 3/00; B05D 7/00; C12N 5/06; C12N 5/10; C12N 15/09**

(86) International application number:
**PCT/JP2023/032319**

(87) International publication number:
**WO 2024/070524 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.09.2022 JP 2022153774**
**17.10.2022 JP 2022166456**
**23.12.2022 JP 2022206880**

(71) Applicant: **NTN Corporation**
**Osaka-shi Osaka 530-0005 (JP)**

(72) Inventors:
- **CHIKAE, Shohei**
**Iwata-shi, Shizuoka 438-8510 (JP)**
- **TSUKAMOTO, Yoshinari**
**Iwata-shi, Shizuoka 438-8510 (JP)**
- **ODA, Atsushi**
**Iwata-shi, Shizuoka 438-8510 (JP)**

(74) Representative: **Bockhorni & Brüntjen Partnerschaft**
**Patentanwälte mbB**
**Agnes-Bernauer-Straße 88**
**80687 München (DE)**

(54) **CELL TISSUE PRODUCTION METHOD, COATING METHOD AND COATING DEVICE**

(57) In a step of supplying a second application solution (BB) in a cell tissue production method, if a first dimension of each of N first application solutions (AA) is represented as $r_{1n}$ (n is a natural number of $1 \leq n \leq N$), a second dimension of each of M second application solutions is represented as $r_{2m}$ (m is a natural number of $1 \leq m \leq M$), and the distance between the center of any first application solution, a first dimension of which is $r_{ln}$, selected out of the N first application solutions and the center of any second application solution, a second dimension of which is $r_{2m}$, selected out of the M second application solutions is represented as $D_{n,m}$, the any first application solution (AA) and the any second application solution (BB) are supplied such that one of (Math. 1)

$$D_{n,m} < r_{2m} - r_{1n} \ldots (1)$$

and
(Math. 2)

$$D_{n,m} > r_{2m} + r_{1n} \ldots (2)$$

holds between the any first application solution (AA) and the any second application solution (BB).

EP 4 596 676 A1

FIG.8

(A)

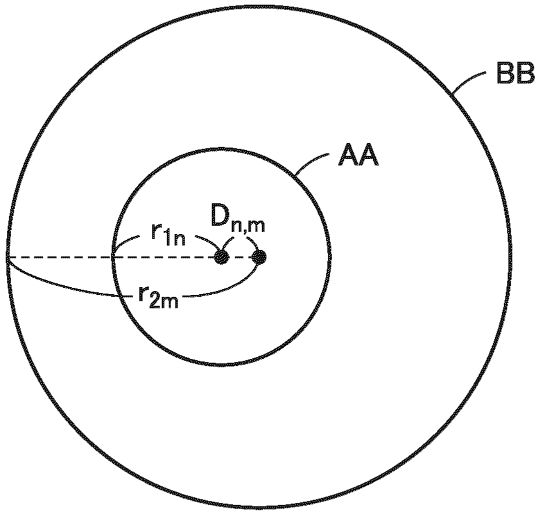


(B)

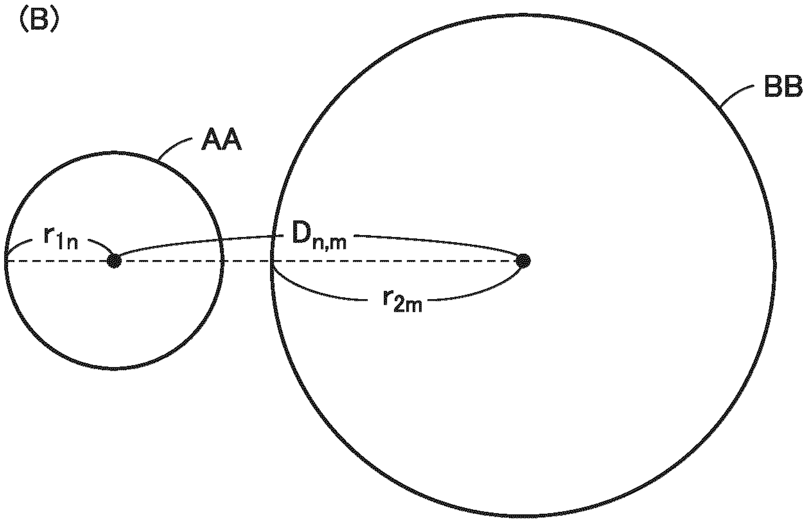

## Description

### TECHNICAL FIELD

The present disclosure relates to a cell tissue production method, an application method and an application device.

### BACKGROUND ART

A technique for culturing different kinds of cells and tissues in close contact in one container to form a cell and a tissue having polarity similar to the polarity of an organism is disclosed in, for example, Japanese Patent Laying-Open No. 2020-178612 (Patent Literature 1). Japanese Patent Laying-Open No. 2020-178612 discloses a method of forming a plurality of cell tissues in one container with an application needle scheme.

### CITATION LIST

### PATENT LITERATURE

PTL 1: Japanese Patent Laying-Open No. 2020-178612

### SUMMARY OF INVENTION

### TECHNICAL PROBLEM

In Japanese Patent Laying-Open No. 2020-178612, a combination of a well, which is an application target object, and a cell tissue serving as a first application solution supplied into the well is exemplified, for example, in paragraph 0096 and Fig. 17.

However, in Japanese Patent Laying-Open No. 2020-178612, a relation among three parameters including the radius of the first application solution containing a cell, the radius of a second application solution covering the first application solution, and the size of the well is not disclosed. If, in Fig. 17(a) of Japanese Patent Laying-Open No. 2020-178612, the second application solution is dripped in a position close to the inner wall of the well using a dispenser, in some cases, the second application solution adheres to the inner wall of the well with static electricity or deviates from a target position. As a result, it is likely that the second application solution cannot completely cover the first application solution and the first application solution collapses.

The present disclosure has been devised in view of the problems described above. An object of the present disclosure is to provide a cell tissue production method capable of highly reproducibly and highly accurately evaluating action with a small scale and a high throughput by supplying an application solution with high position accuracy while considering a dimension and a center position.

Another object of the present disclosure is to provide an application method and an application device that can further reduce a total time required for production of a cell tissue.

### SOLUTION TO PROBLEM

In a cell tissue production method according to the present disclosure, N (a natural number of N≥1) first application solutions are supplied into an application target object and a cell is disposed. M (a natural number of M≥1) second application solutions are supplied into the application target object to cover at least one among the N first application solutions. In a step of supplying the second application solutions, if a first dimension, which is a maximum value of a dimension from a center to an outer edge of each of the N first application solutions, is represented as $r_{1n}$ (n is a natural number of 1≤n≤N), a second dimension, which is a maximum value of a dimension from a center to an outer edge of each of the M second application solutions, is represented as $r_{2m}$ (m is a natural number of 1≤m≤M), and a distance between a center of any first application solution, a first dimension of which is $r_{1n}$, selected out of the N first application solutions and a center of any second application solution, a second dimension of which is $r_{2m}$, selected out of the M second application solutions is represented as $D_{n, m}$, the any first application solution and the any second application solution are supplied such that one of

(Math. 1)

$$D_{n, m} < r_{2m} - r_{1n} \quad \ldots (1)$$

and
(Math. 2)

$$D_{n,m} > r_{2m} + r_{1n} \ldots (2)$$

holds between the any first application solution and the any second application solution.

In an application method according to the present disclosure, a step of supplying, using a first application mechanism, a first application material serving as an application material to a first container included in a plate including a plurality of containers capable of receiving the application material is performed. A pre-simultaneous supply movement step in which a stage on which the plate is placed moves is performed. A simultaneous supply step of supplying, using the first application mechanism, the first application material to a second container disposed at an interval from the first container in the plate and, at the same time, supplying a second application material serving as an application material to the first container using a second application mechanism disposed at an interval from the first application mechanism is performed. Every time the pre-simultaneous supply movement step and the simultaneous supply step are performed, containers equivalent to the first container and the second container are changed in a moving direction of the stage.

An application device according to the present disclosure includes an application mechanism and a stage. The application mechanism is capable of supplying an application material that is to be applied. A plate to which the application material is to be supplied can be placed on the stage. The application mechanism includes a first application mechanism and a second application mechanism adjacent to the first application mechanism. An interval between the first application mechanism and the second application mechanism is equal to a movement amount of the stage between a first application time and a second application time by the application mechanism.

## ADVANTAGEOUS EFFECTS OF INVENTION

According to the present disclosure, it is possible to provide a cell tissue production method capable of highly reproducibly and highly accurately evaluating action with a small scale and a high throughput by supplying an application solution with high position accuracy while considering a dimension and a center position.

According to the present disclosure, it is possible to further reduce a total time required for production of a cell tissue.

## BRIEF DESCRIPTION OF DRAWINGS

Fig. 1 is a schematic diagram of an application device according to a first embodiment.
Fig. 2 is a schematic diagram illustrating an application mechanism of the application device illustrated in Fig. 1.
Fig. 3 is a schematic perspective view of a sample application set.
Fig. 4 is a schematic diagram illustrating the centers and the dimensions of application solutions supplied in steps of a cell tissue production method in the first embodiment.
Fig. 5 is a schematic diagram illustrating a disposition relation between two first application solutions.
Fig. 6 is a schematic diagram illustrating a relation between the centers, the dimensions, and the positions of the first application solution and a first virtual curve covering the first application solution.
Fig. 7 is a schematic diagram illustrating a relation between the first virtual curve and a second application solution.
Fig. 8 is a schematic diagram illustrating a first example of a positional relation between the first application solution and the second application solution.
Fig. 9 is a schematic diagram illustrating a second example of the positional relation between the first application solution and the second application solution.
Fig. 10 is a schematic diagram illustrating a disposition relation between two second application solution.
Fig. 11 is a schematic diagram illustrating a relation between the centers, the dimensions, and the positions of the second application solution and a second virtual curve covering the second application solution.
Fig. 12 is a schematic diagram illustrating a relation between the second virtual curve and a well.
Fig. 13 is a phase-contrast microscopic image in an example 1.
Fig. 14 is a phase-contrast microscopic image in an example 2.
Fig. 15 is a schematic plan view illustrating the positions and the dimensions of application solutions supplied in an example 3.
Fig. 16 is a schematic plan view illustrating the center positions and the distances between the centers of the same application solutions as the application solutions in Fig. 15.

Fig. 17 is a phase-contrast microscopic image in the example 3.

Fig. 18 is a phase-contrast microscopic image in an example 4.

Fig. 19 is a schematic diagram and a photograph illustrating a position where the second application solution is assumed to be applied and a position where the second application solution has been actually applied at the time when an application position in the well of the second application solution has been changed.

Fig. 20 is a schematic diagram illustrating a mode before a first application solution in a second embodiment is applied.

Fig. 21 is a schematic diagram illustrating a composition of the first application solution in the second embodiment.

Fig. 22 is a schematic diagram illustrating a step in which the first application solution in the second embodiment is applied.

Fig. 23 is a schematic diagram illustrating a mode after the first application solution in the second embodiment has been applied.

Fig. 24 is a schematic diagram illustrating a step in which a second application solution in the second embodiment is supplied.

Fig. 25 is a schematic diagram illustrating a mode in a well after the step in Fig. 24 has been performed.

Fig. 26 is a schematic diagram illustrating a step in which a culture medium in the second embodiment is supplied.

Fig. 27 is a schematic diagram illustrating a mode of a cell in the first application solution before culture.

Fig. 28 is a schematic diagram illustrating a mode of the cell in the first application solution after culture.

Fig. 29 is a phase-contrast microscopic image (left) immediately after an application solution is supplied into the well and a phase-contrast microscopic image (right) after culture for six days.

Fig. 30 is a graph illustrating a $Ca^{2+}$ transient waveform in an experiment of an application tissue.

Fig. 31 is a graph illustrating a change in a BPM at the time when isoproterenol is added to each of the application tissue and a 2D tissue.

Fig. 32 is a graph illustrating a change in an AMP at the time when isoproterenol is added to each of the application tissue and the 2D tissue.

Fig. 33 is a schematic front view illustrating a first example of an application device according to a third embodiment.

Fig. 34 is a schematic front view illustrating a second example of the application device according to the third embodiment.

Fig. 35 is a schematic diagram illustrating a needle application mechanism of the application device illustrated in Fig. 33.

Fig. 36 is a schematic perspective view of a plate.

Fig. 37 is a flowchart illustrating an application method in the third embodiment.

Fig. 38 is a schematic sectional view illustrating a part of an application mechanism and an installed plate of the application device used for the application method in the third embodiment.

Fig. 39 is a schematic sectional view illustrating a first step of the application method in the third embodiment.

Fig. 40 is a schematic sectional view illustrating a second step of the application method in the third embodiment.

Fig. 41 is a schematic sectional view illustrating a third step of the application method in the third embodiment.

Fig. 42 is a schematic sectional view illustrating a fourth step of the application method in the third embodiment.

Fig. 43 is a schematic diagram illustrating an example of a three-dimensional cell tissue production method.

Fig. 44 is a photograph of the three-dimensional cell tissue obtained by the production method in Fig. 43.

Fig. 45 is a schematic diagram illustrating a result of a cell tissue being produced in one container included in the plate.

Fig. 46 is a schematic diagram illustrating an example of a plurality of cell tissues formed in a single container as illustrated in (C) in Fig. 45.

Fig. 47 is an example of producing a cell tissue using an application needle having a diameter of 330 $\mu$m.

Fig. 48 is a schematic diagram for comparing timings when application of the application method in the third embodiment and application of an application method in a comparative example are performed.

Fig. 49 is a schematic front view illustrating an example of an application device according to a fourth embodiment.

Fig. 50 is a flowchart illustrating an application method in the fourth embodiment.

Fig. 51 is a schematic sectional view illustrating a part of an application mechanism and an installed plate of an application device used for the application method in the fourth embodiment.

Fig. 52 is a schematic sectional view illustrating a first step of the application method in the fourth embodiment.

Fig. 53 is a schematic sectional view illustrating a second step of the application method in the fourth embodiment.

Fig. 54 is a schematic sectional view illustrating a third step of the application method in the fourth embodiment.

Fig. 55 is a schematic sectional view illustrating a fourth step of the application method in the fourth embodiment.

Fig. 56 is a schematic sectional view illustrating a fifth step of the application method in the fourth embodiment.

Fig. 57 is a schematic diagram for comparing timings when application of the application method in the fourth embodiment and application of an application method in a comparative example are performed.

Fig. 58 is a flowchart illustrating an application method according to a first example of a fifth embodiment.

Fig. 59 is a schematic sectional view illustrating a part of an application mechanism and an installed plate of an application device used for the application method according to the first example of the fifth embodiment.
Fig. 60 is a schematic sectional view illustrating a first step of the application method according to the first example of the fifth embodiment.
Fig. 61 is a schematic sectional view illustrating a second step of the application method according to the first example of the fifth embodiment.
Fig. 62 is a schematic sectional view illustrating a third step of the application method according to the first example of the fifth embodiment.
Fig. 63 is a schematic sectional view illustrating a fourth step of the application method according to the first example of the fifth embodiment.
Fig. 64 is a flowchart illustrating another example of an application method according to a second example of the fifth embodiment.
Fig. 65 is a plan view illustrating a mode of an application material formed in one container in a sixth embodiment.
Fig. 66 is a schematic sectional view illustrating a first example of a part of an application mechanism and an installed plate of an application device used for an application method in a seventh embodiment.
Fig. 67 is a schematic sectional view illustrating a second example of the part of the application mechanism and the installed plate of the application device used for the application method in the seventh embodiment.
Fig. 68 is a schematic sectional view illustrating a third example of the part of the application mechanism and the installed plate of the application device used for the application method in the seventh embodiment.

## DESCRIPTION OF EMBODIMENTS

(Introduction)

First, characteristics of a cell tissue production method in a first embodiment is briefly explained. Referring to Fig. 8, as illustrated in (A), a distance $D_{n,m}$ between the center of a supplied first application solution AA having a first dimension $r_{1n}$ and the center of a second application solution BB having a second dimension $r_{2m}$ covering first application solution AA is
(Math. 1)

$$D_{n,m} < r_{2m} - r_{1n} \ldots (1).$$

Alternatively, as illustrated in (B),
(Math. 2)

$$D_{n,m} > r_{2m} + r_{1n} \ldots (2).$$

The cell tissue production method in the first embodiment is explained below with reference to the drawings.

(First embodiment)

(Device configuration)

Fig. 1 is a schematic diagram of an application device according to the first embodiment. The application device according to the present embodiment is explained with reference to Fig. 1. Note that, for convenience of explanation, an X direction, a Y direction, and a Z direction are introduced. Referring to Fig. 1, the application device according to the present embodiment mainly includes a processing chamber, a Y-axis table 2, an X-axis table 1, and a Z-axis table 3 disposed on the inside of the processing chamber, an application mechanism 4, an observation optical system 6, a CCD camera 7 connected to observation optical system 6, and a control unit. The control unit includes a monitor 9, a control computer 10, and an operation panel 8.
On the inside of the processing chamber, Y-axis table 2 is installed on the bottom of the processing chamber. Y-axis table 2 is movable in the Y-axis direction. Specifically, a guide section is installed on the lower surface of Y-axis table 2. The guide section is slidably connected to a guide rail installed on the bottom surface of the processing chamber. A ball screw is connected to the lower surface of Y-axis table 2. The ball screw is caused to operate by a driving member such as a motor, whereby Y-axis table 2 is movable (in the Y-axis direction) along the guide rail. An upper surface of Y-axis table 2 is formed as a mounting surface for mounting a sample application set 11 explained below. That is, Y-axis table 2 has a function of a holding table that holds sample application set 11 that is a target object to which a liquid material is applied.
X-axis table 1 is installed on Y-axis table 2. X-axis table 1 is disposed on a structure installed to straddle Y-axis table 2 in the X-axis direction. On X-axis table 1, a mobile body to which Z-axis table 3 is connected is installed to be movable in the X-axis direction. The mobile body is capable of moving in the X-axis direction using, for example, a ball screw. Note that X-axis table 1 is fixed to the bottom surface of the processing chamber via the structure described above. For that reason, Y-axis table 2 explained above is movable in the Y-axis direction with respect to X-axis table 1.

As explained above, Z-axis table 3 is installed on the mobile body connected to X-axis table 1. Observation optical system 6 and application mechanism 4 are connected to Z-axis table 3. Observation optical system 6 is an optical system for observing an application position of sample application set 11 that is a target object to be applied. CCD camera 7 converts an observed image into an electric signal. Z-axis table 3 holds observation optical system 6 and application mechanism 4 to be movable in the Z-axis direction.

Control computer 10 and operation panel 8 for controlling Y-axis table 2, X-axis table 1, Z-axis table 3, observation optical system 6, and application mechanism 4 and monitor 9 incidental to the control computer are installed on the outside of the processing chamber. Monitor 9 displays image data converted by CCD camera 7 explained above and output data from control computer 10. Operation panel 8 is used to input a command to control computer 10.

Fig. 2 is a schematic diagram illustrating the application mechanism of the application device illustrated in Fig. 1. Referring to Fig. 2, application mechanism 4 in the present embodiment mainly includes a servomotor 41, a cam 43, a bearing 44 held in a state of being in contact with a cam surface of cam 43, a cam coupling plate 45, a movable unit 46, a movable base 35 that holds an application needle holder 20, and an application material container 21. Application needle holder 20 is detachably attachable to movable base 35. In other words, movable base 35 serving as a base body detachably holds application needle holder 20.

In application mechanism 4, servomotor 41 is installed such that the center axis extends in a direction along the Z-axis direction illustrated in Fig. 1. Cam 43 is connected to a rotating shaft of servomotor 41. Cam 43 is capable of rotating centered on the center axis of servomotor 41. Cam 43 includes a center portion connected to the rotating shaft of servomotor 41 and a flange section connected to one end portion of the center portion. The upper surface (the surface on servomotor 41 side) of the flange section is formed as a cam surface. The cam surface is annularly formed along the outer circumference of the center portion and is formed in a slope shape such that the distance from the bottom surface of the flange section fluctuates. Specifically, the cam surface includes an (thick) upper end flat region where the distance from the bottom surface of the cam surface is the longest, a lower end flat region disposed at an interval from the upper end flat region, and a slope section that smoothly connects the upper end flat region and the lower end flat region. The lower end flat region is a (thin) region where the distance from the bottom surface is the shortest.

Bearing 44 is disposed to be in contact with the cam surface of cam 43. Cam coupling plate 45 is connected to bearing 44. In cam coupling plate 45, the other end portion on the side opposite to one end portion connected to bearing 44 is fixed to movable unit 46. Movable base 35 serving as the base body is connected to movable unit 46. Application needle holder 20 is installed in movable base 35. Application needle holder 20 includes an application needle 24. Application needle 24 is capable of applying a liquid material to, for example, a well 12 of sample application set 11. Application needle 24 is disposed to protrude from application needle holder 20 on the lower surface (the lower side on the side opposite to the side where servomotor 41 is located) of application needle holder 20. Application material container 21 is disposed under application needle holder 20. Application needle 24 is held in an inserted state into application material container 21.

A fixed pin is fixed to movable unit 46. The other fixed pin is fixed to a stand that holds servomotor 41. A spring is installed to connect the fixed pins. With the spring, movable unit 46 is in a state of receiving force directed to application material container 21 side. With the force of the spring, bearing 44 maintains a state of being pressed against the cam surface of cam 43.

Movable unit 46 and movable base 35 are connected to a linear guide installed in the stand that holds servomotor 41 and are movable in the Z-axis direction.

In application mechanism 4 explained above, the rotating shaft of servomotor 41 is rotated to rotate cam 43 by driving servomotor 41. As a result, the position in the Z-axis direction of bearing 44 in contact with the cam surface of cam 43 fluctuates according to the rotation of the rotating shaft of servomotor 41. Then, movable unit 46 and movable base 35 move in the Z-axis direction according to the position fluctuation in the Z-axis direction of bearing 44, whereby the position in the Z-axis direction of application needle 24 can be changed. That is, application needle 24 can be reciprocated in the Z-axis direction. According to this operation, when application needle 24 is present upward in the Z-axis direction, the distal end of application needle 24 is immersed in application material container 21 in which the liquid material is stored. In this state, application needle 24 protrudes downward from a distal end hole on the bottom surface of application material container 21, whereby an application operation is performed. In a state in which the liquid material adheres to the distal end of application needle 24, the distal end of application needle 24 protrudes from the distal end hole of application material container 21 and moves out of application material container 21. At this time, the liquid material is lifted upward by surface tension and a substantially fixed amount of the liquid material adheres to the distal end of application needle 24. The liquid material adhering in this way is transferred to sample application set 11, whereby a highly reproducible application step can be implemented.

More specifically, referring to Fig. 1 and Fig. 2, an application speed command value output from operation panel 8 is stored in a storage device of control computer 10. At an application operation time, the application speed command value read from the storage device is transmitted to a control program for application mechanism 4. The control program for application mechanism 4 determines rotating speed of servomotor 41 based on the application speed command value and rotates servomotor 41. Accordingly, the application operation is performed while application needle 24 is reciprocated in the Z-axis direction. When control computer 10 is communicating with a not-illustrated host control system, the application speed command value may be transmitted from the host control system to the control program for application mechanism 4. A parameter corresponding to a type of a liquid material to be applied may be stored in the storage device of control computer 10. The application speed command value may be calculated according to a type, an application amount, and an application dimension of a designated liquid material.

(Characteristics of a production method)

Fig. 3 is a schematic perspective view of the sample application set. Referring to Fig. 3, in the present embodiment, an application solution serving as a liquid material is applied and supplied to the inside of a plurality of wells 12 formed in sample application set 11. However, an application target object to which the application solution is supplied is not limited to this. Sample application set 11 has thickness in the Z-axis direction. Plurality of wells 12 are formed on the top surface of sample application set 11. Plurality of wells 12 are concave portions where the upper surface of sample application set 11 is hollowed. As plurality of wells 12, for example, ninety-six wells 12 in total may be formed in eight rows in the Y-axis direction and twelve rows in the X-axis direction in Fig. 3 at an interval from one another. A plane shape of wells 12 is any shape such as a circular shape.

Fig. 4 is a schematic diagram illustrating the centers and the dimensions of application solutions supplied in steps of a cell tissue production method in the first embodiment. For an application solution in Fig. 4 and the subsequent figures, a shape in plan view from the Z direction is illustrated. Referring to Fig. 4, in the present embodiment, it is preferable that, for example, an application solution circular in plan view is supplied. However, not only this, but the application solution may have a shape slightly deformed from a perfect circular shape or may have an elliptical plane shape greatly deformed slight to one side. Further, the application solution may have a polygonal plane shape such as an octagonal shape or a dodecagonal shape (or a plane shape similar to these polygonal shapes) different from both of the circular shape and the elliptical shape. In any case, it is assumed that the dimension of the application solution explained below means a maximum value of the dimension from the center (the centroid) to the outer edge. If the plane shape of the application solution is the circular shape, the dimension means a radius $r_{1n}$ in (A) in Fig. 4. If the plane shape is the elliptical shape, the dimension means a semimajor radius $r_{1n}$ in (A) in Fig. 4.

First, N first application solutions are supplied to one well 12 selected out of plurality of wells 12 of sample application set 11. N is a natural number of 1 or more. At least one first application solution AA illustrated in (A) is supplied. Note that a plurality of first application solutions AA as liquid temporally continuously supplied by one application mechanism is assumed to be one application solution. First application solutions AA as liquid supplied while being temporally divided by one application mechanism are assumed to be application solutions separate from one another. First application solutions AA as liquid supplied temporarily simultaneously but supplied from application mechanisms different from one another are assumed to be application solutions separate from one another. When plurality of first application solutions AA are supplied, a cell that is to be cultured is contained in at least one of plurality of first application solutions AA. For this reason, the cell that is to be cultured is disposed in well 12 by the supply of first application solutions AA.

The dimension of first application solution AA is represented as a first dimension $r_{1n}$ ($1 \leq n \leq N$). That is, a first dimension of first one of N first application solutions AA is denoted by $r_{11}$ and a first dimension of second one of N first application solutions AA is denoted by $r_{12}$.

Fig. 5 is a schematic diagram illustrating a disposition relation between two first application solutions. Referring to Fig. 5, the respective dimensions of optionally selected two first application solutions AA among N (N is a natural number of 2 or more) first application solutions AA are represented as $r_{1n}$ and $r_{1(n+1)}$. The distances between the centers (centroids) of respective two first application solutions AA are represented as $d_{n,\, n+1}$. At this time, as the disposition relation between the two first application solutions, there are a case in which the two first application solutions do not overlap each other as illustrated in (A) in Fig. 5, a case in which one is completely fit on the inside of the other as illustrated in (B), and a case in which both the first application solutions partially overlap as illustrated in (C). (A) includes a case in which both the first application solutions are circumscribed with each other. (B) includes a case in which one is inscribed with the other.

Expression (3) described below holds in the case of (A) in Fig. 5, Expression (4) described below holds in the case of (B), and Expression (5) described below holds in the case of (C). An equal sign in Expression (3) indicates a case in which two first application solutions AA are circumscribed with each other in (A) in Fig. 5. An equal sign in Expression (4) indicates a case in which one first application solution AA is inscribed with the other in (B) in Fig. 5. If $r_{1n}=r_{1(n+1)}$ in Expression (5), only $d_{n,\, n+1}<r_{1n}+r_{1(n+1)}$ holds.
(Math. 3)

$$d_{n,\,n+1} \geq r_{1n} + r_{1(n+1)} \ \ldots\ (3)$$

(Math. 4)

$$d_{n,\,n+1} \leq r_{1(n+1)} - r_{1n} \ \ldots\ (4)$$

(Math. 5)

$$r_{1(n+1)} - r_{1n} < d_{n,\,n+1} < r_{1n} + r_{1(n+1)} \ \ldots\ (5)$$

M (a natural number of M≥1) second application solutions BB (see (C) in Fig. 4) are supplied into well 12 to cover at least one of N first application solutions AA from the upper side in the Z-axis direction. That is, for example, one of a plurality of second application solutions BB may cover a part of plurality of first application solutions AA and another one of second application solutions BB may cover another part of plurality of first application solutions AA. Fig. 6 is a schematic diagram illustrating a relation between the centers, the dimensions, and the positions of a first application solution and a first virtual curve covering the first application solution. Referring to Fig. 6 and (B) in Fig. 4, a first virtual curve A' is a virtual figure (for example, a circle) used to determine a dimension of one second application solution BB overlapping first application solution AA. It is also possible that a virtual curve is not a curve (is a straight line). However, in the following explanation, the virtual curve including that case is referred to as virtual curve. One or more first application solutions AA overlapping one second application solution BB among N first application solutions AA are selected. The centroid of first application solutions AA is calculated as a first centroid G1 of first virtual curve A' from the centers (centroids) of respective one or more first application solutions AA.

For example, when one second application solutions BB covering all of N first application solutions AA is supplied, as illustrated in Fig. 6, the centers of respective N first application solutions AA are conceived. The position of the center of one first application solution AA determines the centroids of the respective first application solutions AA. The centroid means a point where an application solution is balanced by setting the position of the centroid as a fulcrum. For example, in first application solution AA on which weight uniformly acts such as a circular or elliptical application solution, the centroid of first application solution AA is equal to the center.

A position coordinate of the center of a 1-st first application solution AA is represented as $(x_1, y_1)$ and, similarly, the center of an N-th first application solution AA is represented as $(x_N, y_N)$. The position of the center (a coordinate of the center) is equal to a coordinate of the centroid of first application solution AA when first application solution AA is circular or elliptical. The coordinate of the center is calculated from an output of monitor 9 included in the control unit of the application device. At this time, when N=3, by dividing coordinates of center points by 3, a coordinate $(X_1, Y_1)$ of first centroid G1 is obtained as a centroid of a triangle formed by connecting the coordinates of the center points. When N≥4, the coordinate $(X_1, Y_1)$ is obtained by dividing an N-polygon formed by connecting the centers into (N-2) triangles, totaling areas $s_n$ of the respective triangles multiplied by centroid coordinates $gx_n$ $(gy_n)$ of the triangles, and dividing the total by a total area of the N-polygon. That is,

[Math. 6]

$$X_1 = \frac{\sum_{n=1}^{N-2} s_n gx_n}{\sum_{n=1}^{N-2} s_n} \ \cdot\cdot\cdot\ (6)$$

[Math. 7]

$$Y_1 = \frac{\sum_{n=1}^{N-2} s_n gy_n}{\sum_{n=1}^{N-2} s_n} \ \cdot\cdot\cdot\ (7)$$

A first virtual dimension $r_{1v}$ of first virtual curve A' is calculated to cover entire first application solution AA. First virtual dimension $r_{1v}$ is calculated as a dimension (of a circle) of first virtual curve A' at the time when n=3 or n=N first application solutions AA separated from first centroid G1 most as illustrated in Fig. 6 is inscribed with the circle of first virtual curve A'. That is, first virtual curve A' is present on the outermost side when viewed from first centroid G1 among plurality of first application solutions AA and is formed to be larger in a dimension (generate a maximum dimension) by being inscribed with first application solution AA on the outermost side than being inscribed with the other first application solutions AA. That is, relation of

(Math. 8)

$$r_{1v} \geq r_{1n} \ldots (8)$$

holds between dimension $r_{1n}$ of the first application solution and first virtual dimension $r_{1v}$ of first virtual curve A'. In particular, when the number of first application solutions AA is one, Expression (8) described above is an equation.

A distance L1 between first centroid G1 and the center of a n=3 (third) first application solution AA (inscribed with first virtual curve A') having a dimension $r_{13}$ is

(Math. 9)

$$L1 = r_{1v} - r_{13} \ldots (9).$$

Note that, as illustrated in Fig. 6, a disposition state among plurality of first application solutions AA in a single first virtual curve A' may be any mode of (A), (B), and (C) in Fig. 5. That is, a plurality of first application solutions AA covered by second application solution BB (explained below) may be separated from one another, one may be fit on the inside of the other, or plurality of first application solutions AA may be disposed to partially intersect.

Second dimension $r_{2m}$ of second application solution BB is larger than first virtual dimension $r_{1v}$ of first virtual curve A' formed as explained above. As explained above, the second dimension of second application solution BB means the maximum value of the dimension from the center (the centroid) to the outer edge. This is illustrated in Fig. 7. Fig. 7 is a schematic diagram illustrating a relation between the first virtual curve and the second application solution. Referring to Fig. 7 and (C) in Fig. 4, second application solution BB is supplied to cover entire first virtual curve A'. Second application solution BB is supplied to entirely overlap second application solution BB without being inscribed with first virtual curve A'. For this reason, if the dimension of an m-th second application solution BB selected out of M second application solutions BB is represented as $r_{2m}(1 \leq m \leq M)$, naturally,

(Math. 10)

$$r_{2m} > r_{1v} \ldots (10)$$

holds. In Fig. 7, a distance L2 between first centroid G1 and the center of second application solution BB having dimension $r_{2m}$ is represented by

(Math. 11)

$$L2 < r_{2m} - r_{1v} \ldots (11).$$

Note that a dimension $r_{1v}' = \alpha \times r_{1v}$ ($\alpha > 1.0$) larger than first virtual dimension $r_{1v}$ may be set as a reference dimension. Second dimension $r_{2m}$ of second application solution BB may be determined to be larger than reference dimension $r_{1v}'$. $\alpha$ is preferably 1.1 or more. If $\alpha$ is increased, a gap between the outer edges of first application solution AA and second application solution BB can be increased. If all of this fact and the fact that second dimension $r_{2m}$ is determined to be larger than first virtual dimension $r_{1v}$ ($\alpha$=1.0 described above) are summarized, second dimension $r_{2m}$ is larger than reference dimension $r_{1v}'$ equal to or larger than first virtual dimension $r_{1v}$.

Fig. 8 is a schematic diagram illustrating a first example of a positional relation between a first application solution and a second application solution. Referring to Fig. 8, for example, as illustrated in (A), first application solution AA may be covered by second application solution BB to be completely fit on the inner side of second application solution BB without being inscribed with second application solution BB. Alternatively, as illustrated in (B), first application solution AA may completely deviate to the outer side of second application solution BB without being circumscribed with and overlapping second application solution BB. In the case of (B), first application solution AA is completely covered not to be inscribed with not-illustrated another second application solution present on the left side of illustrated second application solution BB.

Both of first application solution AA and second application solution BB in the figures in Fig. 8 are any application solutions selected out of one or more application solutions. For this reason, if the distance between the center of n-th first application solution AA and the center of the m-th second application solution is represented as $D_{n,m}$, in both the cases of (A) and (B), naturally,

(Math. 12)

$$r_{2m} > r_{1n} \ldots (12)$$

holds. Then, in the case of (A),
(Math. 13)

$$D_{n,\ m} < r_{2m} - r_{1n} \ldots (1)$$

holds. In the case of (B),
(Math. 14)

$$D_{n,\ m} > r_{2m} + r_{1n} \ldots (2)$$

holds. Second application solution BB is supplied such that one of Expressions (1) and (2) described above holds.

Fig. 9 is a schematic diagram illustrating a second example of the positional relation between the first application solution and the second application solution. Referring to Fig. 9, in the present embodiment, first application solution AA only has to be disposed not to be inscribed and circumscribed with second application solution BB. In Fig. 9, both of a first application solution A1o on the left side and a first application solution A2o on the right side are not inscribed and circumscribed with both of second application solutions B1o and B2o. For this reason, as illustrated in Fig. 9, second application solution B1o and second application solution B2o may partially overlap. In the case of Fig. 9, Expression (1) holds between first application solution A1o and second application solution B1o and Expression (2) holds between first application solution A1o and second application solution B2o. Expression (1) holds between first application solution A2o and second application solution B2o and Expression (2) holds between first application solution A2o and second application solution B1o.

Fig. 10 is a schematic diagram illustrating a disposition relation between two second application solutions. Referring to Fig. 10, the dimensions of respective optionally selected two second application solutions BB among M (M is a natural number of 2 or more) second application solutions BB are represented as $r_{2m}$ and $r_{2(m+1)}$. The distance between the centers (centroids) of respective two second application solutions BB is represented as $p_{m,\ m+1}$. At this time, as the disposition relation between the two second application solutions, there are a case in which the two second application solutions do not overlap each other as illustrated in (A) in Fig. 10, a case in which one is completely fit on the inside of the other as illustrated in (B), and a case in which both of the two second application solutions partially overlap as illustrated in (C). (A) includes a case in which both of the two second application solutions are circumscribed with each other. (B) includes a case in which one is inscribed with the other.

Expression (13) described below holds in the case of (A) in Fig. 10, Expression (14) described below holds in the case of (B), and Expression (15) described below holds in the case of (C). An equal sign in Expression (13) indicates a case in which two second application solutions BB are circumscribed with each other in (A) in Fig. 10. An equal sign in Expression (14) indicates a case in which one second application solution BB is inscribed with the other second application solution BB in (B) in Fig. 10. If $r_{2m}=r_{2(m+1)}$ in Expression (15), only $p_{m,\ m+1}<r_{2m}+r_{2(m+1)}$ holds.
(Math. 15)

$$p_{m,\ m+1} \geq r_{2m} + r_{2(m+1)} \ldots (13)$$

(Math. 16)

$$p_{m,\ m+1} \leq r_{2(m+1)} - r_{2m} \ldots (14)$$

(Math. 17)

$$r_{2(m+1)} - r_{2m} < p_{m,\ m+1} < r_{2m} + r_{2(m+1)} \ldots (15)$$

Subsequently, a relation between second application solutions BB and the dimension of well 12 to which second application solutions BB are supplied is conceived. All of second application solutions BB are supplied into the same (single) well 12. Fig. 11 is a schematic diagram illustrating a relation between the centers, the dimensions, and the positions of a second application solution and a second virtual curve covering the second application solution. Referring to Fig. 11 and (D) in Fig. 4, a second virtual curve B' is a virtual figure (for example, a circle) used to determine a dimension of well 12 to which second application solution BB is to be supplied. From the centers (centroids) of respective M second application solutions B supplied into well 12, the centroid of all M second application solutions BB is calculated as a second centroid G2 of second virtual curve B'.

For example, as illustrated in Fig. 11, M second application solutions BB are respectively represented as m=1, 2, ..., and M. Note that a method of measuring the center position of second application solution BB is the same as the method of measuring the center position of first application solution AA.

A position coordinate of the center of a 1-st second application solution BB is represented as $(x_1, y_1)$ and, similarly, the center of an M-th second application solution BB is represented as $(x_M, y_M)$. The center positions (the coordinates of the centers) are equal to a coordinate of the centroid of second application solutions BB when second application solutions BB are circular or elliptical. The coordinates of the centers are calculated from, for example, an output of monitor 9 included in the control unit of the application device. At this time, when M=3, by dividing coordinates of center points by 3, a coordinate $(X_2, Y_2)$ of second centroid G2 is obtained as a centroid of a triangle formed by connecting the coordinates of the center points. When M≥4, the coordinate $(X_2, Y_2)$ is obtained by dividing an M-polygon formed by connecting the centers into (M-2) triangles, totaling areas $s_m$ of the respective triangles multiplied by centroid coordinates $gx_m$ $(gy_m)$ of the triangles, and dividing the total by a total area of the M-polygon. That is,

[Math. 18]

$$X_2 = \frac{\sum_{m=1}^{M-2} s_m g x_m}{\sum_{m=1}^{M-2} s_m} \cdots (16)$$

[Math. 19]

$$Y_2 = \frac{\sum_{m=1}^{M-2} s_m g y_m}{\sum_{m=1}^{M-2} s_m} \cdots (17)$$

A second virtual dimension $r_{2v}$ of second virtual curve B' is calculated to cover all of M second application solutions BB. Second virtual dimension $r_{2v}$ is calculated as a dimension (of a circle) of second virtual curve B' at the time when m=3 or m=M second application solutions BB separated from second centroid G2 most as illustrated in Fig. 11 is inscribed with the circle of second virtual curve B'. That is, second virtual curve B' is present on the outermost side when viewed from second centroid G2 among plurality of second application solutions BB and is formed to be larger (generate a maximum dimension) by being inscribed with second application solution BB on the outermost side than being inscribed with the other second application solutions BB. That is, a relation of

(Math. 20)

$$r_{2v} \geq r_{2m} \ldots (18)$$

holds between dimension $r_{2m}$ of the second application solution and second virtual dimension $r_{2v}$ of second virtual curve B'. In particular, when the number of second application solutions BB is one, Expression (18) described above is an equation.

A distance L2 between second centroid G2 and the center of an m=3 (third) second application solution BB (inscribed with second virtual curve B') having a dimension $r_{23}$ is

(Math. 21)

$$L2 = r_{2v} - r_{23} \ldots (19).$$

Note that, as illustrated in Fig. 11, a disposition state among plurality of second application solutions BB in second virtual curve B' may be any mode of (A), (B), and (C) in Fig. 10. That is, a plurality of second application solutions BB supplied into well 12 (explained below) may be separated from one another, one may be fit on the inside of the other, or plurality of second application solutions BB may be disposed to partially intersect.

A third dimension $r_3$ of well 12 is larger than second virtual dimension $r_{2v}$ of second virtual curve B' formed as explained above. That is illustrated in Fig. 12. Fig. 12 is a schematic diagram illustrating a relation between the second virtual curve and the well. Referring to Fig. 12 and (E) in Fig. 4, well 12 stores second virtual curve B' without being inscribed. For this reason, if the dimension of well 12 is represented as $r_3$, naturally,

(Math. 22)

$$r_3 > r_{2v} \ldots (20)$$

holds. In Fig. 12, a distance L3 between second centroid G2 and the center of well 12 having dimension $r_3$ is represented by

(Math. 23)

$$L3<r_3-r_{2v} \ldots (21).$$

Note that a dimension $r_{2v}'=\beta\times r_{2v}$ ($\beta>1.0$) larger than second virtual dimension $r_{2v}$ may be set as a reference dimension. Third dimension $r_3$ of well 12 may be larger than reference dimension $r_{2v}'$. $\beta$ is preferably 1.1 or more. If $\beta$ is increased, a gap between the outer edge of second application solution BB and the inner wall surface of well 12 can be increased. If all of this fact and the fact that third dimension $r_3$ is larger than second virtual dimension $r_{2v}$ ($\beta$=1.0 described above) are summarized, third dimension $r_3$ is larger than reference dimension $r_{2v}'$ equal to or larger than second virtual dimension $r_{2v}$.

(Action effects)

In the cell tissue production method according to the present embodiment, N (a natural number of N≥1) first application solutions AA are supplied into well 12 serving as the application target object and a cell is disposed. The M (a natural number of M≥1) second application solutions BB are supplied into well 12 to cover at least one of N first application solutions AA. In a step of supplying second application solutions BB, if a first dimension that is a maximum value of the dimensions from the centers to the outer edges of respective N first application solutions AA is represented as $r_{1n}$ (a natural number of 1≤n≤N), a second dimension that is a maximum value of the dimensions from the centers to the outer edges of respective M second application solutions BB is represented as $r_{2m}$ (a natural number of 1≤m≤M), and the distance between the center of any first application solution AA, a first dimension of which is $r_{1n}$, selected out of N first application solution AA and the center of any second application solution BB, a second dimension of which is $r_{2m}$, selected out of M second application solutions BB, is represented as $D_{n,m}$, second application solutions BB are supplied such that one of (Math. 24)

$$D_{n,m}<r_{2m}-r_{1n} \ldots (1)$$

and
(Math. 25)

$$D_{n,m}>r_{2m}+r_{1n} \ldots (2)$$

holds between any first application solution AA and any second application solution BB.

If Expression (1) holds, as illustrated in (A) in Fig. 8, first application solution AA is stored without being inscribed with second application solution BB. For this reason, it is possible to suppress collapse of first application solution AA at the time when second application solution BB is dripped on first application solution AA. If Expression (2) holds, as illustrated in (B) in Fig. 8, since first application solution AA does not come into contact with second application solution BB at all, it is possible to suppress collapse of first application solution AA at the time when second application solution BB is dripped on first application solution AA. For this reason, by supplying an application solution with high position accuracy while considering a dimension and a center position, it is possible to provide a cell tissue production method capable of highly reproducibly and highly accurately evaluate action with a small scale and a high throughput.

In the cell tissue production method, second dimension $r_{2m}$ is larger than the reference dimension equal to or larger than first virtual dimension $r_{1v}$ that is the maximum dimension of first virtual curve A' calculated to be centered on first centroid G1 of one or more first application solutions AA overlapping second application solution BB among N first application solutions AA, to cover all of one or more first application solutions AA, and to be inscribed with any one of one or more first application solutions AA. First centroid G1 is calculated from center positions of respective one or more first application solutions AA overlapping second application solution BB.

Since the maximum dimension at which any first application solution AA and first virtual curve A' can be inscribed is first virtual dimension $r_{1v}$, if second dimension $r_{2m}$ is set larger than first virtual dimension $r_{1v}$, second application solution BB overlapping first application solution AA is not inscribed with first application solution AA. For this reason, it is possible to suppress collapse of first application solution AA at the time when second application solution BB is dripped on first application solution AA.

In the cell tissue production method explained above, third dimension $r_3$ of well 12 is larger than the reference dimension equal to or larger than second virtual dimension $r_{2v}$ that is the maximum dimension of second vertical curve B' calculated to be centered on second centroid G2 of M second application solutions BB, to cover all of M second application solutions BB, and to be inscribed with any one of the M second application solutions. Second centroid G2 is calculated from the center positions of respective M second application solutions BB.

Since the maximum dimension at which any second application solution BB and second virtual curve B' can be inscribed is second virtual dimension $r_{2v}$, if third dimension $r_3$ is set larger than second virtual dimension $r_{2v}$, second

application solution BB does not adhere to the inner wall surface of well 12. For this reason, it is possible to suppress collapse of first application solution AA at the time when second application solution BB is dripped on first application solution AA.

(Modification of the application solution supply method)

In the cell tissue production method in the present embodiment, when the application step (the supply of the application solution) explained above is performed using the application device in Fig. 1 and Fig. 2, an application needle scheme is used in the step of supplying first application solution AA. Accordingly, it is possible to apply first application solution AA in well 12 by a very small amount, that is, to have an extremely small dimension. With the application needle scheme, it is possible to apply first application solution AA even if first application solution AA has high viscosity.

In the cell tissue production method explained above, in the step of supplying second application solution BB, any one selected out of a group consisting of an application needle scheme, an inkjet scheme, a dispenser scheme, a laser print scheme, and a pipet scheme may be used. When the viscosity of second application solution BB is high (second application solution BB is a high viscosity solution), the application needle scheme may be used. However, the inkjet scheme, the dispenser scheme, the laser print scheme, or the pipet scheme other than the application needle scheme is used, whereby second application solution BB is supplied onto first application solution AA by being dripped. For this reason, it is possible to suppress a deficiency of, for example, first application solution AA being mixed in and contaminating the inside of the application material container that stores second application solution BB of the application device including the application needle.

In the cell tissue production method explained above, in the step of supplying M second application solutions BB, at least one second application solution BB among M second application solutions BB may be supplied by a first scheme selected out of a group consisting of the application needle scheme, the inkjet scheme, the dispenser scheme, the laser print scheme, and the pipet scheme and another second application solution BB different from at least the one among M second application solutions BB may be supplied by a second scheme different from the first scheme. That is, first application solution AA may be supplied by the application needle scheme and second application solution BB may be supplied by the inkjet scheme. Alternatively, first application solution AA may be supplied by the application needle scheme and second application solution BB may be supplied by the dispenser scheme. By using a preferred scheme as appropriate according to characteristics and order of an application solution to be applied, it is possible to improve the efficiency of the application step and the quality of an application solution to be formed.

Note that, when the scheme other than the application needle scheme is used, it is preferable that a device for the inkjet scheme, a device for the dispenser scheme, or the like corresponding to the scheme is combined with the application device in Fig. 1 and Fig. 2. Alternatively, for example, when first application solution AA is supplied by the application needle scheme and second application solution BB is supplied by the dispenser scheme, first application solution AA may be supplied by the application device in Fig. 1 and Fig. 2 and second application solution BB may be supplied by an application device for the dispenser scheme different from the application device for first application solution AA. Further, for example, the application device in Fig. 1 and Fig. 2 includes only the single application mechanism 4, an application device including a plurality of application mechanisms 4 may be used, whereby all of a plurality of application solutions may be supplied by different application mechanisms 4 in a single application device.

(Cell tissue)

A first example of a cell tissue to be formed is as explained below. First application solution AA contains a cell that is to be cultured and a gelling agent and second application solution BB is a high viscosity solution. The high viscosity solution means a solution having viscosity higher than the viscosity of the gelling agent. Accordingly, an effect that firmness of the first application solution is improved when second application solution BB and a culture medium are added into well 12 can be obtained. Further, an effect that a cell tissue containing a collagen gel and a scaffold can be constructed as the cell tissue can be obtained. Specifically, an application solution obtained by mixing a cell and a collagen solution serving as the gelling agent is prepared as first application solution AA and supplied into well 12. Methylcellulose having viscosity higher than the viscosity of the collagen solution is used as second application solution BB and supplied to cover first application solution AA. Further, the cell tissue is formed by dripping the culture medium onto first application solution AA and second application solution BB in well 12. In the cell tissue, first application solution AA and second application solution BB are immersed in the culture medium.

The cell tissue described above is cultured, for example, under an environment of 37°C and 5% $CO_2$. Accordingly, the collagen solution is gelled and the application tissue is fixed in well 12. The high viscosity solution serving as second application solution BB can be removed by performing culture medium exchange.

A second example of the cell tissue to be formed is as explained below. First application solution AA includes a cell that is to be cultured and second application solution BB includes a gelling agent. Accordingly, an effect that the gelling agent is gelled on the interface between first application solution AA and second application solution BB and a scaffold-free cell tissue can be formed in a gel dome can be obtained. Specifically, as first application solution AA, the cell, thrombin that is a gelation initiator, and sodium hyaluronate that is a high viscosity polymer are mixed. Second application solution BB containing fibrinogen that is a gelling agent is dripped to cover first application solution AA. Accordingly, the interface between first application solution AA and second application solution BB is gelled and a so-called gel dome is formed. Entire second application solution BB is gelled soon. The cell in first application solution AA is deposited in the gel dome, whereby a cell tissue is formed. Thereafter, as in the first example, a culture medium is dripped and is cultured under the same environment as the environment in the first example. A cell tissue is formed from the cell supplied into well 12 using the gelling agent in a step of supplying first application solution AA and subsequent steps. However, if fibrinogen is contained in first application solution AA, a gel dome is not formed.

To summarize the above, at least one of collagen and fibrinogen serving as the gelling agent is contained in at least one of first application solution AA and second application solution BB. That is, one of collagen and fibrinogen serving as the gelling agent may be contained in at least one of first application solution AA and second application solution BB. Although not explained above, both of collagen and fibrinogen serving as the gelling agent may be contained in at least one of first application solution AA and second application solution BB.

(About the gelling agent)

As in the second example of the cell tissue explained above, in the present embodiment, first application solution AA and second application solution BB may be supplied into well 12 by a two-liquid mixing scheme. Note that the two-liquid mixing scheme means that both of a first application solution and a second application solution contain at least one of a gelling agent and a gelation initiator. That is, first application solution AA may contain the cell and the gelation initiator and second application solution BB may contain the gelling agent. In this case, a scaffold-free cell tissue can be constructed. First application solution AA may contain the cell and the gelling agent and second application solution BB may contain the gelation initiator. In this case, a cell tissue containing a scaffold can be constructed. Alternatively, both of first application solution AA and second application solution BB may contain the gelling agent.

The gelling agent is not particularly limited. A temperature responsive gelling agent can be used besides the gelling agent used in the two-liquid mixing scheme. For example, protein, a sugar chain, a natural polymer, a synthetic polymer, peptide, and the like may be contained in the gelling agent. More specifically, collagen, fibrinogen, gelatin, GelMA, sodium alginate, polyamino acid, polyethylene glycol, a thermosensitive polymer, Matrigel, and the like may be contained in the gelling agent. A cell tissue can be constructed by combining a plurality of kinds selected as appropriate from the gelling agents described above.

When the gelling agent is used in the two-liquid mixing scheme, a gelation initiator corresponding to the gelling agent needs to be contained. For example, if the gelling agent is fibrinogen, thrombin is used as the gelation initiator. If the gelling agent is sodium alginate, the gelling initiator is calcium chloride.

The high viscosity solution having the viscosity higher than the viscosity of the gelling agent is not particularly limited. Specifically, as the high viscosity solution, any one selected out of a group consisting of sodium alginate, sodium hyaluronate, methylcellulose, hydroxybutylcellulose, and cellulose nanofiber is used. A plurality of kinds selected as appropriate from the high viscosity solutions described above can be used in combination.

(About materials other than the gelling agent)

A cell that can be used in the present embodiment is not particularly limited. However, the cell may be, for example, any one selected out of a group consisting of a cardiomyocyte, a neuron, a hepatocyte, a cancer cell, a vascular endothelial cell, and a fibroblast. More specifically, the cell may be any one among various primary cells such as a cardiomyocyte, a neuron, a hepatocyte, a vascular endothelial cell, a fibroblast, an epidermal cell, a smooth muscle cell, a gastrointestinal cell, a renal cell, and a pancreatic cell, a differentiated cell derived from a stem cell (iPS, ES, and the like), various cancer cells, and the like.

As the cell, a cell obtained by mixing a plurality of cells may be cultured regardless of whether the cells are disease-like or normal-like. That is, a third cell and a fourth cell different from the third cell may be cocultured to be contained as the cell in one first application solution AA among N first application solutions AA. The third cell may be the same as or different from a first cell explained below. The fourth cell may be the same as or different from a second cell explained below.

A ratio of the volume of a cell contained in first application solution AA is represented as cell volume concentration. That is, the cell volume concentration indicates a ratio of the volume of the cell to the volume of entire first application solution AA. The cell volume concentration of first application solution AA is preferably 0.001 vol% or more and 50 vol% or less. In the present specification, the cell volume concentration is described in volume percentage (unit vol%). For example, 50 vol% is equal to 50 v/v%. 50 vol/% is also equal to the ratio of the volume being 50%. For this reason, the volume percentage may be described in unit v/v%. Alternatively, the volume percentage may be described in unit % in the present specification. That is, a numerical value of the volume percentage represented by unit vol% is equal to a numerical value of the volume percentage represented by unit v/v% and a numerical value of a percentage represented by unit %. It is preferable that the cell volume concentration of first application solution AA is 1 vol% or more and 30 vol% or less in the cell volume concentration described above. It is preferable that the cell volume concentration of first application solution AA is 25 vol% in the cell volume concentration.

When N (a natural number of $N \geq 2$) first application solution AA are supplied into well 12, a cell may be contained in at least one of N first application solutions AA supplied into well 12, a cell may not be contained in at least another one first application solution AA different from the at least one, and at least any one selected out of a group consisting of a cytokine, a drug, an ECM, and an additive factor may be contained in other one first application solution AA. That is, at least any one selected out of the group consisting of the cytokine, the ECM, and the additive factor may be contained in first application solution AA or a plurality among the cytokine, the ECM, and the additive factor may be mixed. The cytokine, the drug, the ECM, and the additive factor acts on a cell contained in at least one first application solution AA.

Further, when a plurality of first application solutions AA are applied in one well 12, for example, cells of kinds different among first application solutions AA may be contained such that one of plurality of first application solutions AA is a cardiomyocyte and another one is a cancer cell. Alternatively, a cardiomyocyte may be contained in one of plurality of first application solutions AA and the cardiomyocyte of the same kind may be contained in another one. That is, a cell contained in at least one of N first application solutions AA supplied into well 12 is a first cell and a cell contained in another at least one first application solution AA different from the at least one is a second cell. The second cell may be a type that is the same as or different from the first cell. Note that, in first application solution AA containing the first cell or the second cell, an additive such as an extracellular matrix component such as fibronectin, gelatin, collagen, laminin, elastin, or Matrigel or a cell growth factor such as a fibroblast growth factor and a platelet-derived growth factor may be contained. Accordingly, an environment in which a cell contained in first application solution AA can stably adhere and proliferate is given.

The culture medium is not particularly limited. As the culture medium, any one selected out of a group consisting of DMEM, RPMI1640, M199, and MEM can be used. Various additives not particularly limited such as an antibiotic, an FBS, and various growth factors can be added to the culture medium. The same solution can be used as the culture medium and second application solution BB. However, when the same solution is used as the culture medium and second application solution BB, the culture medium needs to be a high viscosity solution.

A position where the culture medium is dripped is preferably a position planarly overlapping the center of second application solution BB but is not limited to this. The culture medium may be the same solution as second application solution BB. However, a solution for forming the culture medium is supplied independently of second application solution BB to immerse second application solution BB.

Further, for example, in the first example or the second example of the cell tissue explained above, a polysaccharide thickener may be mixed in at least one of first application solution AA and second application solution BB. As the polysaccharide thickener, specifically, any one selected out of a group consisting of sodium alginate, sodium hyaluronate, methylcellulose, hydroxybutylcellulose, and cellulose nanofiber may be used. An effect of improvement of firmness of first application solution AA can be obtained by mixing the polysaccharide thickener.

[Example 1]

An example in which only one first application solution AA is formed and a gelling agent is contained in first application solution AA is explained.

As a first application solution, a solution obtained by mixing 1.05 mg/mL of a collagen solution in a solution containing iPS cardiomyocytes (iCell cardiomyocyte 2) and cardiac fibroblasts at a number ratio of 4:1 was used. The cell volume concentration of the solution containing the iPS cardiomyocytes and the cardiac fibroblasts at the number ratio of 4:1 was 25 vol%. As a second application solution, a PBS (+) solution in which 0.75 g of methylcellulose was dissolved in 100 mL of a solution and that contained calcium ions and magnesium ions was used. As a culture medium, an iCell seeding culture medium was used. As an application target object, one well 12 included in sample application set 11 including ninety-six wells 12 (see Fig. 3, referred to as 96 well plate) was used. The first application solution was supplied to the center of a circular well having a diameter of 6.5 mm of the 96 well plate using an application needle having a needle diameter of 700 μm. The second application solution having a diameter of 4.5 mm was dripped right on the center of the first application solution by a dispenser. Further, 100 μL of a culture medium was dripped on the first application solution and the second application solution using a pipette.

Fig. 13 is a phase-contrast microscopic image in the example 1. Referring to Fig. 13, a tissue was successfully formed without the first application solution being collapsed. Note that numerical values of the parameters described above in this example are as described below and satisfy all of the formulas described above. $r_{11}=r_{1v}=0.7$ mm, L1=0 mm, $r_{21}=r_{2v}=4.5$ mm, L2=0 mm, $D_{n,m}=0$ mm, $r_3=6.5$ mm, and L3=0 mm.

[Example 2]

An example in which only one first application solution AA is formed and a gelling agent is contained in second application solution BB is explained.

As a first application solution, a solution obtained by mixing 3 mg/mL of a sodium hyaluronate solution and 260 unit/mL of a thrombin solution in a solution containing iPS cardiomyocytes (iCell cardiomyocyte 2) and cardiac fibroblasts at a number ratio of 4:1 was used. The cell volume concentration of the solution containing the iPS cardiomyocytes and the cardiac fibroblasts at the number ratio of 4:1 was 10 vol%. As a second application solution, a PBS (+) solution in which 5 mg/mL of a sodium hyaluronate solution and 5 mg/mL of a fibrinogen solution were mixed and that contained calcium ions and magnesium ions was prepared. As a culture medium, an iCell seeding culture medium was used. As an application target object, one well 12 of a 96 well plate was used. The first application solution was supplied to the center of a circular well having a diameter of 6.5 mm of the 96 well plate using an application needle having a needle diameter of 700 μm. The second application solution having a diameter of 3.5 mm was dripped right on the center of the first application solution by a dispenser. Further, 100 μL of a culture medium was dripped on the first application solution and the second application solution using a pipette.

Fig. 14 is a phase-contrast microscopic image in the example 2. Referring to Fig. 14, a tissue was successfully formed without the first application solution being collapsed. Note that numerical values of the parameters described above in this example are as described below and satisfy all of the formulas described above. $r_{11}=r_{1v}=0.7$ mm, L1=0 mm, $r_{21}=r_{2v}=3.5$ mm, L2=0 mm, $D_{1,1}=0$ mm, $r_3=6.5$ mm, and L3=0 mm.

[Example 3]

An example in which two first application solutions AA are formed, one second application solution BB is formed, and a gelling agent is contained in first application solutions AA is explained.

As a first application solution, a solution obtained by mixing 1.05 mg/mL of a collagen solution in a solution containing iPS cardiomyocytes (iCell cardiomyocyte 2) and cardiac fibroblasts at a number ratio of 4:1 was used. The cell volume concentration of the solution containing the iPS cardiomyocytes and the cardiac fibroblasts at the number ratio of 4:1 was 25 vol%. As a second application solution, a PBS (+) solution in which 0.75 g of methylcellulose was dissolved in 100 mL of a solution and that contained calcium ions and magnesium ions was used. As a culture medium, an iCell seeding culture medium was used. As an application target object, one well of a 96 well plate was used.

Fig. 15 is a schematic plan view illustrating the positions and the dimensions of application solutions supplied in the example 3. Fig. 16 is a schematic plan view illustrating the center positions and the distances between the centers of the same application solutions as the application solutions in Fig. 15. Referring to Fig. 15 and Fig. 16, 1-st first application solution A1o was supplied to, using an application needle having a needle diameter of 1000 μm, a position deviating 1.0 mm from the center of a circular well having a diameter of 6.5 mm of the 96 well plate. A 2-nd first application solution A2o was supplied, using an application needle having a needle diameter of 700 μm, to a position deviating 2.0 mm from the center of first application solution A1o. Second application solution BB having a diameter of 4.5 mm was dripped by a dispenser centered on a position (the outer edge of first application solution A1o) deviating 0.5 mm from the center of first application solution A1o on a straight line connecting the centers of two first application solutions A1o and A2o. Further, 100 μL of a culture medium was dripped on the first application solution and the second application solution using a pipette.

Fig. 17 is a phase-contrast microscopic image in the example 3. Referring to Fig. 17, a tissue was successfully formed without the two first application solutions being collapsed. Note that numerical values of the parameters described above in this example are as described below and satisfy all of the formulas described above. $r_{11}=0.7$ mm, $r_{12}=1.0$ mm, $d_{1,1}=2.0$ mm, $r_{1v}=1.425$ mm, $r_{21}=r_{2v}=4.5$ mm, L2=0 mm, $D_{1,1}=0$ mm, $P_{1,1}=0$ mm, $r_3=6.5$ mm, and L3=0.5 mm.

[Example 4]

An example in which a large number of (three or more) first application solutions AA are formed, one second application solution BB is formed, and a gelling agent is contained in second application solution BB is explained.

As a first application solution, a solution obtained by mixing 3 mg/mL of a sodium hyaluronate solution and 260 unit/mL of a thrombin solution in a solution containing iPS cardiomyocytes (iCell cardiomyocyte 2) and cardiac fibroblasts at a number ratio of 4:1 was used. The cell volume concentration of the solution containing the iPS cardiomyocytes and the cardiac fibroblasts at the number ratio of 4:1 was 10 vol%. As a second application solution, a PBS (+) solution in which 5 mg/mL of a sodium hyaluronate solution and 5 mg/mL of a fibrinogen solution were mixed and that contained calcium ions and magnesium ions was prepared. As a culture medium, an iCell seeding culture medium was used. As an application target object, one well of a 96 well plate was used.

Fig. 18 is a phase-contrast microscopic image in the example 4. Referring to Fig. 18, all of circles in the figure indicate the outer edges of first application solutions. That is, a photograph on the upper left in Fig. 18 illustrates a cell tissue to which four first application solutions are applied at intervals to not come into contact with one another. A photograph on the upper right in Fig. 18 illustrates a cell tissue to which five first application solutions are linearly applied to partially come into contact with one another. A photograph on the lower left in Fig. 18 illustrates a cell tissue in which eight first application solutions are arranged to form the periphery of a square to partially come into contact with one another. A photograph on the lower right in Fig. 18 illustrates a cell tissue in which six first application solutions are arranged to form a solid triangle to partially come into contact with one another.

Although not illustrated in Fig. 18, a second application solution neither being inscribed nor circumscribed with the first application solution and having second dimension $r_{2m}$ sufficiently larger than first virtual dimension $r_{1v}$ of a first virtual curve was supplied. For this reason, a tissue was successfully formed without the first application solution being collapsed. In examples of all the photographs, dimension $r_{1n}$ of the first application solution is 0.2 mm. A value of $d_{n,m}$ is 0.3 mm only in the photograph on the upper left and is 0.15 mm in all of the photographs other than the photograph on the upper left.

[Example 5]

An example in which only second application solution BB is applied in well 12 while an application position being changed is explained.

Fig. 19 is a schematic diagram and a photograph illustrating a position where the second application solution is assumed to be applied and a position where the second application solution has been actually applied at the time when an application position in a well of the second application solution has been changed. An upper part of Fig. 19 is a schematic diagram illustrating the position where the second application solution is assumed to be applied. A middle part of Fig. 19 is a schematic diagram illustrating the position where the second application solution has been actually applied. A lower part of Fig. 19 is an actual photograph for the schematic diagram illustrated in the middle part of Fig. 19. As the second application solution, a PBS (+) solution in which 0.75 g of methylcellulose was dissolved in 100 mL of a solution and that contained calcium ions and magnesium ions was used. The dimension of the second application solution is 4.5 mm and the diameter of the well is 6.5 mm.

Referring to Fig. 19, a result as assumed was obtained when the second application solution was applied targeting the center of the well as illustrated in a left row. An application result as assumed was also obtained when the second application solution was applied targeting a position separated to the upper side by 0.5 mm from the center of the well as illustrated in a middle row. However, when the second application solution was applied targeting a position separated to the upper side by 1.0 mm from the center of the well as illustrated in a right row, unlike an assumed schematic diagram, the second application solution adheres to the inner wall on the upper side of the well and an elliptical second application solution was obtained. Accordingly, if the second application solution is applied to be inscribed with the inner wall of the well, it is likely that the position and the shape of the second application solution are different from assumption and the second application solution is disposed in a position deviating from the first application solution. For this reason, it is highly likely that the first application solution collapses. Therefore, it is requested to supply the second application solution not to come into contact with the inner wall of the well.

(Second embodiment)

A technical field of the present embodiment relates to a method of producing a myocardial tissue from a cardiomyocyte using an application device (a bioprinter). Specifically, the technical field relates to production of a myocardial tissue using the same application device as the application device in the first embodiment.

Fig. 20 is a schematic diagram illustrating a mode before a first application solution in the second embodiment is applied. Referring to Fig. 20, first, the distal end of application needle 24 configuring the bioprinter is immersed in first application solution AA (a first bioink) and first application solution AA is deposited on the distal end of application needle 24. Fig. 21 is a schematic diagram illustrating a composition of the first application solution in the second embodiment. Referring to Fig. 21, first application solution AA is obtained by mixing a cell C that is to be cultured, collagen serving as a gelling agent, and a first solvent m. Cell C in Fig. 21 includes C1 and C2, which are explained below. Fig. 22 is a schematic diagram illustrating a step of applying the first application solution in the second embodiment. Referring to Fig. 22, the distal end of application needle 24 where first application solution AA is deposited comes into contact with, for example, the bottom (a well bottom 12a) of well 12 (see Fig. 3). This is performed by application needle 24 on which first application solution AA is deposited moving downward as indicated by an arrow M1 in Fig. 20. Accordingly, first application solution AA is applied to well bottom 12a. Fig. 23 is a schematic diagram illustrating a mode after the first application solution in the second embodiment has been applied. Referring to Fig. 23, thereafter, application needle 24 moves upward as indicated by an arrow M2. In this way, first application solution AA is applied to a container such as well 12 using a so-called pin-type bioprinter.

Fig. 24 is a schematic diagram illustrating a step in which a second application solution in the second embodiment is supplied. Fig. 25 is a schematic diagram illustrating a mode in the well after the step in Fig. 24 has been performed. Referring to Fig. 24 and Fig. 25, second application solution BB (a second bioink) is supplied into well 12 to cover first application solution AA applied in well 12. Second application solution BB may be dripped by, for example, a dispenser. However, a method of supplying second application solution BB is not limited to this. Second application solution BB may be supplied by any one scheme selected out of a group consisting of a pin scheme, an inkjet scheme, a dispenser scheme, and manual operation using a pipette.

Fig. 26 is a schematic diagram illustrating a step in which a culture medium in the second embodiment is supplied. Referring to Fig. 26, culture medium M is supplied into well 12 after a step of supplying second application solution BB. Culture medium M is supplied such that first application solution AA and second application solution BB are immersed and covered. A method of dripping culture medium M is not particularly limited. Culture medium M may be supplied by any one scheme selected out of a group consisting of a pin scheme, an inkjet scheme, a dispenser scheme, and manual operation using a pipette. Alternatively, culture medium M may be supplied by a dispenser or a micropump.

Fig. 27 is a schematic diagram illustrating a mode of a cell in the first application solution before culture. Fig. 28 is a schematic diagram illustrating a mode of the cell in the first application solution after the culture. Referring to Fig. 27 and Fig. 28 (and Fig. 21), cell C in first application solution AA before culture includes cardiomyocytes C1 and cardiac fibroblasts C2. A ratio of the number of cardiomyocytes C1 in cell C is 75% or more but is preferably 80% or more. A ratio of the number of cardiac fibroblasts C2 in cell C is 20% or less. Therefore, when the ratio of the number of cardiomyocytes C1 is the lowest, C1:C2=80:20. When the ratio of the number of cardiomyocytes C1 is the highest, C1:C2=100:0. In other words, in the comparison formulas described above, number ratios (%) in cell C are $80 \leq C1 \leq 100$ (or $75 \leq C1 \leq 100$) and is $0 \leq C2 \leq 20$. The cell volume concentration of cardiomyocytes C1 and cardiac fibroblasts C2 contained in first application solution AA is preferably 0.001 vol% or more and 50 vol% or less. In that range, the cell volume concentration is more preferably 1 vol% or more and 30 vol% or less. In that range, the cell volume concentration is most preferably 25 vol%. By setting the ratios of the numbers and the cell volume concentration of cardiomyocytes C1 and cardiac fibroblasts C2 as explained above, a cell tissue by culturing a cell can be stably formed as in an example 6 explained below. By the culture, both of cardiomyocytes C1 and cardiac fibroblasts C2 grow from a state in Fig. 27 as illustrated in Fig. 28 and a myocardial tissue is formed.

(Materials)

Here, materials usable in the application solutions and the like in the present embodiment are explained.

In first application solution AA (the first bioink), cell C, the collagen, and first solvent m are mixed. In first application solution AA, various additives may be added to the mixed solution described above.

Cardiomyocyte C1 included in cell C may be at least one of a normal cardiomyocyte and a cardiomyocytes derived from a disease. The normal cardiomyocyte is, for example, a human iPS cell-derived cardiomyocyte and the cardiomyocyte derived from a disease is, for example, a disease-derived human iPS cell-derived cardiomyocyte. Cardiomyocyte C1 may be one of a primary cardiomyocyte and a stem cell-derived cardiomyocyte. The primary cardiomyocyte may be cardiomyocyte derived from various animal species such as a human, a mouse, and a rat. Examples of the stem cell-derived cardiomyocyte include the iPS cell-derived cardiomyocyte (described above), an ES cell-derived cardiomyocyte, and a directory programming cell-derived cardiomyocyte. Examples of the cardiomyocyte derived from a disease include a cell collected from animal species having a disease, a cell obtained by differentiating and inducing a stem cell produced from a cell collected from animal species that have developed a disease, and a cell subjected to genetic modification. Other than cardiomyocyte C1, a cardiac fibroblast C2, a vascular endothelial cell, a neuron, and the like may be mixed in cell C. As the cells other than the cardiomyocyte, like the cardiomyocyte, a cell derived from an animal, a cell derived from a disease, or the like can be used. If these are used, a cell tissue obtained by culture can be made more closely similar to the myocardial tissue.

As the collagen serving as the gelling agent, collagens type I, type II, type III, and type V are used. However, in the present embodiment, it is preferable that the collagen type I is used and it is particularly preferable that a collagen type I-A is used. This is because, among collagens, the collagen type I-A most often appears in the heart. The concentration of the collagen type I-A is 0.01 mg/mL or more and 2.1 mg/mL or less, and, in that range, 0.1 mg/mL or more and 2.1 mg/mL or less is preferable. Further, in that range, 0.5 mg/mL or more and 2.1 mg/mL or less is particularly preferable. A fibrin gel, Matrigel, gelatin, GelMA (gelatin methacryloyl), and the like may be mixed in a gel of the collagen in first application solution AA at any ratios.

The material of first solvent m in first application solution AA is not particularly limited. However, a culture medium or a buffer solution is preferably used as first solvent m. For example, as the culture medium serving as first solvent m, it is preferable to use at least any one selected out of a group consisting of DMEM, DMEM/Ham F-12, αMEM, RPMI1640 (for example, a culture medium manufactured by Lonza, manufactured by Thermo, manufactured by Nacalai Tesque, or manufactured by Wako; model number is, for example, 30264-85 (Nacalai Tesque, Inc.)), a William culture medium, M199, a commercially available culture cell exclusive for cardiomyocyte (a culture medium for iCell cardiomyocyte defrosting, an iCell cardiomyocyte maintenance culture medium, or the like), and a commercially available culture medium (FGM-3) exclusive for fibroblast. As the buffer solution of first solvent m, it is preferable to use at least any one selected out of a group consisting of PBS (+/-), a Tris buffer solution, and a Tyload buffer solution. As first solvent m, a solvent obtained by selecting two or more kinds from at least one of the various culture media and the various buffer solutions explained above and mixing the two or more kinds may be used. In that case, a ratio of mixing the two or more kinds may be optional.

The various additives in first application solution AA are not particularly limited. However, as the various additives in first application solution AA, a thickener, a cell growth factor, a protein, a drug, and the like may be added. The thickener of first application solution AA may contain one or more substances selected from a group consisting of cellulose, cellulose nanofiber, chitin, chitosan, chitin nanofiber, chitosan nanofiber, methyl cellulose, carboxymethyl cellulose, hydroxybutyl cellulose, sodium alginate, sodium hyaluronate, polyethylene glycol, gellan gum, carrageenan, pectin, xanthan gum, gelatin, agarose, and polyvinyl alcohol.

The cell grows factor, the protein, the drug, and the like serving as the various additives in first application solution AA may be selected out of a group consisting of an FGF (a fibroblast growth factor), a B27 (registered trademark) supplement, triiodothyronine, dexamethasone, a growth factor for insulin (IGF-I), insulin, a nerve growth factor (NGF), isoproterenol, an epidermal growth factor (EGF), a hepatocyte growth factor (HGF), laminin, fibronectin, collagen, gelatin, sodium hyaluronate, proteoglycans, nidogen, a Rho kinase inhibitor (Rock Inhibitor), and Matrigel.

Second application solution BB (the second bioink) is composed as a mixed solution of the thickener and the second solvent. In second application solution BB, various additives may be added to the mixed solution explained above.

The thickener of second application solution BB may contain one or more substances selected out of a group consisting of cellulose, cellulose nanofiber, chitin, chitosan, chitin nanofiber, chitosan nanofiber, methyl cellulose, carboxymethyl cellulose, hydroxybutyl cellulose, sodium alginate, sodium hyaluronate, polyethylene glycol, gellan gum, carrageenan, pectin, xanthan gum, gelatin, agarose, and polyvinyl alcohol. If these substances are used, it is possible to improve firmness of second application solution BB covering first application solution AA and suppress a deficiency of second application solution BB being disposed in a deviated position other than a position for covering first application solution AA.

The material of the second solvent is not particularly limited. However, it is preferable to use a culture medium or a buffer solution as the second solvent. The culture medium and the buffer solution serving as the second solvent may be selected out of the same material group as the material group of the culture medium and the buffer solution serving as first solvent m explained above and may be mixed at the same ratio as the ratio of first solvent m and used. Therefore, the explanation of the culture medium and the buffer solution is not repeated. The various additives to the second solvent (second application solution BB) may be selected out of the same material group as the material group of the various additives to first application solution AA (first solvent m) explained above. Therefore, the explanation of the various additives is not repeated.

Culture medium M supplied after second application solution BB is selected out of the same material group as the material group of the culture medium used for first solvent m and the second solvent explained above and may be mixed at the same ratio as the ratio of first solvent m and the second solvent and used. Therefore, the explanation of culture medium M is not repeated. The various additives added to culture medium M may be selected out of the same material group as the material group of the various additives to first application solution AA and second application solution BB explained above. Therefore, the explanation of the various additives is not repeated. However, as an example, as in the example 6 explained below, it is preferable that culture medium M is RPMI1640 and additive factors (additives) to culture medium M are one or more (all of four kinds) selected out of a group consisting of a B27 (registered trademark) supplement, triiodothyronine, dexamethasone, and an insulin-like growth factor. Accordingly, as in the example 6 explained below, it is possible to stably form a cell tissue by culturing of a cell.

(Background art and problems to be solved in the second embodiment)

WO2019/088224 discloses a technique for applying a cell containing solution using a pin type bioprinter. However, according to the technique of WO2019/088224, it is likely that, in an application step in a bioprinting technique such as an application needle, an application solution unintentionally causes gelation and clogging on the inside of an application solution container before the application step is executed. Such a phenomenon is likely to occur, in particular, in an application solution in which an cardiomyocyte is dispersed in fibrinogen. Thus, Japanese Patent Laying-Open No. 2020-141599 discloses a technique for suppressing unintended gelation of an application solution before such an application step is executed. Specifically, in Japanese Patent Laying-Open No. 2020-141599, the cell, the gelation initiator, and the thickener are applied as the first application solution and the gelling agent is dripped as the second application solution, whereby a so-called gel dome is formed.

Japanese Patent Laying-Open No. 2020-141599 proposes a technique for suppressing a deficiency of the first application solution being unintentionally gelled before application. However, since the application solution is supplied by a so-called two-liquid mixing scheme, after the application, the gel dome is present as an outer shell of the application solution in which a cell tissue is stored. This makes it difficult for a drug, protein, and the like supplied from the outside of the application solution to pass through meshes of the gel dome serving as the outer shell. As a result, there is a problem in that the drug, the protein, and the like less easily permeate and act on the cell tissue. Japanese Patent Laying-Open No. 2020-141599 does not disclose an example in which protein most abundantly present in the heart of an adult is used as a gelling agent. Accordingly, it is likely that a constructed myocardial tissue cannot reproduce the heart of the adult with a high function.

The present disclosure has been devised in view of the problems described above. An object of the present disclosure is to provide a myocardial tissue production method capable of forming a myocardial tissue more similar to the heart of an adult and having a high function without permeation of a drug and the like from the outside into a cell tissue and action of the drug and the like on the cell tissue being hindered.

Thus, in the myocardial tissue production method according to the present disclosure, the distal end of application needle 24 included in the application device is immersed in first application solution AA, whereby first application solution AA is deposited on the distal end of application needle 24. First application solution AA deposited on the distal end of application needle 24 is applied in well 12 that is the application target object. First application solution AA is a mixed solution of a cell that is to be cultured, collagen serving a gelling agent, and first solvent m.

With the myocardial tissue production method of the present disclosure, since a so-called two-liquid mixing scheme is not applied, a gel dome generated in the two-liquid mixing scheme is not formed. For this reason, hinderance of permeation of a drug, protein, and the like into a cell tissue and action of the drug, the protein, and the like on the cell tissue due to the gel dome less easily occurs. Therefore, it is possible to construct a cell tissue capable of causing the drug and the protein to easily permeate and act. Collagen (in particular, collagen type I-A) is protein most abundantly present in the heart of an adult. For this reason, by containing the collagen serving as the gelling agent in first application solution AA, it is possible to construct a high function myocardial tissue more similar to the heart of the adult. Further, with the myocardial tissue production method explained above, as long as second application solution BB and the culture medium (RPMI 1640 or the like) are supplied and an application solution is not controlled to a gellable temperature (for example, 37°C), the collagen is not gelled. For this reason, it is possible to prevent unintended gelation of first application solution AA at a point in time before the supply of the application solution is completed.

[Example 6]

(Formation of a myocardial tissue)

An experiment for actually forming a myocardial tissue based on the myocardial tissue production method in the second embodiment was performed. In the experiment, an application solution was prepared as explained below using the application device (see Fig. 1). In cell C contained in first application solution AA, an iPS cardiomyocyte (iCell Cardiomyocyte 2) serving as cardiomyocyte C1 and a normal human cardiac fibroblast serving as cardiac fibroblast C2 were set to 4:1 in a number ratio. The cell volume concentration of cell C in first application solution AA was set to 25 vol%.

The gelling agent contained in first application solution AA was collagen and was collagen type I-A having concentration of 0.7 mg/mL. First solvent m contained in first application solution AA was RPMI1640.

Subsequently, second application solution BB was a mixed solution of a thickener and a second solvent. Specifically, methylcellulose was used as a thickener for improving firmness of second application solution BB. The second solvent was phosphate buffered saline (+) (PBS(+)).

A culture medium supplied after supply of second application solution BB was RPMI1640. An additive factor to the culture medium was a factor composed of a B27 (registered trademark) supplement, 75 nM of triiodothyronine, 1 μM of dexamethasone, and 100 ng/mL of an insulin-like growth factor (IGF-I).

In this example, in a step in Fig. 20, application needle 24, a cross section of which cut to be orthogonal to an extending direction in a region where a needle diameter of application needle 24 other than the distal end potion was fixed was a circle having a diameter of 1000 μm, was used. First application solution AA was applied to one well 12 (see Fig. 3) of the 96 well plate. Second application solution BB was dripped using a dispenser to cover first application solution AA. Further, culture medium RPMI1640 added in well 12 was exchanged once in two or three days and cultured for six days to seven days, whereby a cell tissue, that is, a myocardial tissue was constructed. The number of culture days is preferably six days or more. The number of culture days may be two weeks or more.

Fig. 29 is a phase-contrast microscopic image (left) immediate after an application solution is supplied into a well and a phase-contrast microscopic image (right) after being cultured for six days. Referring to Fig. 29, an application diameter of fifty-six second application solutions BB was 1095±42 μm. In this way, a myocardial tissue was successfully stably formed.

(Ca$^{2+}$ transient measurement)

After a myocardial tissue after being cultured for seven days was dyed, Ca$^{2+}$ transient measurement was performed from the viewpoint of checking a function of myocardial contraction. The measurement was performed for a myocardial tissue after culture (hereinafter described as "application tissue") illustrated in Fig. 29 in this example and a planarly cultured tissue (hereinafter described as "2D tissue") serving as a comparative example. In the myocardial tissue in the comparative example, iPS cardiomyocytes (iCell Cardiomyocyte 2) and normal human cardiac fibroblast were set to 4:1 in a number ratio. In the myocardial tissue in the comparative example, 50000 cells per one well were seeded and planarly cultured in the same culture medium as the culture medium of the application tissue obtained by the same application device as the application device in the second embodiment. A result of the Ca$^{2+}$ transient measurement is illustrated in the following Table 1.

[Table 1]

Table 1

| | Application tissue | 2D tissue |
|---|---|---|
| Heart rate (BPM) /min | 32.2+3.4 | 16.9±1.88 |
| Peak width (PWD90cF) msec | 945±36 | 1805±134 |

As illustrated in Table 1, in the application tissue, a heart rate (BPM) of the obtained myocardial tissue was approximately twice as large as the heart rate in 2D tissue. In the application tissue, a peak width of fluorescence intensity of a Ca$^{2+}$ transient waveform detected by the contraction of the myocardial tissue was approximately a half of the peak width in the 2D tissue. Note that the peak width of the fluorescence intensity means a time width of a Ca$^{2+}$ transient waveform illustrated in Fig. 30 referred to next. Accordingly, it was successfully confirmed that the application tissue is a tissue stabler than the 2D tissue.

Fig. 30 is a graph illustrating a Ca$^{2+}$ transient waveform in the experiment of the application tissue. The horizontal axis of Fig. 30 indicates an elapsed time and the vertical axis indicates detected fluorescence intensity. Referring to Fig. 30, it is seen that a peak width of the fluorescence intensity in the application tissue is approximately 945 msec. Note that, in the graph of Fig. 30, the fluorescence intensity of the Ca$^{2+}$ transient waveform is indicated by AMP and the peak width of the fluorescence intensity is indicated by PWD90.

Subsequently, a change in a Ca$^{2+}$ transient waveform at the time when isoproterenol that is a β adrenergic receptor agonist was added was evaluated. In general, the isoproterenol raises the heart rate and the contraction force of a cardiomyocyte. The contraction force of the cardiomyocyte has a correlation with the fluorescence intensity (AMP) of the Ca$^{2+}$ transient measurement. That is, whereas the fluorescence intensity rises in a matured cardiomyocyte, the fluorescence intensity does not rise in an immature cardiomyocyte.

Fig. 31 is a graph illustrating a change in BPM at the time when the isoproterenol was added to each of the application tissue and the 2D tissue. The horizontal axis of Fig. 31 indicates an addition amount of the isoproterenol in a unit nM and the vertical axis indicates a change amount of the BPM in a unit %. Referring to Fig. 31, a value of the BPM rose according to an increase in the isoproterenol concentration in both of the application tissue and the 2D tissue.

Fig. 32 is a graph illustrating a change in AMP at the time when the isoproterenol was added to each of the application tissue and the 2D tissue. The horizontal axis of Fig. 32 indicates an addition amount of the isoproterenol in a unit nM and the vertical axis indicates a change amount of the AMP in a unit %. Referring to Fig. 32, a value of the AMP rose depending on the concentration of the isoproterenol in the application tissue but did not rise much even if the concentration of the isoproterenol increased in the 2D tissue. This result indicates possibility that the cardiomyocyte has matured and has a high function in the application tissue compared with the 2D tissue.

When the above is comprehensively examined, compared with the comparative example in which gel is not mixed and only the cell is planarly cultured, an excellent effect was obtained in this example in which the solution in which the collagen serving as the gelling agent was mixed in the cell was supplied by the application device.

(Third embodiment)

In the following explanation, a culture container may be described as "plate" and an individual well formed in the plate may be described as "container".

First, characteristics of the present embodiment are briefly explained. Referring to Fig. 39, the present embodiment includes a step of supplying, using a first application mechanism 104, a first application material A1 serving as an application material to a first container 112A-1 included in a plate 111 including a plurality of containers 112A capable of receiving the application material. Referring to Fig. 33 and Fig. 40, the present embodiment includes a step in which a stage 101 on which plate 111 is placed moves. Referring to Fig. 40, the present embodiment includes a step of supplying, using first application mechanism 104, first application material A1 to a second container 112A-2 adjacent to first container 112A-1 of plate 111 and, at the same time, supplying, using a second application mechanism 105 adjacent to first application mechanism 104, a second application material B serving as the application material to first container 112A-1. This is explained in detail below.

(Configuration of the application device)

Fig. 33 is a schematic front view illustrating a first example of the application device according to the third embodiment. Note that, for convenience of explanation, an X direction, a Y direction, and a Z direction are introduced. Referring to Fig. 33, an application device 100 includes a needle application mechanism 104 and dripping mechanisms 105 as an application mechanism 107 capable of supplying an application material that is to be applied. In this way, in the present specification, "application" includes both of supply of the application material using an application needle explained below and supply by dripping of the application material. For this reason, the former supply using the application needle may be referred to as "needle application" in the present specification. Application device 100 in Fig. 33 includes one needle application mechanism 104 and two dripping mechanisms 105. Two dripping mechanisms 105 are a dripping mechanism 105-1 and a dripping mechanism 105-2, which are arranged side by side at an interval in the X direction. Needle application mechanism 104 and dripping mechanisms 105 are arranged side by side at intervals in the X direction. These mutual X-direction intervals are fixed and do not change.

An X-axis stage 101 (the stage) is movable in the X direction that is the horizonal direction. A Y-axis stage 102 is movable in the Y direction that is the horizontal direction. Specifically, for example, a guide unit is installed on the lower surface of X-axis stage 101 or Y-axis stage 102. The guide unit is slidably connected to a not-illustrated guide rail. For example, the surface on the upper side of X-axis stage 101 is formed as a mounting surface on which plate 111 can be placed. In Fig. 33, X-axis stage 101 is disposed on Y-axis stage 102 and plate 111 is placed on X-axis stage 101. However, conversely, X-axis stage 101 may be disposed on Y-axis stage 102 and plate 111 may be placed on X-axis stage 101.

Needle application mechanism 104, dripping mechanisms 105, and an observation optical system 106 are connected to, for example, a Z-axis table that is a member movable in the Z direction. That is, needle application mechanism 104, dripping mechanisms 105, and observation optical system 106 are held to be movable in the Z direction in application device 100. Observation optical system 106 observes and measures, for example, a position where an application material is to be applied on plate 111. A CCD camera that converts an observed image into an electric signal may be installed in observation optical system 106. The observation of plate 111 and the like by observation optical system 106 may be performed by visible light. However, the observation of plate 111 and the like may be performed not only by the visible light but also by an infrared ray, an X ray, ultrasound, or the like. The observation of plate 111 can be performed using magnetism depending on the material of plate 111. Plate 111 observed by means other than the visible light does not need to be transparent or semitransparent and may be opaque.

Fig. 34 is a schematic front view illustrating a second example of the application device according to the third embodiment. Referring to Fig. 34, application device 100 according to the second example has basically the same configuration as the configuration of application device 100 according to the first example. Therefore, the same components are denoted by the same reference numerals and signs and explanation of the components is not repeated as long as functions and the like are the same. Application device 100 in Fig. 34 includes three needle application mechanisms 104 and two dripping mechanisms 105. Three needle application mechanisms 104 are a needle application mechanism 104-1, a needle application mechanism 104-2, and a needle application mechanism 104-3. The three needle application mechanisms are arranged side by side at intervals in the X direction. The mutual X-direction intervals are fixed and do not change. For example, all of X-direction intervals between mechanisms (the centers of through-holes) adjacent to each other of each of three needle application mechanisms 104-1 to 104-3 and two dripping mechanisms 105-1 and 105-2 may be equal to a first interval D1 between containers 112A explained below. In the following explanation of the third embodiment, application device 100 including only a single needle application mechanism 104 in Fig. 33 is used.

Fig. 35 is a schematic diagram illustrating a needle application mechanism of the application device illustrated in Fig. 33. Referring to Fig. 35, needle application mechanism 104 in the present embodiment (needle application mechanisms 104-1 and needle application mechanism 104-2 in Fig. 34 are the same as needle application mechanism 104) mainly includes servomotor 41, cam 43, bearing 44 held in a state of being in contact with a cam surface of cam 43, cam coupling plate 45, movable unit 46, movable base 35 that holds application needle holder 20, and application material container 21. Application needle holder 20 is detachably attachable to movable base 35. In other words, movable base 35 serving as a base body detachably holds application needle holder 20.

Fig. 36 is a schematic perspective view of the plate. Referring to Fig. 36, in the present embodiment, an application material serving as a liquid material is applied and supplied to the inside of a plurality of containers 112A formed in plate 111. However, an application target object to which the application material is supplied is not limited to this. Plate 111 has thickness in the Z-axis direction. Plurality of containers 112A are formed on the top surface of plate 111. Plurality of containers 112A are concave portions where the upper surface of plate 111 is hollowed. As plurality of containers 112A, ninety-six containers 112A in total may be formed at intervals from one another, for example, in eight rows in the Y-axis direction and twelve rows in the X-axis direction in Fig. 36. A plane shape of containers 112A is any shape such as a circular shape.

(Application method)

Fig. 37 is a flowchart illustrating an application method in the third embodiment. Referring to Fig. 37, first, a Z axis of application mechanism 107 is lowered (S1).

Fig. 38 is a schematic sectional view illustrating a part of an application mechanism and an installed plate of an application device used for the application method in the third embodiment. Referring to Fig. 38, according to a step (S1), needle application mechanism 104 (a first application mechanism) in Fig. 38 and dripping mechanism 105 (a second application mechanism), an interval of which in the X direction from needle application mechanism 104 is a first interval D1, are lowered. Needle application mechanism 104 (needle application mechanism 104 in Fig. 33) includes a first application material container 21A-1 in which first application material A1 is stored. Dripping mechanism 105 (dripping mechanism 105-1 in Fig. 33) includes a second application material container 21B in which second application material B is stored. Through-holes 25 for supplying an application material are formed in the bottoms of application material containers 21. The intervals among the centers of through-holes 25 are first interval D1 between needle application mechanism 104 and dripping mechanism 105.

Referring to Fig. 37 again, subsequently, a first application needle 24A-1 is lowered (S2). The lowering of first application needle 24A-1 is performed by causing a Z-axis stage 103 (see Fig. 33) included in application device 100 to operate. This enables movement of the stage by simple control.

Fig. 39 is a schematic sectional view illustrating the first step of the application method in the third embodiment. Referring to Fig. 37 and Fig. 39, in a step (S2), first application needle 24A-1 serving as application needle 24 (see Fig. 35) included in needle application mechanism 104 falls in a direction of an arrow in the figure.

At least the distal end portion (the bottom portion) of first application needle 24A-1 is stored in first application material container 21A-1. First application material A1 serving as the application material is disposed in first application material container 21A-1. For this reason, at least the distal end portion of first application needle 24A-1 is immersed in first application material A1.

Plurality of containers 112A are formed in plate 111 (see Fig. 36) installed in application device 100 (see Fig. 33). In Fig. 39, as containers 112A, container 112A-1, container 112A-2 adjacent to the X-direction left side of container 112A-1, and a container 112A-3 adjacent to the X-direction left side of container 112A-2 are illustrated. A first interval in the X direction of these containers is equal to first interval D1 between needle application mechanism 104 and dripping mechanism 105. Note that the first interval D1 is a distance between centers of respective containers 112A adjacent to each other in the X direction. In an initial state, for example, as in Fig. 39, container 112A-1 is disposed in a position planarly overlapping first application needle 24A-1.

Subsequently, first application material A1 (the first application material) is applied by first application needle 24A-1 (a part of the first application mechanism) (S3). Specifically, as in Fig. 39, first application material A1 adhering to the distal end portion of first application needle 24A-1 is supplied into container 112A-1 (the first container) by the fall of first application needle 24A-1.

Thereafter, first application needle 24A-1 rises (S4). Subsequently, plate 111 moves by first interval D1 (S5).

Fig. 40 is a schematic sectional view illustrating a second step of the application method in the third embodiment. Referring to Fig. 37 and Fig. 40, in a step (S5), a pre-simultaneous supply movement step in which, for example, X-axis stage 101 on which plate 111 is placed moves to, for example, the right side in the X direction as indicated by an arrow M is performed. Accordingly, X-axis stage 101 and plate 111 on X-axis stage 101 move to, for example, the right side in the X direction. However, Y-axis stage 102 may be moved in the Y direction as explained below. At this time, Y-axis stage 102, X-axis stage 101, and plate 111 move in the Y direction. This enables movement of the stage by simple control.

A distance of X-axis stage 101 moving to the right side in the X direction in Fig. 40 is equal to first interval D1. As explained above, the distance between through-holes 25 of needle application mechanism 104 and dripping mechanism 105-1 is equal to first interval D1. Therefore, first interval D1 between needle application mechanism 104 and dripping mechanism 105-1 is equal to a movement amount of X-axis stage 101. For this reason, in Fig. 40, container 112A-2 is disposed in a position planarly overlapping first application needle 24A-1. In Fig. 40, container 112A-1 is disposed in a position planarly overlapping through-hole 25 of dripping mechanism 105.

Subsequently, in Fig. 40, the steps (S2) to (S4) are repeated as in Fig. 39. That is, first application material A1 adhering to the distal end portion of first application needle 24A-1 is supplied into container 112A-2 (the second container).

At the same time, a simultaneous supply step in which second application material B dripped from through-hole 25 of second application material container 21B is supplied into container 112A-1 (the first container) is performed (S21). Second application material B is supplied to cover first application material A1.

Fig. 41 is a schematic sectional view illustrating a third step of the application method in the third embodiment. Referring to Fig. 37 and Fig. 41, it is determined whether first application material A1 has been finished being supplied to all of regions to which first application material A1 is to be supplied (S100). In Fig. 41, first application material A1 is not supplied yet in container 112A-3. Thus, the third step proceeds from (S100) in the direction of N and, as illustrated by an arrow M in the figure, plate 111 moves again by first interval D1 (S5). Accordingly, container 112A-3 is disposed in a position planarly overlapping first application needle 24A-1. In Fig. 41, container 112A-2 is disposed in a position planarly overlapping through-hole 25 of dripping mechanism 105. Thereafter, as in Fig. 39 and Fig. 40, first application material A1 adhering to the distal end portion of first application needle 24A-1 is supplied into container 112A-3. At the same time, as in Fig. 40, a simultaneous supply step in which second application material B dripped from through-hole 25 of second application material container 21B is supplied into container 112A-2 is performed (S21). The simultaneous supply step in Fig. 41 is repeated until containers 112A, to which first application material A1 is to be supplied, for example, arranged in a row in the X direction become absent. Y-axis stage 102 moves in the Y direction, whereby X-axis stage 101 and plate 111 present on Y-axis stage 102 in Fig. 33 also move in the Y direction. Thereafter, the same supply as the supply explained above may be performed to containers 112A adjacent in Y direction and arranged in a row.

In the step in Fig. 40, container 112A-2 is the first container and container 112A-1 is the second container. In contrast, in the step in Fig. 41, container 112A-3 is the first container and container 112A-2 is second container. In this way, every time the pre-simultaneous supply movement step and the simultaneous supply step are performed, containers equivalent to the first container and the second container are changed to shift one by one in a moving direction of X-axis stage 101 (the X direction).

Fig. 42 is a schematic sectional view illustrating a fourth step of the application method in the third embodiment. Referring to Fig. 37 and Fig. 42, it is determined whether first application material A1 has been finished being supplied to all regions to which first application material A1 is to be supplied (S100). In Fig. 42, first application material A1 has been finished being applied to all of container 112A-1 to container 112A-3. Thus, the fourth step proceeds from (S100) in the direction of Y and plate 111 moves as indicated by an arrow M in the figure by first interval D1 again (S5). Accordingly, in Fig. 42, container 112A-3 is disposed in a position planarly overlapping through-hole 25 of dripping mechanism 105. Thereafter, as in Fig. 40 and Fig. 41, second application material B is dripped into container 112A-3 (the second container) from through-hole 25 of second application material container 21B (S21). Thereafter, the Z axis of application mechanism 107 rises (S22) and the step ends.

Note that, at appropriate timing after first application material A1 and second application material B have been finished being supplied, a culture medium is supplied from dripping mechanism 105-2 into containers 112A. The culture medium is supplied such that the culture medium occupies most portions in containers 112A and first application material A1 and second application material B are immersed.

(About an application material to be supplied)

In first application material A1, a cell that is to be cultured and thrombin that is a gelation initiator are mixed. As an example, in first application material A1, for example, a cardiomyocyte derived from an iPS cell and a human cardiac fibroblast are mixed at a number ratio of 3:1 at concentration of $8\times10^{-7}$ cells/mL. The concentration of the thrombin is, for example, 800 unit/mL. 13 mg/mL of sodium hyaluronate may be mixed in first application material A1. Further, a PBS solution may be mixed in first application material A1. The viscosity of a finally obtained first application material may be, for example, $1.5\times10^{-4}$ mPa·s.

Second application material B contains, as a main component, fibrinogen that is a gelling agent. However, sodium hyaluronate and the PBS solution may be mixed. As an example, in second application material B, 10 mg/mL of fibrinogen and 0.5 mg/mL of sodium hyaluronate are mixed. Accordingly, cell C contained in first application material A1 can be cultured.

(Three-dimensional cell tissue production method)

Fig. 43 is a schematic diagram illustrating an example of a three-dimensional cell tissue production method. Referring to Fig. 43, as illustrated in (A) and (B), first application material A (same as first application material A1) in which cell C to be cultured is suspended in a PBS solution or the like containing thrombin is prepared. First application material A is disposed in, for example, application material container 21 of the application device as illustrated in (C) and is supplied onto a substrate 112B or the like by application needle 24. Thereafter, as illustrated in (E), second application material B is supplied to cover first application material A by, for example, manual operation by micropipette 18. Accordingly, a contact surface of first application material A and second application material B to the outer circumference of the contact surface (second application material B) is gelled (solidified). This is installed in container 112A as illustrated in (F). Culture medium M that immerses these is supplied into container 112A. Then, as time elapses, cell C in first application material A precipitates and deposits. Accordingly, a high-density three-dimensional cell tissue is obtained. Since the inside of first application material A is maintained in a liquid state, drying of cell C in first application material A is suppressed. Fig. 44 is a photograph of a three-dimensional cell tissue obtained by the production method in Fig. 43. Referring to Fig. 44, the obtained three-dimensional cell tissue has thickness of approximately 50 μm to 200 μm. Cells are densely stacked in the three-dimensional cell tissue. Further, as a result of cell nuclei, actin, and troponin staining, it was confirmed that a cell was alive in the three-dimensional cell tissue in Fig. 44 and development of actin and troponin characteristic in a cardiomyocyte was confirmed.

Fig. 45 is a schematic diagram illustrating a result of a cell tissue being produced in one container included in the plate. Referring to Fig. 45, as illustrated in (A), in the cell seeding method using the micropipette of the related art, cell C spreads to the entire inside of container 112A. However, if the method illustrated in Fig. 43 is used, as illustrated in (B), for example, it is possible to supply first application material A and second application material B to a range having a diameter of 1.0 mm in container 112A having a diameter of 6.5 mm and produce a cell tissue. Further, as illustrated in (C), it is also possible to form a plurality of cell tissues illustrated in (B) in container 112A at intervals from one another. Fig. 46 is a schematic diagram illustrating an example of a plurality of cell tissues formed in a single container as illustrated in (C) in Fig. 45. Referring to Fig. 46, for example, it is also possible to obtain a tissue containing a plurality of (for example, eight) cells C in the circumferential direction in circular container 112A as illustrated in (A). For example, it is also possible to obtain a tissue including a plurality of (for example, four) cells C at corners in square container 112A as illustrated in (B). It is also possible to obtain a larger number of cell tissues as illustrated in (C).

By producing tissues of cells C independently in a plurality of parts in single container 112A as in Fig. 46, drug and pharmacological evaluation and safety evaluation in a drug discovery process can be efficiently performed. For example, when a cell tissue is formed as in Fig. 45(A) without using the method illustrated in Fig. 46, only a single analysis result can be acquired from a cell tissue in container 112A. However, a plurality of analysis results can be acquired from a plurality of cell tissues in container 112A. For this reason, a cell tissue with a high throughput and high evaluation efficiency can be obtained.

Fig. 47 is an example of producing a cell tissue using an application needle having a diameter of 330 μm. Referring to Fig. 47, (A) is a mode immediately after application and (B) is a mode six days after the application. As in Fig. 47, even if a thick application needle is used, it is possible to produce cell tissues at the interval from each other as illustrated in a photograph.

(Action effects)

With the application method according to the present embodiment, the step of supplying, using the first application mechanism (needle application mechanism 104), first application material A1 serving as the application material to the first container included in plate 111 including plurality of containers 112A capable of receiving the application material is performed. The pre-simultaneous supply movement step (S5) in which the stage (for example, X-axis stage 101) on which plate 111 is placed moves is performed. The simultaneous supply step (S3, S21) of supplying, using the first application mechanism (needle application mechanism 104), first application material A1 to the second container disposed at an interval from the first container of plate 111 and, at the same time, supplying second application material B serving as the application material to the first container (for example, container 112A-1) using the second application mechanism (dripping mechanism 105-1) disposed at an interval from the first application mechanism (needle application mechanism 104) is performed. Every time the pre-simultaneous supply movement step and the simultaneous supply step are performed, containers equivalent to the first container and the second container are changed in the moving direction of the stage (the X direction). That is, both of the first container and the second container sequentially move from container 112A-1 to container 112A-2 and container 112A-3.

Fig. 48 is a schematic diagram for comparing timings when the application of the application method in the third embodiment and application of an application method in a comparative example are performed. Referring to Fig. 48, for example, after first application material A1 is supplied to container 112A-1, X-axis stage 101 moves, dripping mechanism 105 is installed right above container 112A-1, and second application material B is supplied to container 112A-1. Thereafter, X-axis stage 101 moves again and needle application mechanism 104 is installed right above container 112A-2 and first application material A1 is supplied to container 112A-2. Thereafter, the moving step and the supply step are repeated in the same manner as explained above. That is, second application material B is supplied to container 112A-2, XY stage 101 moves, first application material A1 is supplied to container 112A-3, XY stage 101 moves, and second application material B is supplied to container 112A-3. In this case, a time T1 required for finishing supplying the application material to all containers 112A increases.

Thus, if the method in the present embodiment is applied, for example, after first application material A1 is supplied to container 112A-1, dripping mechanism 105-1 is installed right above container 112A-1 and needle application mechanism 104 is installed right above container 112A-2 by the pre-simultaneous supply movement step. Then, second application material B and first application material A1 are simultaneously respectively supplied to container 112A-1 and container 112A-2. Thereafter, the pre-simultaneous supply movement step and the simultaneous supply step are repeated and first application material A1 and second application material B are repeatedly simultaneously supplied. In this way, in most of the application processes, simultaneous supply to a plurality of parts is possible. For this reason, time T2 required to finish supplying the application material to all containers 112A can be greatly reduced from time T1 in the comparative example. For example, it is assumed that an application time of first application material A1 is one second, a dripping time of second application material B is one second, and a moving time of XY stage 101 is two seconds. At this time, whereas a time required to finish supplying the application material to five containers 112A is 30 seconds in the comparative example, the time is 18 seconds in the present embodiment.

In the application method, second application material B is supplied to cover first application material A. The interval between the first application mechanism (needle application mechanism 104) and the second application mechanism (dripping mechanism 105-1) (the interval between the centers of respective through-holes 25: first interval D1) is equal to natural number n times (n≥1) of first interval D1 (between the centers in the X direction) between the first container (for example, container 112A-1) of plate 111 and an adjacent container adjacent to first container 112A-1. In the third embodiment, since the adjacent container of container 112A-1 is container 112A-2 and the application material is supplied to both of the containers adjacent to each other in the X direction, n=1. Since the application method has such characteristics, as explained above, by moving XY stage 101 by first interval D1 after first application material A1 is supplied to container 112A-1 by needle application mechanism 104, it is possible to perform the simultaneous supply step to container 112A-2 and container 112A-1. For this reason, it is possible to reduce an entire cell tissue production time and produce a cell tissue with a high throughput.

Application device 100 according to the present embodiment includes application mechanism 107 capable of supplying an application material (first application material A1 and second application material B) that is to be applied and the stage (XY stage 101) on which plate 111 to which the application material is to be supplied can be placed. Application mechanism 107 includes the first application mechanism (needle application mechanism 104) and the second application mechanism (dripping mechanism 105-1) adjacent to the first application mechanism. The interval (first interval D1) between the first application mechanism (needle application mechanism 104) and the second application mechanism (dripping mechanism 105-1) is equal to a movement amount of XY stage 101 between a first application time (the application time of first application material A1 to container 112A-1 in the above explanation) and a second application time (the simultaneous supply step time of the application of first application material A1 to container 112A-2 and the dripping of second application material B to container 112A-1) by application mechanism 107 (first interval D1). Since application device 100 has such characteristics, by moving XY stage 101 by first interval D1 after supplying first application material A1 to container 112A-1 with needle application mechanism 104 as explained above, it is possible to perform the simultaneous supply step to container 112A-2 and container 112A-1.

(Fourth embodiment)

(Configuration of the application device)

Fig. 49 is a schematic front view illustrating an example of the application device according to the fourth embodiment. Referring to Fig. 49, application device 100 according to the fourth embodiment has basically the same configuration as the configuration of application device 100 according to the examples of the third embodiment. Therefore, the same components are denoted by the same reference numerals and signs and explanation is not repeated as long as functions and the like are the same. Application device 100 in Fig. 49 includes two needle application mechanisms 104 and two dripping mechanisms 105. Two needle application mechanisms 104 are needle application mechanism 104-1 and needle application mechanism 104-2. Although two needle application mechanisms 104 and two dripping mechanisms 105 are respectively arranged side by side at intervals in the X direction, the mutual X-direction intervals are fixed and do not change. The X-direction intervals are equal to the X-direction interval in application device 100 in Fig. 34. Application device 100 may have such a configuration.

Fig. 50 is a flowchart illustrating an application method in the fourth embodiment. Referring to Fig. 50, first, a Z axis of an application mechanism is lowered (S1).

Fig. 51 is a schematic sectional view illustrating a part of an application mechanism and an installed plate of an application device used for the application method in the fourth embodiment. Referring to Fig. 51, explanation is not repeated about the same matters as the matters in Fig. 38. According to a step (S1), needle application mechanism 104-1 (a first application mechanism), needle application mechanism 104-2 (another first application mechanism), and dripping mechanism 105 (a second application mechanism) in Fig. 51 are lowered. Needle application mechanism 104-2 is disposed between needle application mechanism 104-1 and dripping mechanism 105 (105-1). Needle application mechanism 104-2 includes another first application material container 21A-2 in which another first application material A2 is stored. In an initial state, the distal end portion of second application needle 24A-2 is immersed in the other first application material A2. The other first application material A2 may be different from first application material A1 in first application material container 21A-1 in a kind of a cell to be suspended. Through-hole 25 for supplying an application material is formed in the bottom of the other first application material container 21A-2. The interval between the centers of through-holes 25 of the application mechanisms is the interval between the application mechanisms.

Referring to Fig. 50 again, subsequently, first application needle 24A-1 is lowered (S2). Fig. 52 is a schematic sectional view illustrating a first step of the application method in the fourth embodiment. Referring to Fig. 50 and Fig. 52, a step (S2) to a step (S4) are basically the same as the steps in Fig. 39.

Fig. 53 is a schematic sectional view illustrating a second step of the application method in the fourth embodiment. Referring to Fig. 50 and Fig. 53, a step (S5) is also basically the same as the step in Fig. 40. That is, a step in which XY stage 101 on which plate 111 is placed moves to the right side in the X direction by D1 as indicated by an arrow M in the figure is performed.

Here, referring to Fig. 51 again, the interval between (the center of through-hole 25 of) needle application mechanism 104-1 and (the center of through-hole 25 of) needle application mechanism 104-2 is equal to a sum D1+D2 of first interval D1 between container 112A-1 and container 112A-2 and a second interval D2 between first application material A1 (a first application material) supplied into container 112A-1 and the other first application material A2 (another first application material) supplied into container 112A-1. For this reason, in Fig. 53, container 112A-2 is disposed in a position planarly overlapping first application needle 24A-1 and container 112A-1 is disposed in a position planarly overlapping second application needle 24A-2. In Fig. 51, the interval between (the center of through-hole 25 of) needle application mechanism 104-1 and (the center of through-hole 25 of) dripping mechanism 105 is equal to a sum of a double of first interval D1 and a half of second interval D2, that is, 2D1+D2/2.

Second interval D2 is, for example, an application interval between the centers in the X direction between first application material A1 and the other first application material A2. However, second interval D2 also indicates a relative position in the X direction of the other first application material A2 with respect to the center position in the X direction of first application material A1. Therefore, for example, in Fig. 53, since the other first application material A2 is present further on the right side (the positive side in the X direction) than first application material A1, a value of D2 is positive. However, for example, although not illustrated, when the other first application material A2 is present further on the left side (the negative side in the X direction) than first application material A1, the value of D2 is negative.

Subsequently, in Fig. 53, the steps (S2) to (S4) are repeated as in Fig. 52. That is, first application material A1 is supplied into container 112A-2. At the same time, the other first application material A2 is supplied into container 112A-1. At this time, second application needle 24A-2 operates like first application needle 24A-1. That is, second application needle 24A-2 falls in a direction of an arrow in the figure (S11). The other first application material A2 is applied by second application needle 24A-2 (S12). Thereafter, second application needle 24A-2 rises (S13). Here, an application interval between the other first application material A2 supplied into container 112A-1 and first application material A1 supplied into container 112A-1 is D2.

Fig. 54 is a schematic sectional view illustrating a third step of the application method in the fourth embodiment. Referring to Fig. 50 and Fig. 54, a pre-simultaneous supply movement step (S5) in which XY stage 101 on which plate 111 is placed moves again to the right side in the X direction by D1 as indicated by an arrow M in the figure is performed. Accordingly, container 112A-3 is disposed in a position planarly overlapping first application needle 24A-1. Container 112A-2 is disposed in a position planarly overlapping second application needle 24A-2. Container 112A-1 is disposed in a position planarly overlapping through-hole 25 of dripping mechanism 105.

Thereafter, in Fig. 54, the steps (S2) to (S4) are repeated as in Fig. 52 and Fig. 53. That is, first application material A1 is supplied into container 112A-3 (first container). At the same time, the steps (S11) to (S13) are repeated as in Fig. 53. That is, the other first application material A2 is supplied into container 112A-2 (a third container). Further, at the same time, the step (S21) is performed as in Fig. 40. That is, second application material B is dripped into container 112A-1 (a second container). As in Fig. 54, the other first application material A2 is supplied to be adjacent to, at interval D2, first application material A1 already supplied in container 112A-2. Second application material B is supplied to cover first application material A1 and the other first application material A2 in container 112A-1.

Fig. 55 is a schematic sectional view illustrating a fourth step of the application method in the fourth embodiment. Referring to Fig. 50 and Fig. 55, it is determined whether first application material A1 has been finished being supplied to all regions to which first application material A1 is to be supplied (S100). If first application material A1 is not supplied yet, the fourth step proceeds from (S100) in the direction of N and, after plate 111 moves by first interval D1 (S5), the same step as the step in Fig. 54 is repeated. In Fig. 55, first application material A1 has been finished being applied to all of container 112A-1 to container 112A-3. Thus, the fourth step proceeds from (S100) in the direction of Y and plate 111 moves again by first interval D1 as indicated by an arrow M in the figure (S5). Accordingly, in Fig. 55, container 112A-3 is disposed in a position planarly overlapping second application needle 24A-2. In Fig. 41, container 112A-2 is disposed in a position planarly overlapping trough-hole 25 of dripping mechanism 105.

Thereafter, in Fig. 55, the steps (S11) to (S13) are repeated as in Fig. 53 and Fig. 54. That is, the other first application material A2 is supplied into container 112A-3. At the same time, the step (S21) is repeated as in Fig. 54. That is, second application material B is dripped into container 112A-2.

Fig. 56 is a schematic sectional view illustrating a fifth step of the application method in the fourth embodiment. Referring to Fig. 50 and Fig. 56, plate 111 moves again by first interval D1 as indicated by an arrow M in the figure (S5). Accordingly, in Fig. 56, container 112A-3 is disposed in a position planarly overlapping through-hole 25 of dripping mechanism 105. Thereafter, as in Fig. 54 and Fig. 55, second application material B is dripped into container 112A-3 (S21). Thereafter, a Z axis of application mechanism 107 rises (S22) and the step ends.

Note that a culture medium is supplied from dripping mechanism 105-2 into containers 112A at appropriate timing after first application material A1 and second application material B are finished being supplied. The culture medium occupies most portions in containers 112A and is supplied such that first application material A1 and second application material B are immersed.

(Action effects)

With the application method according to the present embodiment, in the simultaneous supply step, the step of supplying the other first application material A2 serving as the application material simultaneously with first application material A1 and second application material B is performed. The step of supplying the other first application material A2 is performed to the third container disposed between the first container and the second container using the other first application mechanism (needle application mechanism 104-2) disposed between the first application mechanism (needle application mechanism 104-1) and the second application mechanism (dripping mechanism 105). The other first application material A2 is supplied to the third container to be adjacent to first application material A1. Second application material B is supplied to cover first application material A1 and the other first application material A2. Since the first container and the second container move in the X direction every time as explained above, the third container disposed between the first container and the second container also moves in the X direction.

Fig. 57 is a schematic diagram for comparing timings when application of the application method in the fourth embodiment and application of an application method in a comparative example are performed. Referring to Fig. 57, in the comparative example, for example, after first application material A1 is supplied to container 112A-1, XY stage 101 moves, needle application mechanism 104-2 is installed right above container 112A-1, and the other first application material A2 is supplied to container 112A-1. Thereafter, XY stage 101 moves again, dripping mechanism 105 is installed right above container 112A-1, and second application material B is supplied to container 112A-1. Thereafter, the moving step and the supply step are repeated in the same manner as explained above. That is, first application material A1 is supplied to container 112A-2, XY stage 101 moves, the other first application material A2 is supplied to container 112A-2, XY stage 101 moves, and second application material B is supplied to container 112A-2. In this case, time T1 required to finish supplying the application material to all containers 112A increases.

Thus, if the method in the present embodiment is applied, for example, after first application material A1 is supplied to container 112A-1, needle application mechanism 104-2 is installed right above container 112A-1 and needle application mechanism 104-1 is installed right above container 112A-2 by the pre-simultaneous supply movement step. Then, the other first application material A2 and first application material A1 are respectively simultaneously supplied to container 112A-1 and container 112A-2. Subsequently, second application material B, the other first application material A2, and first application material A1 are respectively simultaneously supplied to container 112A-1, container 112A-2, and container 112A-3 by the pre-simultaneous supply movement step and the simultaneous supply step. Thereafter, the pre-simultaneous supply movement step and the simultaneous supply step are repeated and first application material A1 and second application material B are repeatedly simultaneously supplied. In this way, in most of the application steps, simultaneous supply to a plurality of parts is possible. For this reason, time T2 required to finish supplying the application material to all containers 112A can be greatly reduced from time T1 in the comparative example. For example, an application time of first application material A1 and the other first application material A2 is assumed to be one second, a dripping time of second application material B is assumed to be one second, and a moving time of XY stage 101 is assumed to be two seconds. At this time, whereas a time required to finish supplying the application material to five containers 112A is 45 seconds in the comparative example, the time is 21 seconds in the present embodiment.

In the application method explained above, the interval between the first application mechanism (needle application mechanism 104-1) and the other first application mechanism (needle application mechanism 104-2) is equal to a sum of a natural number n times ($n \geq 1$) of first interval D1 between the first container of plate 111 and an adjacent container adjacent to first container and second interval D2 between the first application material and the other first application material supplied into the first container. In the fourth embodiment, since the adjacent container of container 112A-1 is container 112A-2 and the application material is supplied to both of the containers adjacent to each other in the X direction, n=1. Since the application method has such characteristics, as explained above, it is possible to repeat the pre-simultaneous supply movement step and the simultaneous supply step.

In the present embodiment, the intervals between needle application mechanism 104 and dripping mechanism 105 of application mechanism 107 are determined (designed) considering interval D1 of container 112A and interval D2 in the X direction between first application material A1 and the other first application material A2 to be actually applied. For this reason, simply by moving the stage of container 112A by interval D1 of container 112A at a time at an actual operation time in Fig. 52 to Fig. 56, it is possible to easily apply the application material to a desired position.

(Fifth embodiment)

Fig. 58 is a flowchart illustrating an application method according to a first example of a fifth embodiment. Referring to Fig. 58, first, a Z axis of application mechanism 107 is lowered (S1).

Fig. 59 is a schematic sectional view illustrating a part of an application mechanism and an installed plate of an application device used for the application method according to the first example of the fifth embodiment. Referring to Fig. 59, application device 100 used in the present embodiment is basically the same as application device 100 used in the fourth embodiment. That is, application device 100 in the present embodiment includes two needle application mechanisms 104 and two dripping mechanisms 105. Two needle application mechanisms 104 are needle application mechanism 104-1 and needle application mechanism 104-2. The interval in the X direction between each of two needle application mechanisms 104 and two dripping mechanisms 105 is the same as the interval in the fourth embodiment. That is, in Fig. 59, as in Fig. 51, the interval between (the center of through-hole 25 of) needle application mechanism 104-1 and (the center of through-hole 25 of) needle application mechanism 104-2 is D1+D2. In Fig. 59, as in Fig. 51, the interval between (the center of through-hole 25 of) needle application mechanism 104-1 and (the center of through-hole 25 of) dripping mechanism 105 is equal to a sum of a double of first interval D1 and a half of second interval D2, that is, 2D1+D2/2. In the present embodiment as well, as in the other embodiments, the interval between a pair of containers 112A adjacent to each other is D1.

However, as in Fig. 59, in the present embodiment, an application interval (between the centers in the X direction) between first application material A1 and the other first application material A2 is different from D2 in the fourth embodiment and is D3. In this case, the application step is as explained below.

Fig. 60 is a schematic sectional view illustrating a first step of the application method according to the first example of the fifth embodiment. Referring to Fig. 58 and Fig. 60, as in Fig. 52, first application material A1 is supplied to container 112A-1 by first steps (S2) to (S4). Subsequently, as in Fig. 53, a step in which XY stage 101 on which plate 111 is placed moves to the right side in the X direction by D1 as indicated by an arrow M in the figure is performed by the step (S5). Thereafter, the steps (S2) to (S4) are performed again and first application material A1 is supplied to container 112A-2.

Fig. 61 is a schematic sectional view illustrating a second step of the application method according to the first example of the fifth embodiment. Referring to Fig. 58 and Fig. 61, subsequently, plate 111 moves to the right side in the X direction by (D2-D3) (S6). This is based on the difference in the distance of the other first application material A2 between a case in which the other first application material A2 is applied such that the application interval is D2 as in the fourth embodiment and a case in which the other first application material A2 is applied such that the application interval is D3 as in the present embodiment. Thereafter, second application needle 24A-2 falls (S11), first application material A2 is applied (S12), and second application needle 24A-2 rises (S13). Accordingly, third interval D3 is set to, for example, a positive value smaller than second interval D2 in the fourth embodiment such that the interval in the X direction between the centers is third interval D3.

Like second interval D2, third interval D3 also indicates a relative position in the X direction of the other first application material A2 with respect to the center position in the X direction of first application material A1. Therefore, for example, third interval D3 is a positive value in Fig. 61 but, for example, although not illustrated, the value of D3 is negative when the other first application material A2 is present further on the left side (the negative side in the X direction) than first application material A1. D3 may take a positive value larger than D2. As explained above, third interval D3 is a value that can be optionally selected. For this reason, although a moving direction of plate 111 is the right direction in the X direction in the figures, the moving direction may be the left direction in the X direction.

Fig. 62 is a schematic sectional view illustrating a third step of the application method according to the first example of the fifth embodiment. Referring to Fig. 58 and Fig. 62, subsequently, plate 111 moves to the right side in the X direction by (D1-D2+D3) (S7). This is based on the following reason. In both of the insides of container 112A-2 and container 112A-3, it is necessary to supply first application material A1 to the same position in the X direction. For this reason, originally, plate 111 should move by D1 in a period from the application step of first application material A1 to container 112A-2 to the application step of first application material A1 to container 112A-3. However, this is because, since plate 111 has already moved by (D2-D3) in Fig. 61, plate 111 should move by D1-(D2-D3) that is the difference between D1 and (D2-D3).

Thereafter, in Fig. 62, as in Fig. 40, the steps (S2) to (S4) and the step (S21) are simultaneously performed. Accordingly, first application material A1 and second application material B are simultaneously supplied. Specifically, first application material A1 is supplied to container 112A-3 and second application material B is supplied to container 112A-1. Second application material B is supplied to cover first application material A1 and the other first application material A2.

Fig. 63 is a schematic sectional view illustrating a fourth step of the application method according to the first example of the fifth embodiment. Referring to Fig. 58 and Fig. 63, subsequently, as in Fig. 61, plate 111 moves to the right side in the X direction by (D2-D3) (S6) and the other first application material A2 is supplied to container 112A-2 by steps (S11) to (S13).

Referring to Fig. 58, as in the embodiments explained above, according to the determination in the step (S100), while the stage moving, the steps in Fig. 62 and Fig. 63 are repeated until container 112A to which first application material A1 is to be applied is absent.

Fig. 64 is a flowchart illustrating an application method according to a second example of the fifth embodiment. Referring to Fig. 64, in this example, first, as in the steps (S1) to (S4) illustrated first in Fig. 50 and Fig. 52 in the fourth embodiment, first application material A1 is supplied. Subsequently, as in the following step (S5), steps (S2) to (S4), and steps (S11) to (S13) in Fig. 50 and Fig. 53, first application material A1 and the other first application material A2 are simultaneously supplied. Thereafter, for example, plate 111 is moved by a distance D4 (S8) and, thereafter, second application material B is supplied to, for example, container 112A-1 in Fig. 53 (S21). This is, for example, a method used when it is desired to simultaneously supply first application material A1 and the other first application material A2 as in the fourth embodiment but supply only second application material B to a position deviating from the supply position in the fourth embodiment in the X direction by a fourth interval D4. After plate 111 moves to the right side in the X direction by (D1-D4) (S9), when the application method proceeds in the direction of N in a step (S100), the steps explained above are repeated.

In the second example in Fig. 64, as explained above, only second application material B is supplied at timing different from timing when first application material A1 and the other first application material A2 are supplied. The second example is different at this point from the first example in Fig. 58 to Fig. 63 in which only the other first application material A2 is supplied at timing different from timing when first application material A1 and second application material B are supplied. As in Fig. 64, even when only second application material B is dripped to a position different from the position in the fourth embodiment, it is possible to drip second application material B to any position by controlling a movement amount of plate 111 as explained above. Although not explained above, for example, it is also possible to simultaneously supply the other first application material A2 and second application material B and supply only first application material A1 to any position by separately moving plate 111 by any movement amount at the supply time of first application material A1.

(Action effects)

With the application method according to the present embodiment, second application material B is supplied to cover the first application material (first application material A1 and the other first application material A2). In the pre-simultaneous supply movement step (steps (S6) and (S7) in Fig. 58), it is possible to select any movement amount of XY stage 101 considering the third interval D3 between first application material A1 that is to be supplied into the first container and the other first application material A2 supplied from the other first application mechanism (needle application mechanism 104-2). That is, (D2-D3) in the step (S6) in Fig. 58 and Fig. 61 and (D1-D2+D3) in the step (S7) in Fig. 58 and Fig. 62 are values selected according to any interval D3 in container 112A between first application material A1 and the other first application material A2.

In the present embodiment, the interval between needle application mechanism 104 and dripping mechanism 105 of application mechanism 107 is determined (designed) to be value D2 unrelated to interval D3 in the X direction between first application material A1 and the other first application material A2 actually about to be applied. For this reason, as in Fig. 58 to Fig. 63, only first application material A1 and second application material B are simultaneously supplied and, since a position needs to be separately adjusted for the other first application material A2, the other first application material A2 is supplied at timing different from timing when first application material A1 and second application material B are supplied. However, if the application position is adjusted by XY stage 101 using a slight time, at least an effect of a time reduction by the simultaneous supply of first application material A1 and second application material B can be expected. If the application position is adjusted using a slight time, the distance between first application material A1 and the other first application material A2 can be set to any value such as D3.

In the fourth embodiment, second application material B is supplied to the position in the center between first application material A1 and the other first application material A2. The term of D2/2 in the interval 4D1+D2/2 between needle application mechanism 104-1 and dripping mechanism 105 in application mechanism 107 is determined considering the fact. However, in the fifth embodiment, as in Fig. 62, second application material B is supplied to the position slightly deviating to the right side from the center between first application material A1 and the other first application material A2. This is because, in the fifth embodiment, although the position of the center between first application material A1 and the other first application material A2 is different from the position in the fourth embodiment, application mechanism 107 in the fifth embodiment is the same as application mechanism 107 in the fourth embodiment. However, since second application material B is supplied to a wide range to cover entire first application material A, a serious problem does not occur even if the position of the center deviates as in Fig. 62.

Note that, when application device 100 includes an interval adjustment mechanism capable of changing the interval between needle application mechanism 104-1 and dripping mechanism 105 in application mechanism 107 from 4D1+D2/2, in Fig. 62, second application material B can be supplied to the center between first application material A1 and the other first application material A2. Specifically, the interval adjustment mechanism may be electric or may be manual. As the manual interval adjustment mechanism, for example, a slide mechanism capable of fixing the position to a base plate material with a screw while shifting the position. Alternatively, as the manual interval adjustment mechanism, a linear motion guide (a rolling guide, a slide guide, or the like) with a stopper may be used. As the electric interval adjustment mechanism, for example, a linear motion guide or a small electric stage incorporating a ball screw may be used.

In the application method explained above, the interval between the first application mechanism (needle application mechanism 104-1) and the other first application mechanism (needle application mechanism 104-2) is any value equal to or larger than natural number n times (n≥1) of first interval D1 between the first container of plate 111 and the adjacent container adjacent to the first container and equal to or smaller than a sum of an assumable maximum value of third interval D3 and the natural number n times of first interval D1. The assumable maximum value of third interval D3 is, for example, an X-direction dimension of individual container 112A. In the fifth embodiment, since the adjacent container of container 112A-1 is container 112A-2 and the application material is supplied to both of the containers adjacent to each other in the X direction, n=1. Accordingly, if third interval D3 is optionally determined, it is possible to produce a three-dimensional cell tissue having any third interval D3 by optionally determining a movement amount of XY stage 101 according to third interval D3.

(Sixth embodiment)

Fig. 65 is a plan view illustrating a mode of an application material formed in one container in a sixth embodiment. Referring to Fig. 65, here, first application material A1, the other first application material A2, and still another first application material A3 are applied in container 112A as a first application material. These first application materials are covered by second application material B.

In the third to third embodiments explained above, the explanation is made assuming that XY stage 101 moves in the X direction with the X-axis stage (the mechanism for moving XY stage 101 in the X direction) not illustrated in Fig. 33 and the like. However, actually, a plurality of containers 112A are arranged not only in the X direction but also in the Y direction of plate 111. For this reason, it is more preferable to combine an operation in the Y direction of XY stage 101 by a Y-axis stage to the operation in the X direction of plate 111 explained in the third to third embodiments. Accordingly, as in Fig. 65, it is possible to form a three-dimensional cell tissue in which the first application material is applied to be arranged not only in the X direction but also in the Y direction in one container 112A. In Fig. 65, first application material A1, the other first application material A2, and second application material B are supplied by only the X-direction movement of XY stage 101 as in the fourth and third embodiments. For this reason, the centers of these application materials are linearly arranged side by side in the X direction. In contrast, only the other first application material A3 is applied further on the positive side in the Y direction than the other application materials. This is because the other first application material A3 is supplied when XY stage 101 is moved in the Y direction with respect to the supply time of first application material A1 and the like.

(Seventh embodiment)

Fig. 66 is a schematic sectional view illustrating a first example of a part of an application mechanism and an installed plate of an application device used for an application method in a seventh embodiment. Referring to Fig. 66, application mechanism 107 of application device 100 in the first example of the seventh embodiment is entirely the same as application mechanism 107 of application device 100 illustrated in Fig. 38. However, in Fig. 66, an interval of containers 112A formed in plate 111 to be used is different. Specifically, container 112A-1 and container 112A-2 to which a liquid material is to be supplied are not adjacent to each other and one container that is adjacent to container 112A-1 and to which the liquid material is not supplied is disposed between container 112A-1 and container 112A-2. First interval D1 in the X direction between the centers of containers adjacent to each other is a half of the interval (between the centers of through-holes 25) between needle application mechanism 104 and dripping mechanism 105. That is, an inter-center distance of the containers in Fig. 66 is smaller than the inter-center distance in Fig. 38. For this reason, from the viewpoint of comparison with Fig. 38, actually, the interval between needle application mechanism 104 and dripping mechanism 105 is D1 and the interval between the centers of containers 112A is D1/2. However, here, for convenience of explanation, the former is represented as 2D1 and the latter is represented as D1 (the same applies to Figs. 67 and 36 referred to below).

Fig. 67 is a schematic sectional view illustrating a second example of the part of the application mechanism and the installed plate of the application device used for the application method in the seventh embodiment. Referring to Fig. 67, application mechanism 107 of application device 100 in the second example of the seventh embodiment is entirely the same as application mechanism 107 of application device 100 illustrated in Fig. 51. However, in Fig. 67, as in Fig. 66, container 112A-1 and container 112A-2 to which a liquid material is to be supplied are not adjacent to each other and one container to which the liquid material is not supplied is provided between container 112A-1 and container 112A-2. That is, an inter-center distance of the containers in Fig. 67 is smaller than the inter-center distance in Fig. 51. For this reason, if an inter-center distance of containers 112A is represented as D1, the interval between (the center of through-hole 25 of) needle application mechanism 104-1 and (the center of through-hole 25 of) needle application mechanism 104-2 is equal to a sum of a double of D1 and D2, that is, 2D1+D2. Accordingly, in Fig. 67, the interval between (the center of through-hole 25 of) needle application mechanism 104-1 and (the center of through-hole 25 of) dripping mechanism 105 is equal to a sum of a quadruple of first interval D1 and a half of second interval D2, that is, 4D1+D2/2.

Fig. 68 is a schematic sectional view illustrating a third example of the part of the application mechanism and the installed plate of the application device used for the application method in the seventh embodiment. Referring to Fig. 68, application mechanism 107 and plate 111 of application device 100 in the third example of the seventh embodiment are completely the same as those illustrated in Fig. 59. However, in Fig. 68, as in Fig. 66, container 112A-1 and container 112A-2 to which a liquid material is to be supplied are not adjacent to each other and one container to which the liquid material is not supplied is provided between container 112A-1 and container 112A-2. That is, an inter-center distance of the containers in Fig. 68 is smaller than the inter-center distance in Fig. 59. In Fig. 68, as in Fig. 59 (the fifth embodiment), interval D3 between first application material A1 and the other first application material A2 applied in containers 112A is optionally decided independently of the distance between mechanisms in application mechanism 107.

(Action effects)

In the application method in the first example (Fig. 66) in the present embodiment, second application material B is supplied to cover first application material A. The interval between the first application mechanism (needle application mechanism 104) and the second application mechanism (dripping mechanism 105-1) (the interval between the centers of respective through-holes 25: first interval D1) is equal to natural number n times (n≥1) of first interval D1 (between the centers in the X direction) between the first container (for example, container 112A-1) of plate 111 and the adjacent container adjacent to the first container 112A-1. In the first example of the seventh embodiment, since the adjacent container of container 112A-1 is not container 112A-2 and the application material is intermittently supplied to one of containers 112A arranged side by side in the X direction at a time, n=2. Therefore, for example, when the application material is intermittently supplied to two of containers 112A at a time, n=3 and, when the application material is intermittently supplied to (k-1) of containers 112A at a time, n=k. Since the application method has such characteristics, as explained above, after first application material A1 is supplied to container 112A-1 with needle application mechanism 104, by moving XY stage 101 by, for example, a double of first interval D1, it is possible to perform the simultaneous supply step to container 112A-2 and container 112A-1.

In the application method in the second example (Fig. 67) of the present embodiment, the interval between the first application mechanism (needle application mechanism 104-1) and the other first application mechanism (needle application mechanism 104-2) is equal to a sum of natural number n times (n≥1) of first interval D1 between the first container of plate 111 and the adjacent container adjacent to the first container and second interval D2 between the first application material and the other first application material supplied into the first container. In the second example of the seventh embodiment, since the adjacent container of container 112A-1 is not container 112A-2 and the application material is intermittently supplied to one of containers 112A arranged side by side in the X direction at a time, n=2. As in the first example explained above, when the application material is intermittently supplied to (k-1) of containers 112A at a time, n=k. Since the application method has such characteristics, as explained above, it is possible to repeat the pre-simultaneous supply movement step and the simultaneous supply step.

In the application method in the third example (Fig. 68) of the present embodiment, the interval between the first application mechanism (needle application mechanism 104-1) and the other first application mechanism (needle application mechanism 104-2) is any value equal to or greater than natural number n times (n≥1) of first interval D1 between the first container of plate 111 and the adjacent container adjacent to the first container and equal to or smaller than a sum of an assumable maximum value of third interval D3 and the natural number n times of first interval D1. As in Fig. 66 and Fig. 67, in Fig. 68, n=2 but, when the application material is intermittently supplied to (k-1) of containers 112A at a time, n=k. The assumable maximum value of third interval D3 is, for example, an X-direction dimension of individual container 112A. Accordingly, if third interval D3 is optionally determined, it is possible to produce a three-dimensional cell tissue having any third interval D3 by optionally determining a movement amount of XY stage 101 according to third interval D3.

As plate 111, besides a plate in which ninety-six containers 112A are formed (ninety-six wells: 8×12 wells), there are a plate in which six containers 112A are formed (six wells: 2×3 wells), a plate in which twelve containers 112A are formed (twelve wells: 3×4 wells), a plate in which twenty-four containers 112A are formed (twenty-four wells: 4×6 wells), a plate in which forty-eight containers 112A are formed (forty-eight wells: 6×8 wells), a plate in which 384 containers 112A are formed (384 wells: 16×24 wells), and a plate in which 1536 containers 112A are formed (1536 wells: 32×48 wells). First interval D1 between general adjacent containers 112A is 39 mm in the six wells, 26 mm in the twelve wells, 18 mm in the twenty-four wells, 13 mm in the forty-eight wells, 9 mm in ninety-six wells, 4.5 mm in the 384 wells, and 2.25 mm in the 1536 wells. In this way, interval D1 decreases as the number of wells of plate 111 increases. For this reason, if interval D1 decreases, since a space is extremely narrow, needle application mechanism 104 and dripping mechanism 105 sometimes cannot be installed in the space. In this case, as in the present embodiment, it is preferable to set natural numbers times of interval D1 between wells as a new space. Accordingly, needle application mechanism 104 and dripping mechanism 105 can be installed in the new space.

In particular, in the present embodiment, the width in the X direction of container 112A is smaller compared with the other embodiments. For this reason, two application materials sometimes cannot be applied to have interval D2 in one container as in Fig. 67. In such a case, a method of applying the application materials to have interval D3 smaller than D2 as in Fig. 68 is useful.

(Other modifications for the embodiments)

In the third to seventh embodiments explained above, modifications explained below may be applied.

1) In the third to seventh embodiments, application needle 24 is used to apply first application material A (A1, A2) and dripping mechanism 105 (an air type or cylinder type dispenser) is used to drip second application material B. However, conversely, first application material A (A1, A2) may be supplied by the dripping mechanism and second application material B may be supplied by application needle 24. Alternatively, at least one of first application material A and second application material B may be supplied by an inkjet device or a micropump. The inkjet device is capable of fine dripping and is excellent in rapidity. On the other hand, the micropump is inexpensive as a device and is excellent in a large volume of dripping. On the other hand, application using application needle 24 can eliminate nozzle clogging concerned when the inkjet device is used and can be applied to a wide variety of kinds of liquid materials. Further, fine application is possible by application needle 24. A dispenser is applicable to wider varieties of kinds of liquid materials than the inkjet device and is suitable for dripping with a relatively large volume. It is preferable to properly use or combine the various liquid material supply devices as appropriate making use of the characteristics of the liquid material supply devices as explained above. Then, a high-quality cell tissue is efficiently produced.

2) In the third to seventh embodiments, when the application material is supplied by application needle 24 (24A-1, 24A-2), it is necessary to keep constant the distances in the Z direction between the distal end portion of application needle 24 and flat culture surfaces in containers 112A (the bottom surfaces of containers 112A). This is because the distances affect a liquid amount and a diameter of the supplied application material. For this reason, it is preferable to, before the application step, measure the distances in the Z direction between culture surfaces of at least a part of a large number of containers 112A of plate 111 and the distal end portion of application needle 24 in an initial state and cause application device 100 to store the distances. Then, when the application material is applied in the measured containers 112A, it is preferable to adjust a Z-axis stage and keep constant the distances between the culture surfaces and the distal end of application needle 24. Note that the measurement of the distances in the Z direction between the culture surfaces and the distal end portion of application needle 24 may be performed by a laser beam or an infrared ray or may be performed by a stereo microscope or a phase-contrast microscope. A device that outputs a laser beam or an infrared ray, the stereo microscope, the phase-contrast microscope, or the like may be built in application device 100.

3) In the third to seventh embodiments, a cell tissue including a cell is used for the application materials. However, not only this, but, for example, a general biomaterial or a chemical material may be used for the application materials.

4) In the third to seventh embodiments, as a type of a cell contained in first application material A1, a cell in which a cardiomyocyte derived from an iPS cell and a human cardiac fibroblast are mixed at a number ratio of 3:1 is used. However, another cell may be used. The other cell is also applied to a plurality of parts on a flat culture surface of individual container 112A using application device 100. Accordingly, a cell tissue that is independently present and has less variation in size is produced in a simple step while an overall number of cells being reduced (see Fig. 45(C)).

5) As a combination of the gelation initiator contained in first application material A (first application materials A1 and A2, the same applies below) and the gelling agent contained in second application material B, besides the thrombin and the fibrinogen explained above, calcium chloride and sodium alginate, calcium chloride and carrageenan, alcohols and tamarind seed gum, or the like may be used. Note that, conversely to the above, in the present embodiment, the gelling agent may be contained in first application material A and the gelation initiator may be contained in second application material B.

6) In the third to seventh embodiments, an example is explained in which the cell that is to be cultured and the gelation initiator are contained as first application material A1 (or first application material A2, the same applies below) and the gelling agent is contained as second application material B. However, as an alternative example, the cell and the gelling agent may be contained as first application material A1 and the gelation initiator may be contained as second application material B. According to the alternative example, a solid gel in which the cell is embedded is formed in a short time. For this reason, it is possible to produce a cell tissue having high shape stability and having a shape more similar to a target shape (in particular, a target plane shape).

7) A so-called two-liquid mixed type gelling agent may be used as first application material A1. Note that the two-liquid mixed type gelling agent means, for example, a liquid agent gelled after mixing thrombin, which is a gelation initiator, with fibrinogen, which is a gelling agent. However, for first application material A1, a temperature dependent type, a photocuring type, or the like may be used instead of the two-liquid mixing type. As a gelling agent of the temperature dependent type, there are collagen, gelatin, gellan gum, agarose, chitosan, and the like. As a gelling agent of the photocuring type, there are photo crosslinked gelatin and the like. A type of the gelling agent used in the application material is appropriately selected according to characteristics of the cell and the application material. In particular, when the gelling agent of the temperature dependent type or the photocuring type is used, second application material B may not be used. Alternatively, as second application material B, a material showing biocompatibility represented by a polysaccharide thickener such as methylcellulose, sodium hyaluronate, or sodium alginate may be used.

Accordingly, firmness after the application is ensured.

The characteristics described in the embodiments (the examples) explained above may be applied to be combined as appropriate in a range without technical contradiction.

The embodiments disclosed herein should be considered as illustrative and not limiting in all aspects. The scope of the present invention is indicated not by the above explanation but by the claims. It is intended that all changes in meanings and ranges equivalent to the claims are included in the scope of the present invention.

Various aspects of the present disclosure are collectively described as notes.

(Note 1)

A cell tissue production method comprising:

supplying N (a natural number of N≥1) first application solutions into an application target object and disposing a cell; and

supplying M (a natural number of M≥1) second application solutions into the application target object to cover at least one among the N first application solutions, wherein

in the supplying the second application solutions, when a first dimension, which is a maximum value of a dimension from a center to an outer edge of each of the N first application solutions, is represented as $r_{1n}$ (n is a natural number of 1≤n≤N), a second dimension, which is a maximum value of a dimension from a center to an outer edge of each of the M second application solutions, is represented as $r_{2m}$ (m is a natural number of 1≤m≤M), and a distance between a center of any first application solution, a first dimension of which is $r_{1n}$, selected out of the N first application solutions and a center of any second application solution, a second dimension of which is $r_{2m}$, selected out of the M second application solutions is represented as $D_{n,m}$, the any first application solution and the any second application solution are supplied such that one of

(Math. 1)

$$D_{n,m} < r_{2m} - r_{1n} \quad \dots (1)$$

and

(Math. 2)

$$D_{n,m} > r_{2m} + r_{1n} \quad \dots (2)$$

holds between the any first application solution and the any second application solution.

(Note 2)

The cell tissue production method described in the note 1, wherein the second dimension $r_{2m}$ is larger than a reference dimension equal to or larger than a first virtual dimension $r_{1v}$ that is a maximum dimension of a first virtual curve calculated to be centered on a first centroid of one or more of the first application solutions overlapping the second application solution among the N first application solutions, to cover all of the one or more first application solutions, and to be inscribed with any one of the one or more first application solutions, and the first centroid is calculated from a center position of each of the one or more first application solutions overlapping the second application solution.

(Note 3)

The cell tissue production method described in the note 1 or the note 2, wherein a third dimension $r_3$ of the application target object is larger than a reference dimension equal to or larger than a second virtual dimension $r_{2v}$ that is a maximum dimension of a second virtual curve calculated to be centered on a second centroid of the M second application solutions, to cover all of the M second application solutions, and to be inscribed with any one of the M second application solutions, and the second centroid is calculated from a center position of each of the M second application solutions.

(Note 4)

The cell tissue production method described in any one of the notes 1 to 3, wherein the cell contained in at least one of the N first application solutions supplied into the application target object is a first cell, and the cell contained in at least another one of the first application solutions different from the at least one first application solution is a second cell.

(Note 5)

The cell tissue production method described in any one of the notes 1 to 4, wherein the cell is contained in at least one of the N first application solutions supplied into the application target object, the cell is not contained and at least any one selected out of a group consisting of cytokine, a drug, an ECM, and an additive factor is contained in at least another one of the first application solutions different from the at least one first application solution.

(Note 6)

The cell tissue production method described in any one of the notes 1 to 5, wherein at least any one selected out of a group consisting of a cytokine, an ECM, and an additive factor is contained in the first application solution.

(Note 7)

The cell tissue production method described in any one of the notes 1 to 6, wherein a cell tissue is formed from the cell supplied into the application target object using a gelling agent in or after the supplying the first application solution.

(Note 8)

The cell tissue production method described in the note 7, wherein the first application solution contains the cell and the gelling agent, and the second application solution is a solution having viscosity higher than viscosity of the gelling agent.

(Norte 9)

The cell tissue production method described in the note 7, wherein the first application solution contains the cell, and the second application solution contains the gelling agent.

(Note 10)

The cell tissue production method described in any one of the notes 1 to 9, wherein the first application solution and the second application solution are supplied into the application target object by a two-liquid mixing scheme.

(Note 11)

The cell tissue production method described in the note 10, wherein the first application solution contains the cell and a gelation initiator, and the second application solution contains a gelling agent.

(Note 12)

The cell tissue production method described in the note 10, wherein the first application solution contains the cell and a gelling agent, and the second application solution contains a gelation initiator.

(Note 13)

The cell tissue production method described in any one of the notes 1 to 12, wherein at least one of collagen and fibrinogen serving as a gelling agent is contained in at least one of the first application solution and the second application solution.

(Note 14)

The cell tissue production method described in any one of the notes 1 to 13, wherein a polysaccharide thickener is mixed in at least one of the first application solution and the second application solution.

(Note 15)

The cell tissue production method described in any one of the notes 1 to 14, wherein the cell is any one selected out of a group consisting of a cardiomyocyte, a neuron, a hepatocyte, a cancer cell, a vascular endothelial cell, and a fibroblast.

(Note 16)

The cell tissue production method described in any one of the notes 1 to 15, wherein a cell volume concentration of the first application solution is 1 vol% or more and 30 vol% or less.

(Note 17)

The cell tissue production method described in any one of the notes 1 to 16, wherein a third cell and a fourth cell different from the third cell are cocultured to be contained as the cell in one first application solution among the N first application solutions.

(Note 18)

The cell tissue production method described in any one of the notes 1 to 17, wherein an application needle scheme is used in the supplying the first application solution.

(Note 19)

The cell tissue production method described in any one of the notes 1 to 18, wherein, in the supplying the second application solution, any one selected out of a group consisting of an application needle scheme, an inkjet scheme, a dispenser scheme, a laser print scheme, and a pipette scheme is used.

(Note 20)

The cell tissue production method described in any one of the notes 1 to 19, wherein, in the supplying the M second application solutions, at least one of the M second application solutions is supplied by a first scheme selected out of a group consisting of an application needle scheme, an inkjet scheme, a dispenser scheme, a laser print scheme, and a pipette scheme, and the other second application solution different from at least one among the M second application solutions is supplied by a second scheme different from the first scheme.

(Note 21)

A myocardial tissue production method comprising:

immersing a distal end of an application needle included in an application device in a first application solution to thereby deposit the first application solution at the distal end of the application needle; and
applying, in an application target object, the first application solution deposited at the distal end of the application needle, wherein
the first application solution is a mixed solution of a cell that is to be cultured, collagen serving as a gelling agent, and a first solvent.

(Note 22)

The myocardial tissue production method described in the note 21, further comprising supplying a second application solution into the application target object to cover the first application solution, wherein the second application solution is a mixed solution of a thickener and a second solvent.

(Note 23)

The myocardial tissue production method described in the note 22, wherein the thickener includes one or more substances selected out of a group consisting of cellulose, cellulose nanofiber, chitin, chitosan, chitin nanofiber, methylcellulose, hydroxybutylcellulose, sodium alginate, sodium hyaluronate, polyethylene glycol, gellan gum, carrageenan, pectin, xanthan gum, gelatin, agarose, and polyvinyl alcohol.

(Note 24)

The myocardial tissue production method described in the note 22 or 23, further comprising supplying a culture medium for culturing the cell into the application target object after the supplying the second application solution, wherein

the culture medium is RPMI1640, and
an additive factor to the culture medium is one or more selected out of a group consisting of a B27 (registered trademark) supplement, triiodothyronine, dexamethasone, and an insulin-like growth factor.

(Note 25)

The myocardial tissue production method described in any one of the notes 21 to 24, wherein the collagen is collagen type I-A.

(Note 26)

The cell tissue production method described in any one of the notes 21 to 25, wherein a cell volume concentration of the first application solution is 1 vol% or more and 30 vol% or less.

(Note 27)

The myocardial tissue production method described in any one of the notes 21 to 26, wherein

the cell includes at least a cardiomyocyte,
a ratio of a number of the cardiomyocytes composing the cell is 80% or more, and
a ratio of a number of cardiac fibroblasts composing the cell is 20% or less.

(Note 28)

The myocardial tissue production method described in the note 27, wherein the cardiomyocyte includes at least one of a normal human iPS cell-derived cardiomyocytes and a disease-derived human iPS cell-derived cardiomyocyte.

(Note 101)

An application method comprising:

supplying, using a first application mechanism, a first application material serving as an application material to a first container included in a plate including a plurality of containers capable of receiving the application material;
a pre-simultaneous supply movement step in which a stage on which the plate is placed moves; and
a simultaneous supply step of supplying, using the first application mechanism, the first application material to a second container disposed at an interval from the first container in the plate and, at a same time, supplying a second application material serving as an application material to the first container using a second application mechanism disposed at an interval from the first application mechanism, wherein
every time the pre-simultaneous supply movement step and the simultaneous supply step are performed, the containers equivalent to the first container and the second container are changed in a moving direction of the stage.

(Note 102)

The application method described in the note 101, wherein

the second application material is supplied to cover the first application material, and
an interval between the first application mechanism and the second application mechanism is equal to natural number n times (n≥1) of a first interval between the first container of the plate and an adjacent container adjacent to the first container.

(Note 103)

The application method described in the note 101, wherein

in the simultaneous supply step, supplying another first application material serving as the application material simultaneously with the first application material and the second application material is performed,
the supplying the other first application material is performed to a third container disposed between the first container and the second container using another first application mechanism disposed between the first application mechanism and the second application mechanism,
the other first application material is supplied to the third container to be adjacent to the first application material, and the second application material is supplied to cover the first application material and the other first application material.

(Note 104)

The application method described in the note 103, wherein an interval between the first application mechanism and the other first application mechanism is equal to a sum of natural number n times (n≥1) of a first interval between the first container of the plate and an adjacent container adjacent to the first container and a second interval between the first application material and the other first application material supplied into the first container.

(Note 105)

The application method described in the note 101, wherein

the second application material is suppled to cover the first application material, and
in the pre-simultaneous supply movement step, any movement amount of the stage can be selected considering a third interval between the first application material that is to be supplied into the first container and another first application material serving as the application material supplied from another first application mechanism.

(Note 106)

The application method described in the note 105, wherein an interval between the first application mechanism and the other first application mechanism is any value equal to or larger than natural number n times (n≥1) of a first interval between the first container of the plate and an adjacent container adjacent to the first container and equal to or smaller than a sum of an assumable maximum value of the third interval and natural number n times of the first interval.

(Note 107)

The application method described in any one of the notes 101 to 106, wherein the pre-simultaneous supply movement step is performed by causing the stage to operate.

(Note 108)

The application method described in any one of the notes 101 to 107, wherein the supplying and the simultaneous supply step are performed by causing a Z-axis stage to operate.

(Note 109)

The application method described in any one of the notes 101 to 108, wherein the application material includes a cell that is to be cultured.

(Note 110)

An application device comprising:

an application mechanism capable of supplying an application material that is to be applied; and
a stage on which a plate to which the application material is to be supplied can be placed, wherein
the application mechanism includes a first application mechanism and a second application mechanism adjacent to the first application mechanism, and
an interval between the first application mechanism and the second application mechanism is equal to a movement amount of the stage between a first application time and a second application time by the application mechanism.

REFERENCE SIGNS LIST

1 X-axis table; 2 Y-axis table; 3 Z-axis table; 4, 107 Application mechanism; 6, 106 Observation optical system; 7 CCD camera; 8 Operation panel; 9 Monitor; 10 Control computer; 11 Sample application set; 12 Well; 12a Well bottom; 18 Micropipette; 20 Application needle holder; 21 Application material container; 21A-1 First application material container; 21A-2 Another first application material container; 21B Second application material container; 24 Application needle; 24A-1 First application needle; 24A-2 Second application needle; 35 Movable base; 41 Servomotor; 43 Cam; 44 Bearing; 45 Cam coupling plate; 46 Movable unit; 100 Application device; 101 X-axis stage; 102 Y-axis stage; 103 Z-axis stage; 104, 104-1, 104-2, 104-3 Needle application mechanism; 105, 105-1, 105-2 Dripping mechanism; 106 Observation optical system; 107 Application mechanism; 111 Plate; 112A, 112A-1, 112A-2, 112A-3 Container; 112B Substrate; AA, A1o, A2o First application solution; A, A1 first application material; A2, A3 Another first application material; BB, B1o, B2o Second application solution; B Second application material; C Cell; C1 Cardiomyocyte; C2 Cardiac fibroblast; G1 First centroid; G2 Second centroid; m First solvent; M Culture medium.

## Claims

**1.** A cell tissue production method comprising:

supplying N (a natural number of N≥1) first application solutions into an application target object and disposing a cell; and
supplying M (a natural number of M≥1) second application solutions into the application target object to cover at least one among the N first application solutions, wherein
in the supplying the second application solutions, when a first dimension, which is a maximum value of a dimension from a center to an outer edge of each of the N first application solutions, is represented as $r_{1n}$ (n is a natural number of 1≤n≤N), a second dimension, which is a maximum value of a dimension from a center to an outer edge of each of the M second application solutions, is represented as $r_{2m}$ (m is a natural number of 1≤m≤M), and a distance between a center of any first application solution, a first dimension of which is $r_{ln}$, selected out of the N first application solutions and a center of any second application solution, a second dimension of which is $r_{2m}$, selected out of the M second application solutions is represented as $D_{n,m}$, the any first application solution and the any second application solution are supplied such that one of
(Math. 1)

$$D_{n,m} < r_{2m} - r_{1n} \ldots (1)$$

and
(Math. 2)

$$D_{n,m} > r_{2m} + r_{1n} \ldots (2)$$

holds between the any first application solution and the any second application solution.

**2.** The cell tissue production method according to claim 1, wherein

the second dimension $r_{2m}$ is larger than a reference dimension equal to or larger than a first virtual dimension $r_{1v}$ that is a maximum dimension of a first virtual curve calculated to be centered on a first centroid of one or more of the first application solutions overlapping the second application solution among the N first application solutions, to cover all of the one or more first application solutions, and to be inscribed with any one of the one or more first application solutions, and
the first centroid is calculated from a center position of each of the one or more first application solutions overlapping the second application solution.

3. The cell tissue production method according to claim 1, wherein

a third dimension $r_3$ of the application target object is larger than a reference dimension equal to or larger than a second virtual dimension $r_{2v}$ that is a maximum dimension of a second virtual curve calculated to be centered on a second centroid of the M second application solutions, to cover all of the M second application solutions, and to be inscribed with any one of the M second application solutions, and
the second centroid is calculated from a center position of each of the M second application solutions.

4. The cell tissue production method according to claim 1 or 2, wherein the cell contained in at least one of the N first application solutions supplied into the application target object is a first cell, and the cell contained in at least another one of the first application solutions different from the at least one first application solution is a second cell.

5. The cell tissue production method according to claim 1 or 2, wherein the cell is contained in at least one of the N first application solutions supplied into the application target object, the cell is not contained and at least any one selected out of a group consisting of cytokine, a drug, an ECM, and an additive factor is contained in at least another one of the first application solutions different from the at least one first application solution.

6. The cell tissue production method according to claim 1 or 2, wherein at least any one selected out of a group consisting of a cytokine, an ECM, and an additive factor is contained in the first application solution.

7. The cell tissue production method according to claim 1 or 2, wherein a cell tissue is formed from the cell supplied into the application target object using a gelling agent in or after the supplying the first application solution.

8. The cell tissue production method according to claim 7, wherein the first application solution contains the cell and the gelling agent, and the second application solution is a solution having viscosity higher than viscosity of the gelling agent.

9. The cell tissue production method according to claim 7, wherein the first application solution contains the cell, and the second application solution contains the gelling agent.

10. The cell tissue production method according to claim 1 or 2, wherein the first application solution and the second application solution are supplied into the application target object by a two-liquid mixing scheme.

11. The cell tissue production method according to claim 10, wherein the first application solution contains the cell and a gelation initiator, and the second application solution contains a gelling agent.

12. The cell tissue production method according to claim 10, wherein the first application solution contains the cell and a gelling agent, and the second application solution contains a gelation initiator.

13. The cell tissue production method according to claim 1 or 2, wherein at least one of collagen and fibrinogen serving as a gelling agent is contained in at least one of the first application solution and the second application solution.

14. The cell tissue production method according to claim 1 or 2, wherein a polysaccharide thickener is mixed in at least one of the first application solution and the second application solution.

15. The cell tissue production method according to claim 1 or 2, wherein the cell is any one selected out of a group consisting of a cardiomyocyte, a neuron, a hepatocyte, a cancer cell, a vascular endothelial cell, and a fibroblast.

16. The cell tissue production method according to claim 1 or 2, wherein a cell volume concentration of the first application solution is 1 vol% or more and 30 vol% or less.

17. The cell tissue production method according to claim 1 or 2, wherein a third cell and a fourth cell different from the third cell are cocultured to be contained as the cell in one first application solution among the N first application solutions.

**18.** The cell tissue production method according to claim 1 or 2, wherein an application needle scheme is used in the supplying the first application solution.

**19.** The cell tissue production method according to claim 1 or 2, wherein, in the supplying the second application solution, any one selected out of a group consisting of an application needle scheme, an inkjet scheme, a dispenser scheme, a laser print scheme, and a pipette scheme is used.

**20.** The cell tissue production method according to claim 19, wherein, in the supplying the M second application solutions, at least one of the M second application solutions is supplied by a first scheme selected out of a group consisting of an application needle scheme, an inkjet scheme, a dispenser scheme, a laser print scheme, and a pipette scheme, and the other second application solution different from at least one among the M second application solutions is supplied by a second scheme different from the first scheme.

**21.** An application method comprising:

supplying, using a first application mechanism, a first application material serving as an application material to a first container included in a plate including a plurality of containers capable of receiving the application material;
a pre-simultaneous supply movement step in which a stage on which the plate is placed moves; and
a simultaneous supply step of supplying, using the first application mechanism, the first application material to a second container disposed at an interval from the first container in the plate and, at a same time, supplying a second application material serving as an application material to the first container using a second application mechanism disposed at an interval from the first application mechanism, wherein
every time the pre-simultaneous supply movement step and the simultaneous supply step are performed, the containers equivalent to the first container and the second container are changed in a moving direction of the stage.

**22.** The application method according to claim 21, wherein

the second application material is supplied to cover the first application material, and
an interval between the first application mechanism and the second application mechanism is equal to natural number n times (n≥1) of a first interval between the first container of the plate and an adjacent container adjacent to the first container.

**23.** The application method according to claim 21, wherein

in the simultaneous supply step, supplying another first application material serving as the application material simultaneously with the first application material and the second application material is performed,
the supplying the other first application material is performed to a third container disposed between the first container and the second container using another first application mechanism disposed between the first application mechanism and the second application mechanism,
the other first application material is supplied to the third container to be adjacent to the first application material, and
the second application material is supplied to cover the first application material and the other first application material.

**24.** The application method according to claim 23, wherein an interval between the first application mechanism and the other first application mechanism is equal to a sum of natural number n times (n≥1) of a first interval between the first container of the plate and an adjacent container adjacent to the first container and a second interval between the first application material and the other first application material supplied into the first container.

**25.** The application method according to claim 21, wherein

the second application material is suppled to cover the first application material, and
in the pre-simultaneous supply movement step, any movement amount of the stage can be selected considering a third interval between the first application material that is to be supplied into the first container and another first application material serving as the application material supplied from another first application mechanism.

**26.** The application method according to claim 25, wherein an interval between the first application mechanism and the other first application mechanism is any value equal to or larger than natural number n times ($n \geq 1$) of a first interval between the first container of the plate and an adjacent container adjacent to the first container and equal to or smaller than a sum of an assumable maximum value of the third interval and natural number n times of the first interval.

**27.** The application method according to any one of claims 21 to 26, wherein the pre-simultaneous supply movement step is performed by causing the stage to operate.

**28.** The application method according to claim 21 or 22, wherein the supplying and the simultaneous supply step are performed by causing a Z-axis stage to operate.

**29.** The application method according to claim 21 or 22, wherein the application material includes a cell that is to be cultured.

**30.** An application device comprising:

an application mechanism capable of supplying an application material that is to be applied; and
a stage on which a plate to which the application material is to be supplied can be placed, wherein
the application mechanism includes a first application mechanism and a second application mechanism adjacent to the first application mechanism, and
an interval between the first application mechanism and the second application mechanism is equal to a movement amount of the stage between a first application time and a second application time by the application mechanism.

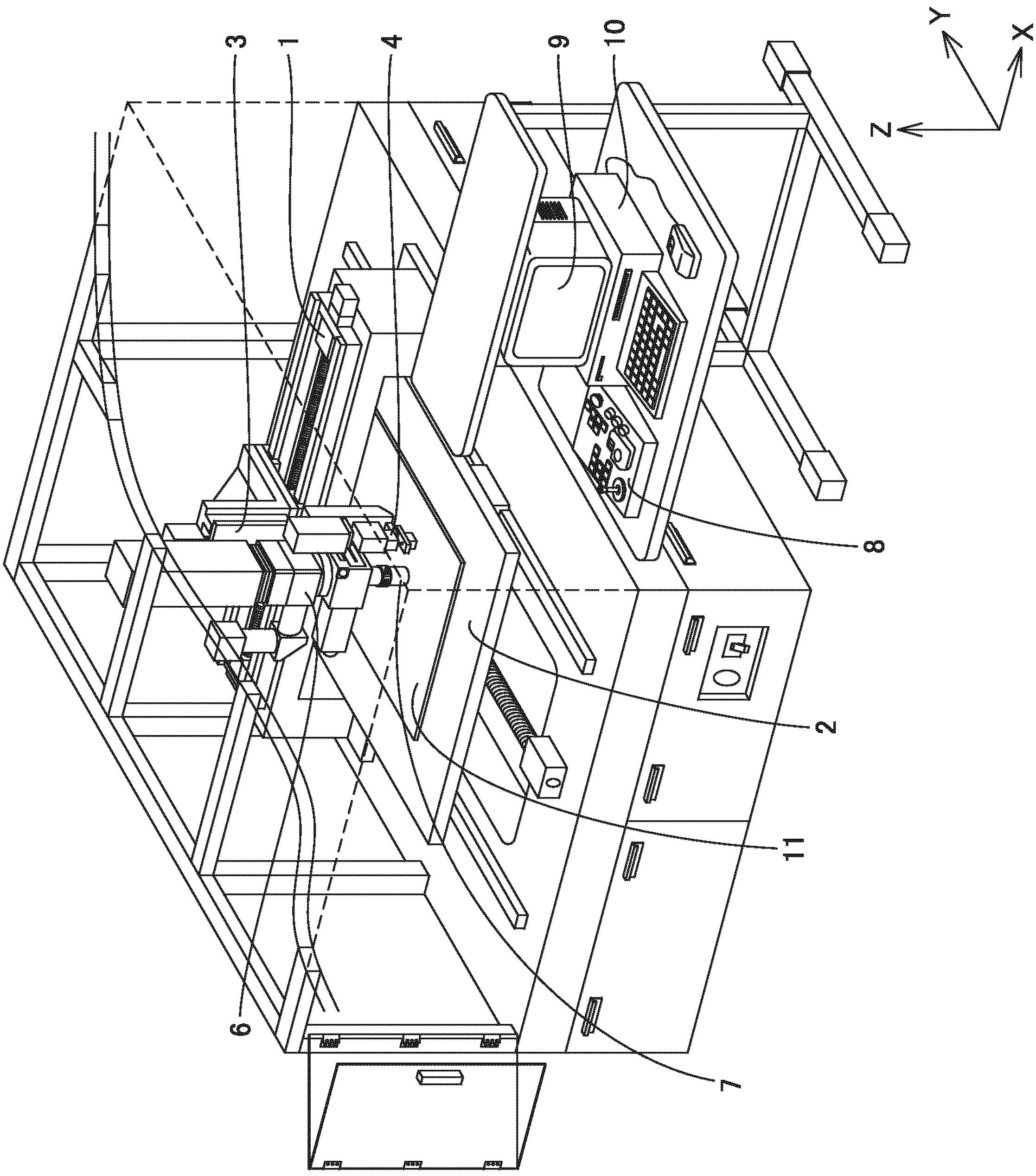


FIG.1

FIG.2

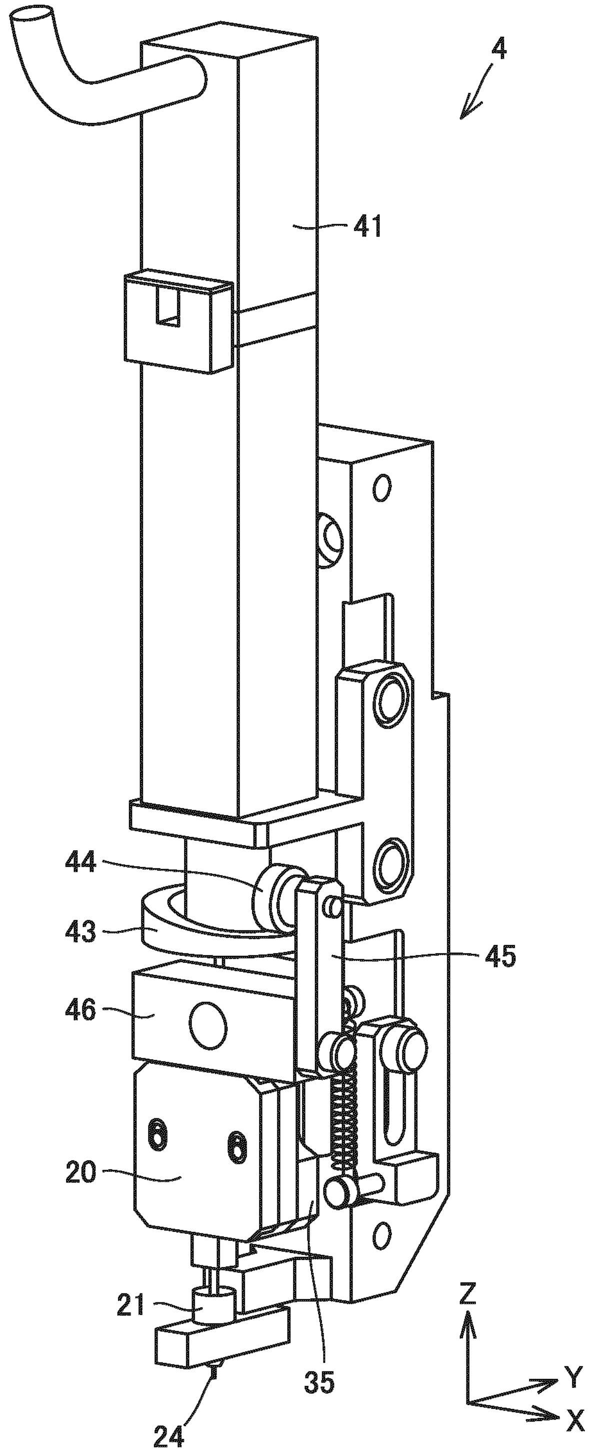

FIG.3

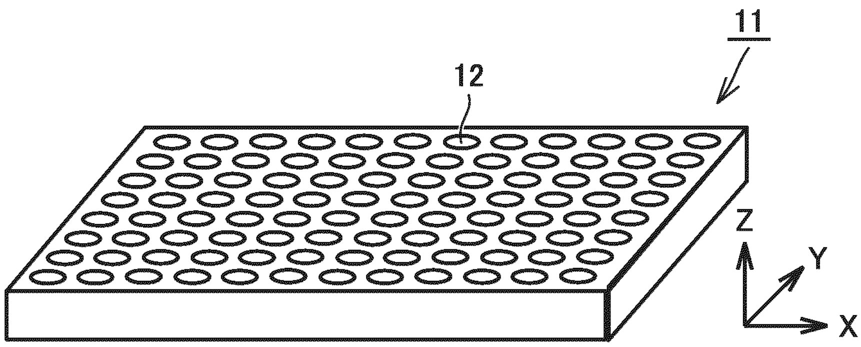

FIG.4

(A) FIRST APPLICATION SOLUTION HAVING FIRST DIMENSION $r_{1n}$ $(1 \leq n \leq N)$

(B) FIRST VIRTUAL CURVE HAVING MINIMUM DIMENSION $r_{1v}$ CENTERED ON FIRST CENTROID CALCULATED FROM CENTERS OF ALL FIRST APPLICATION SOLUTIONS THAT IS TO BE COVERED BY ONE SECOND APPLICATION SOLUTION

(C) SECOND APPLICATION SOLUTION HAVING SECOND DIMENSION $r_{2m}$ $(1 \leq m \leq M)$

(D) SECOND VIRTUAL CURVE HAVING MINIMUM DIMENSION $r_{2v}$ CENTERED ON SECOND CENTROID CALCULATED FROM CENTERS OF ALL SECOND APPLICATION SOLUTIONS PRESENT IN ONE CONTAINER

(E) WELL HAVING THIRD DIMENSION $r_3$

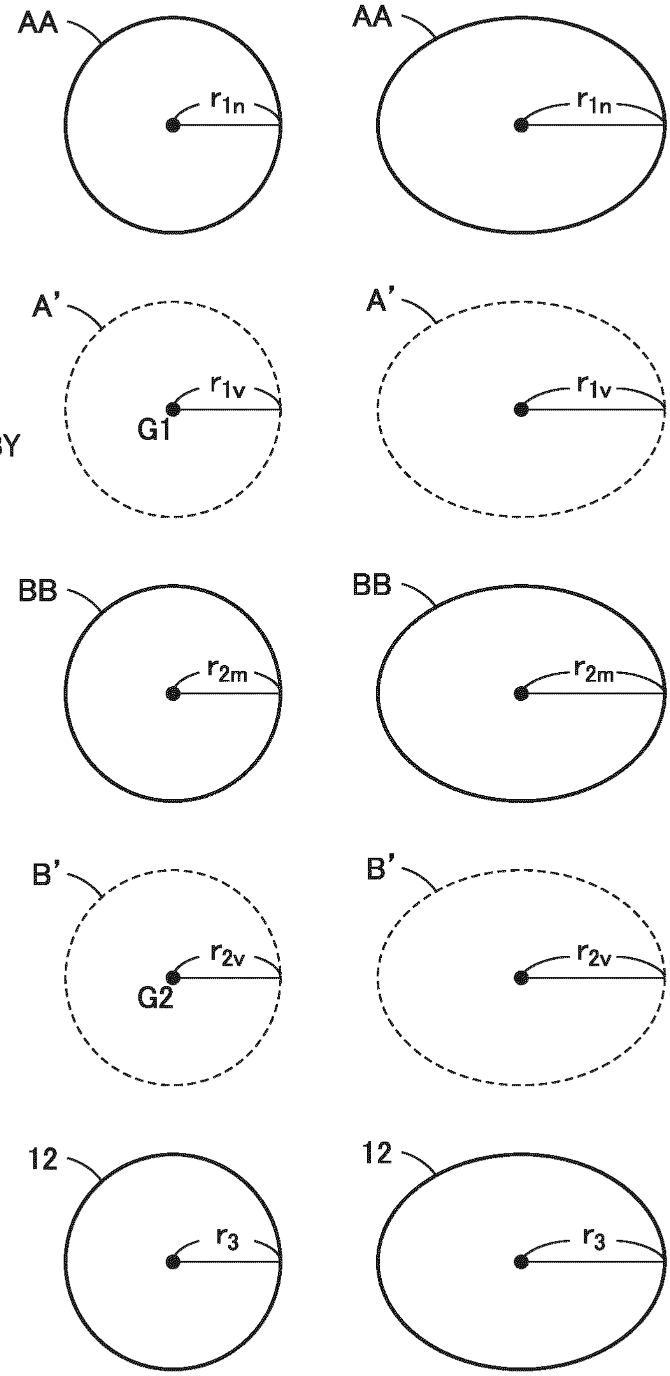

FIG.5

(A)

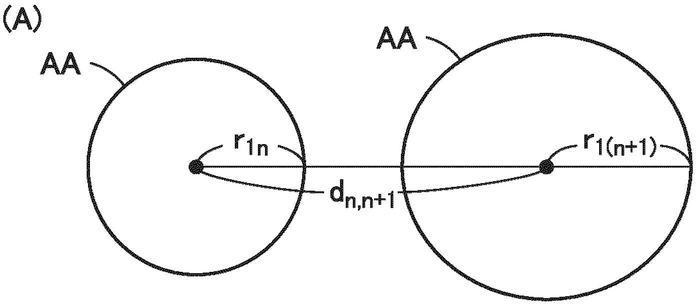


(B)

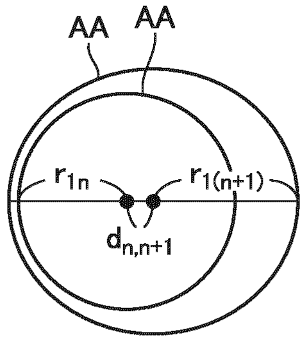


(C)

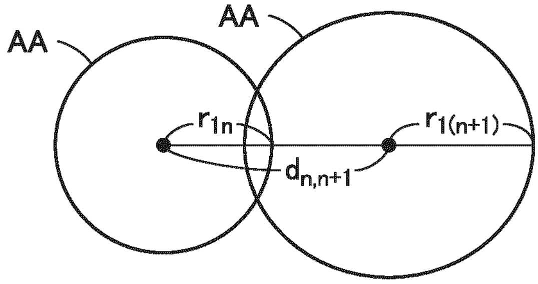

FIG.6

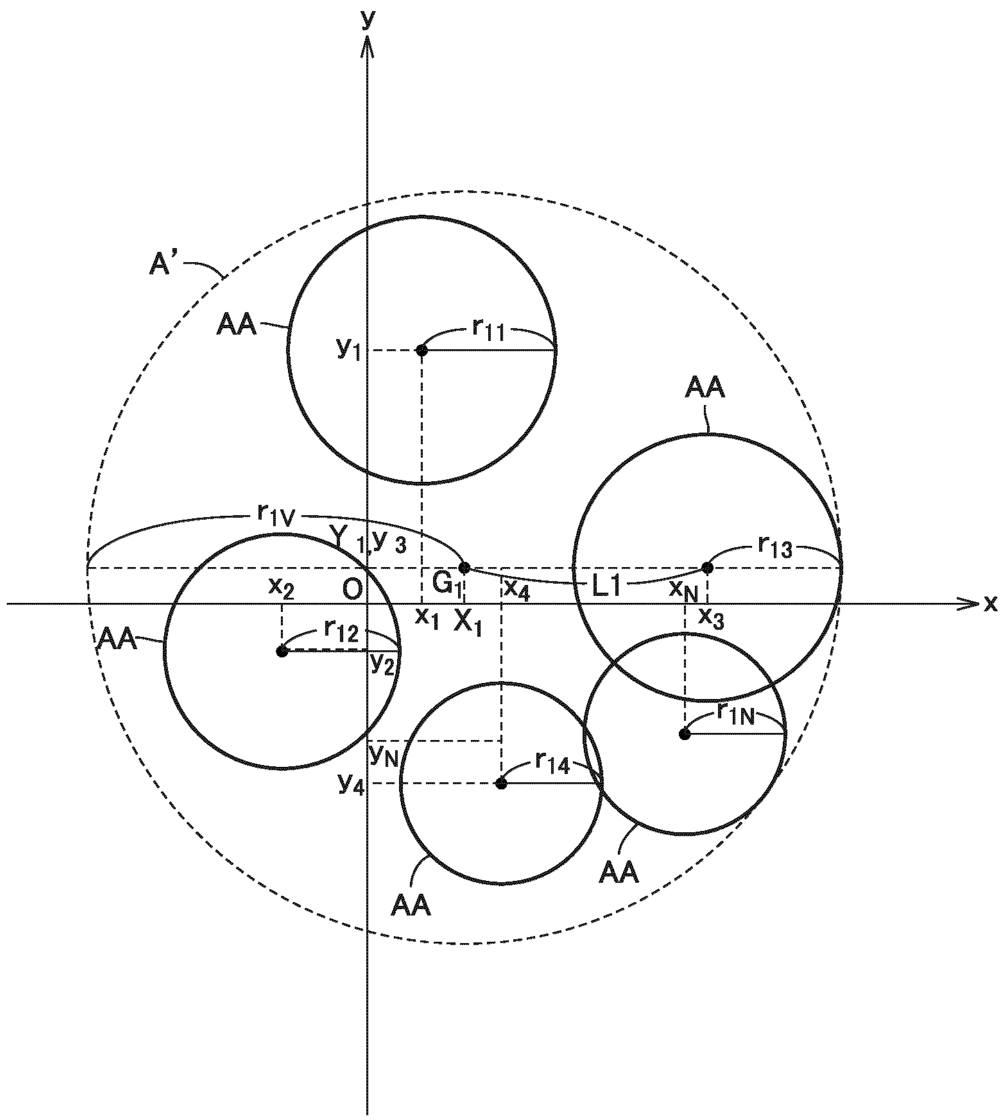
y
x
A'
AA
AA
AA
AA
AA
AA
$y_1$
$r_{11}$
$r_{1V}$
$Y_1, y_3$
$G_1$
$x_2$
O
$x_1$
$X_1$
$x_4$
L1
$x_N$
$x_3$
$r_{13}$
$r_{12}$
$y_2$
$r_{1N}$
$y_N$
$y_4$
$r_{14}$

FIG.7

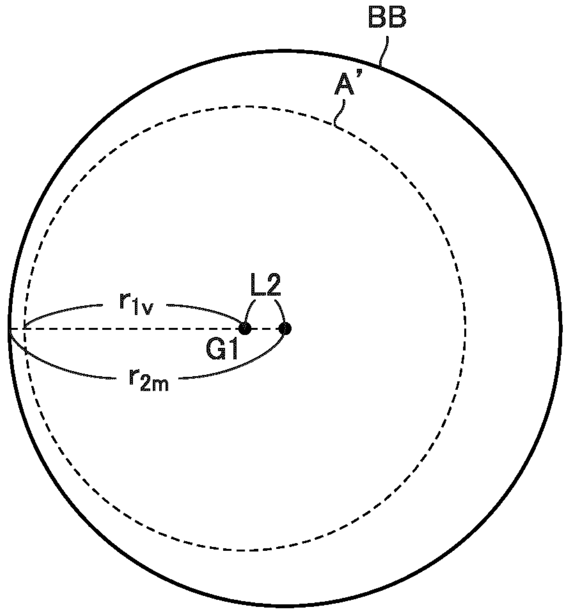

FIG.8

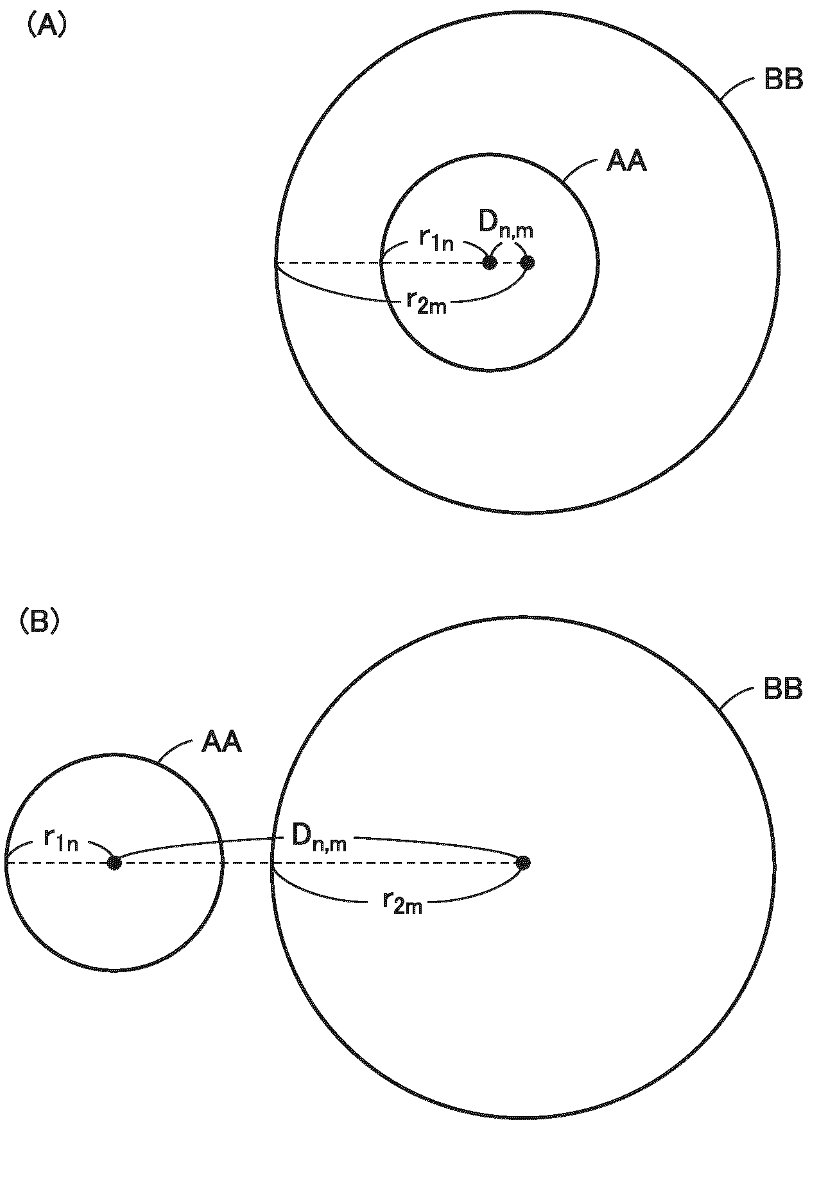

FIG.9

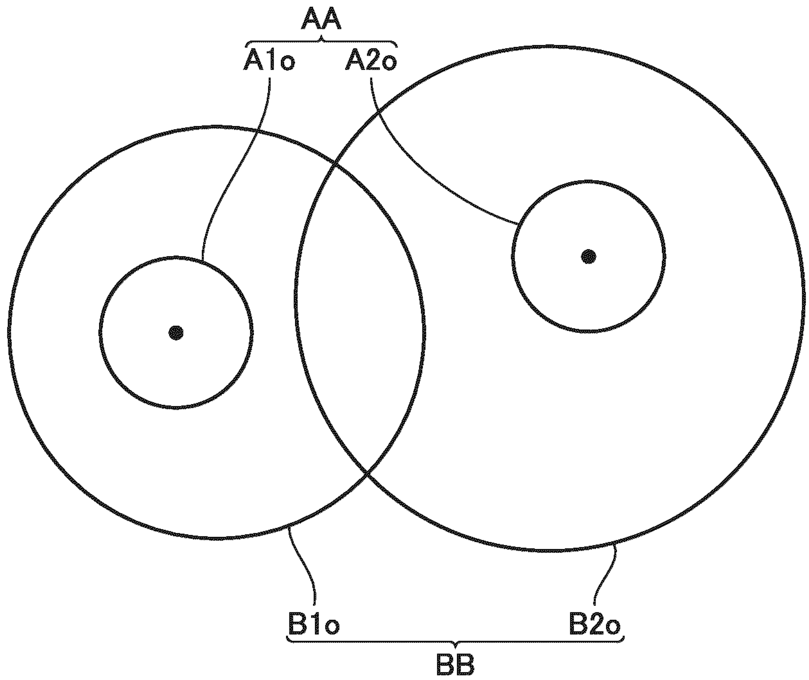

FIG.10

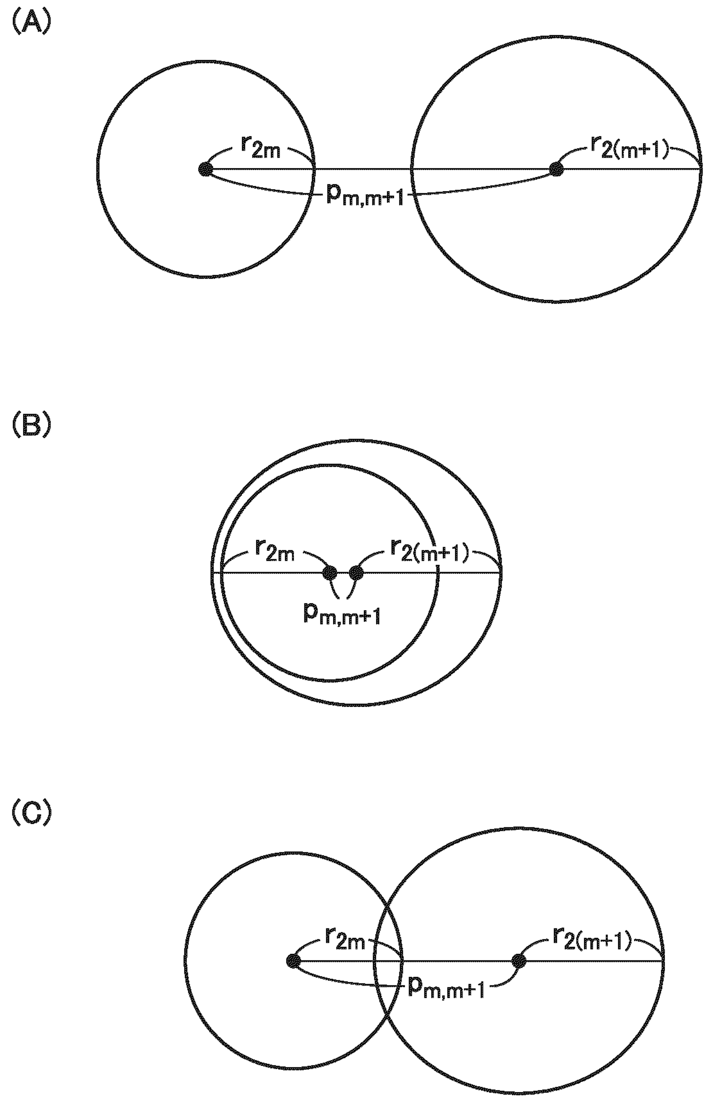

FIG.11

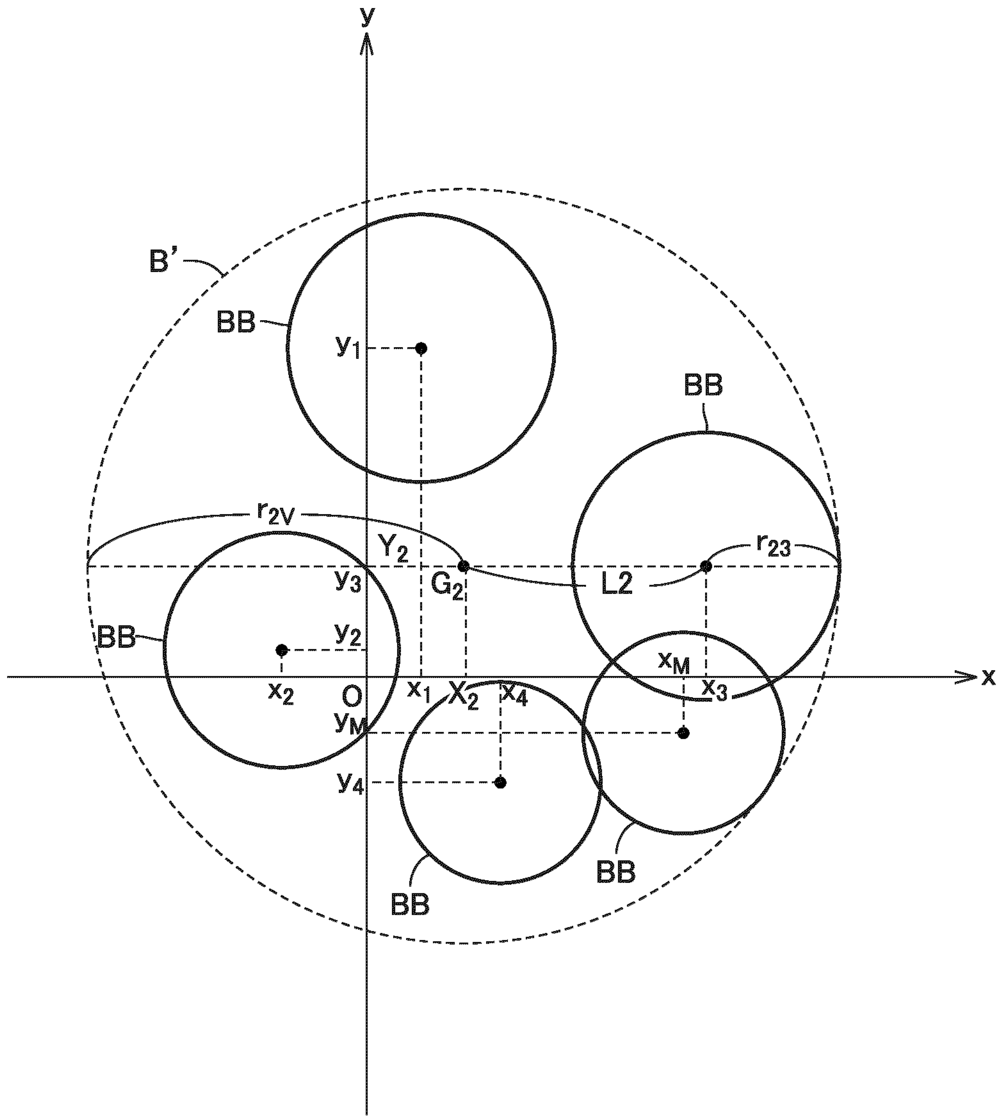

FIG.12

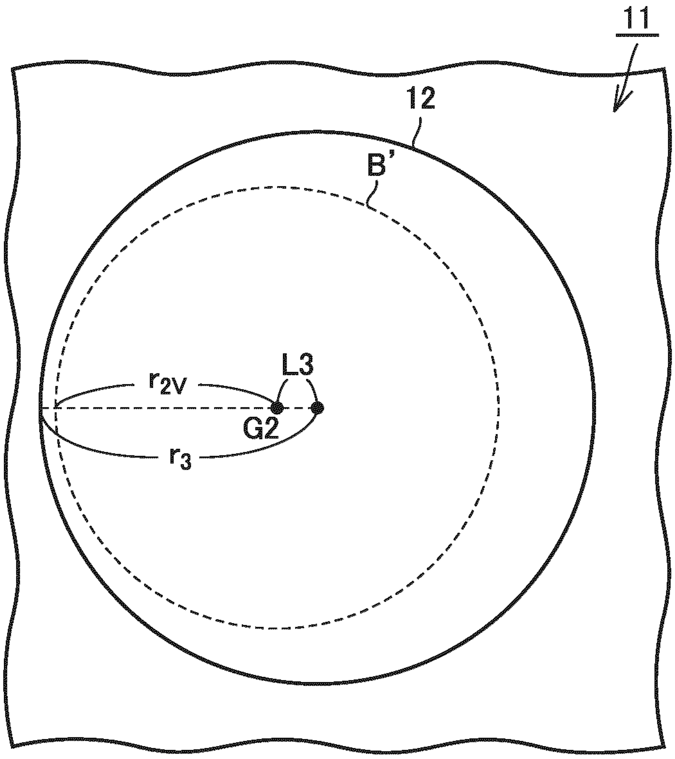


FIG.13

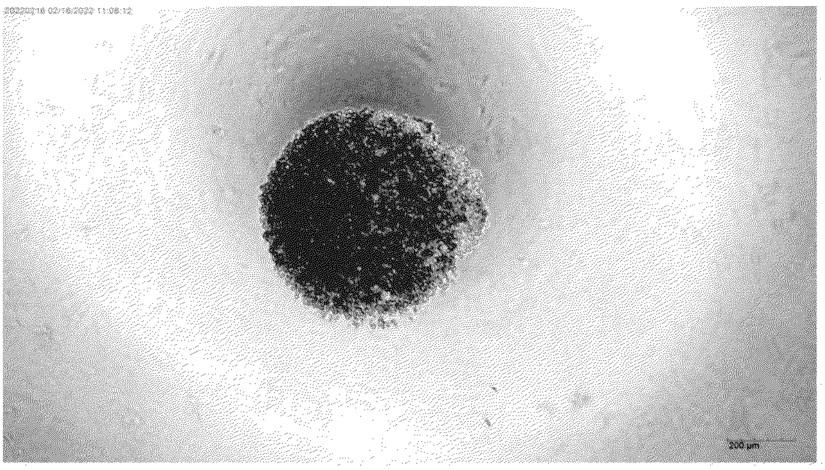

FIG.14

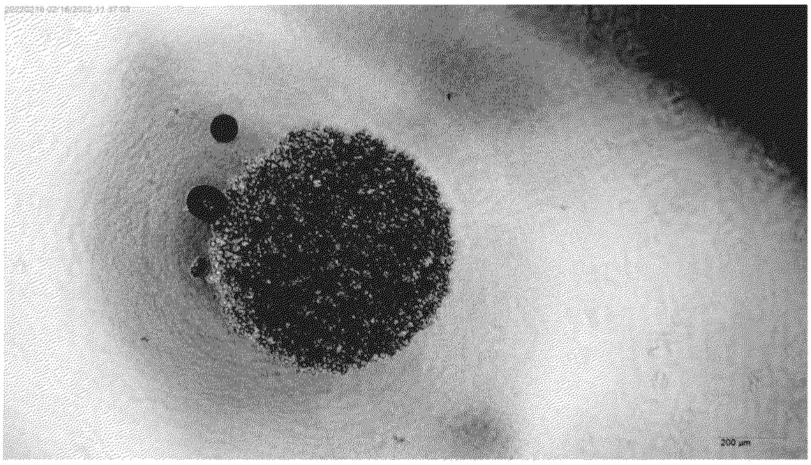

FIG.15

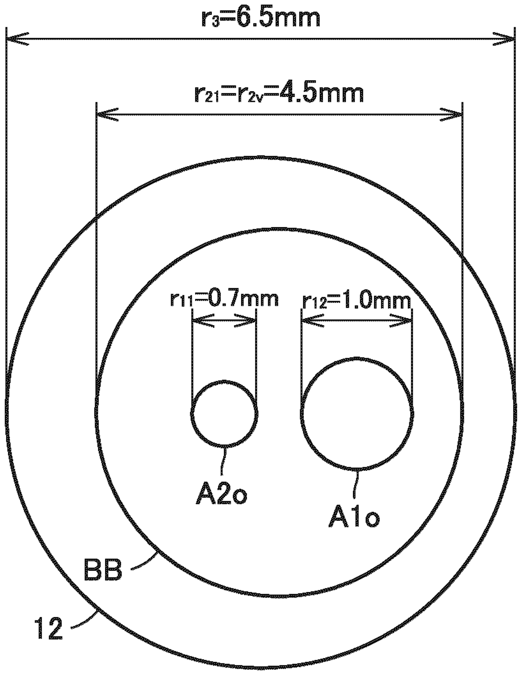

FIG.16

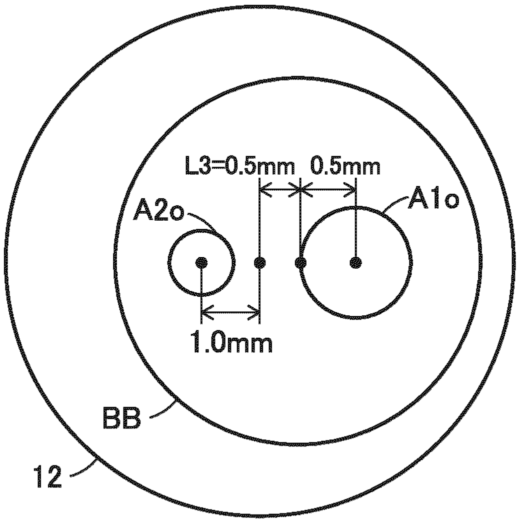


FIG.17

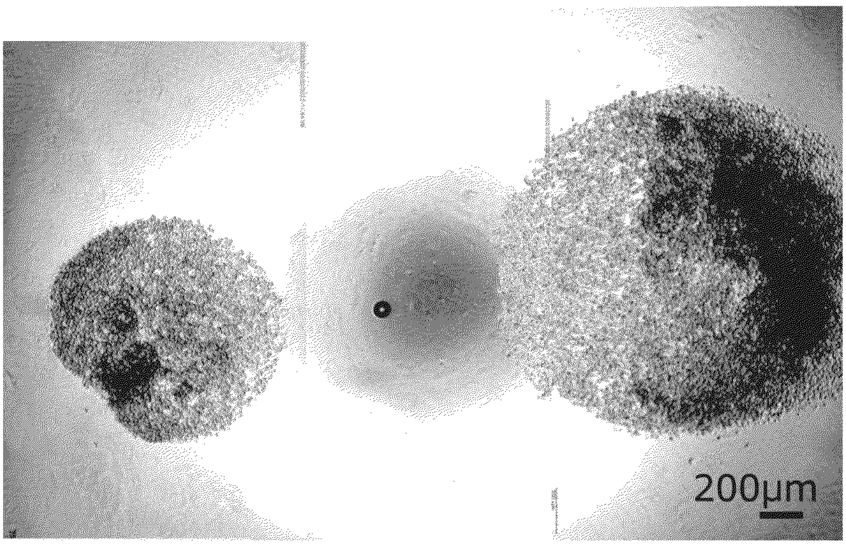

FIG.18

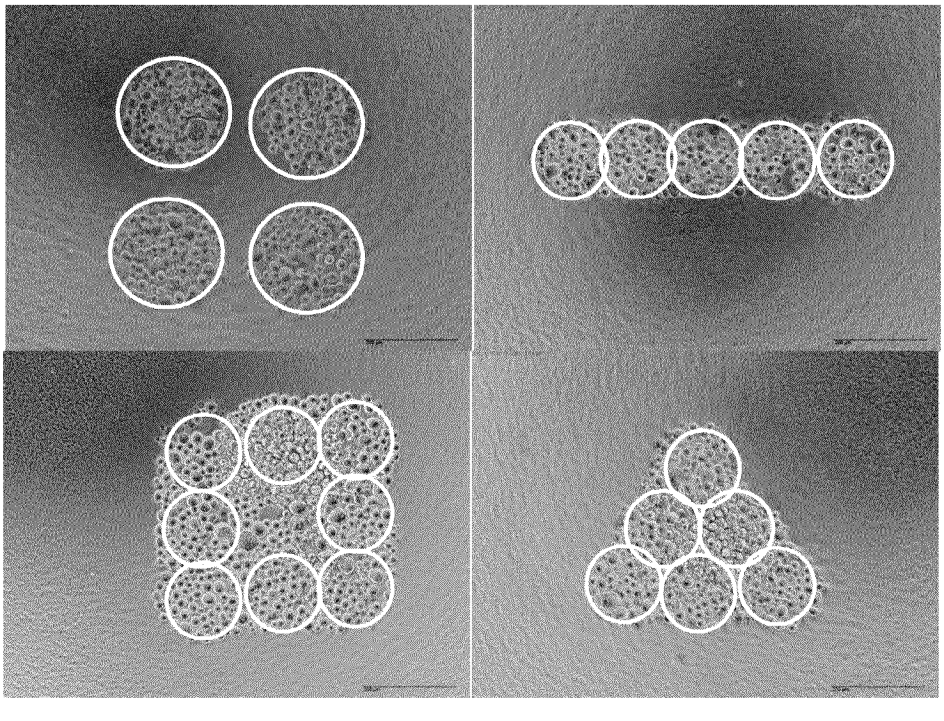

FIG.19

| | APPLIED TO WELL CENTER | APPLIED TO POINT SEPARATED FROM WELL CENER BY 0.5 mm | APPLIED TO POINT SEPARATED FROM WELL CENER BY 1.0 mm |
|---|---|---|---|
| ASSUMED SCHEMATIC DIAGRAM | 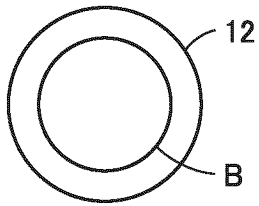 | 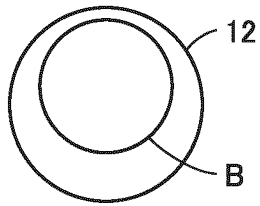 | 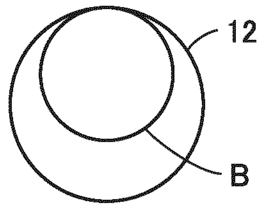 |
| RESULT SCHEMATIC DIAGRAM | 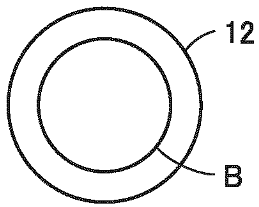 | 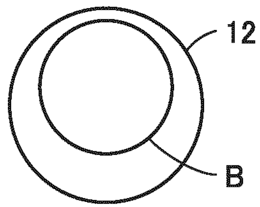 | 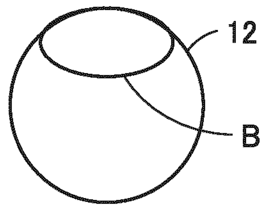 |
| PHOTOGRAPH | 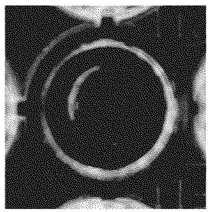 | 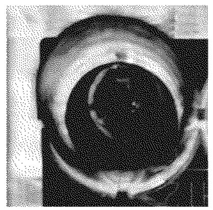 | 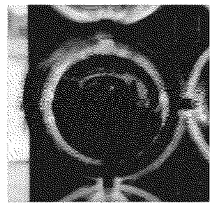 |

FIG.20

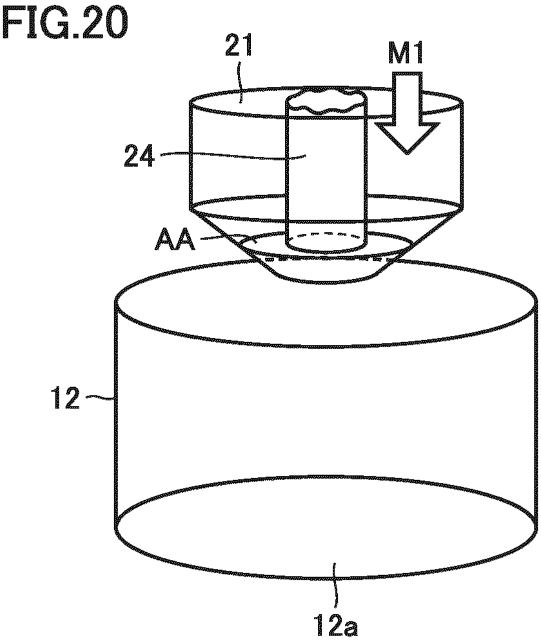


FIG.21

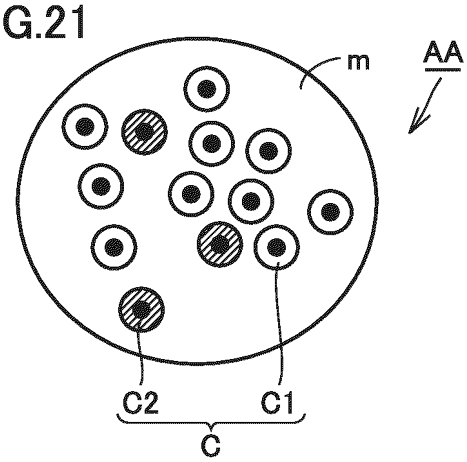

FIG.22

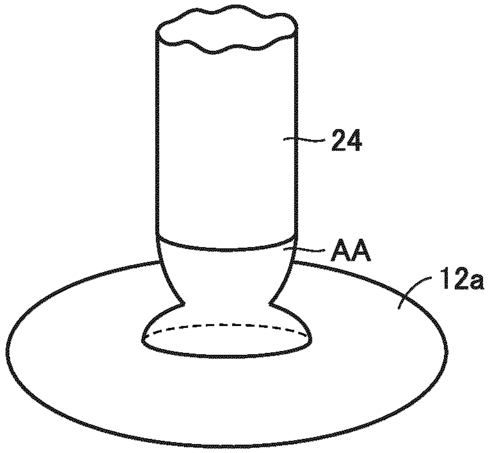


FIG.23

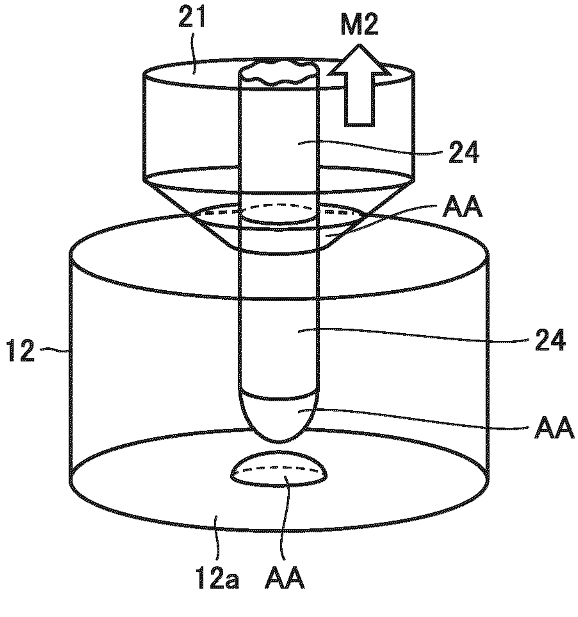

FIG.24

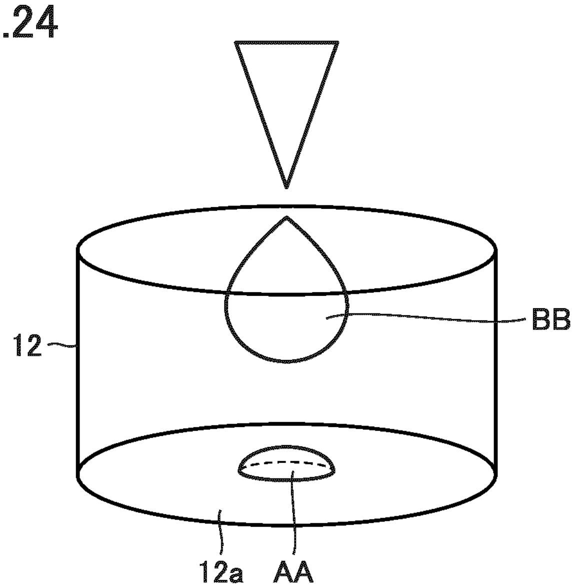


FIG.25

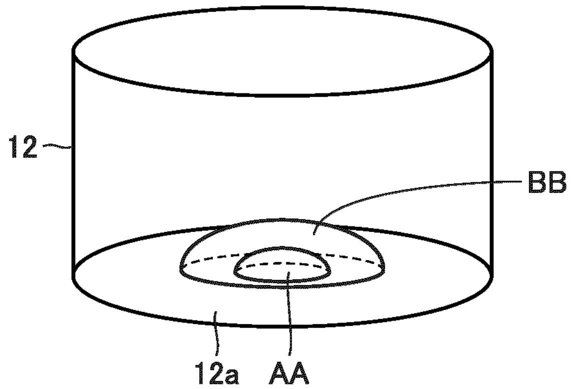


FIG.26

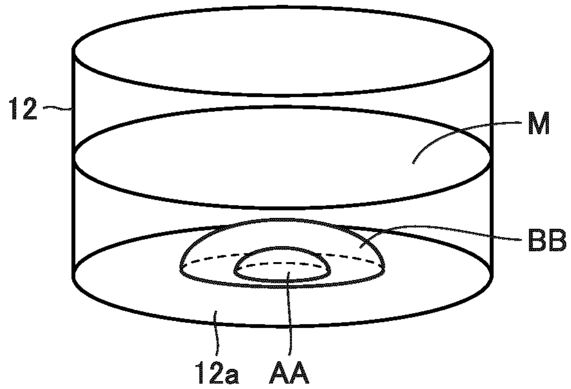

FIG.27

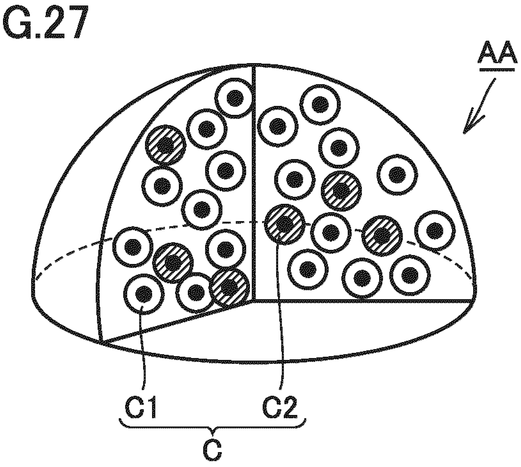


FIG.28

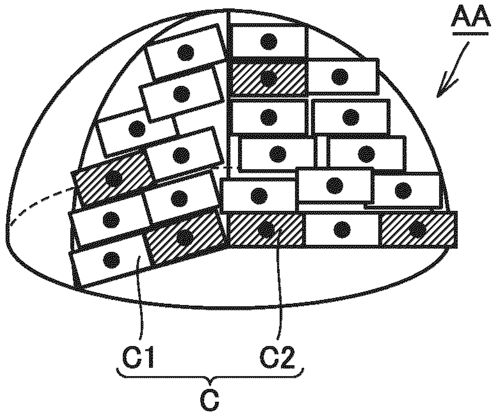


FIG.29

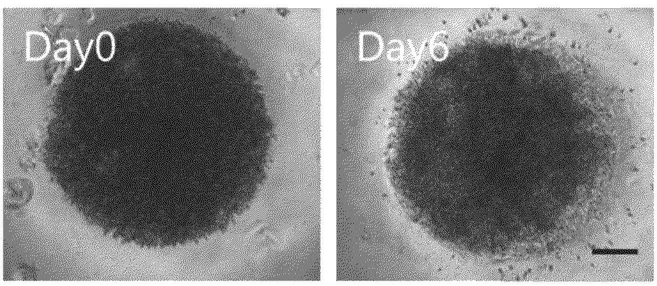


Scale bar 200 μm

FIG.30

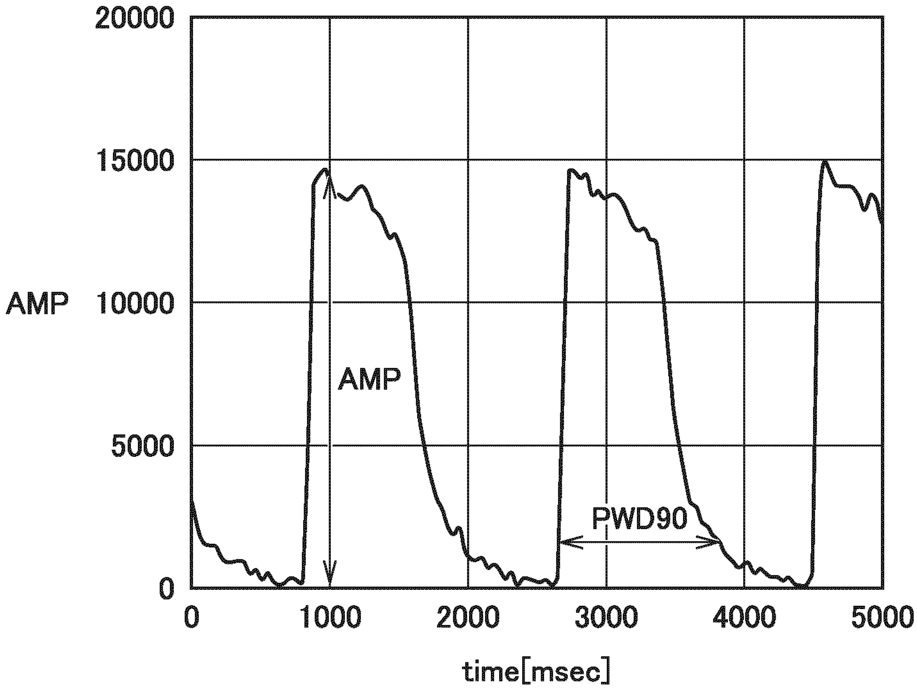
20000
15000
AMP
10000
5000
0
AMP
PWD90
0
1000
2000
3000
4000
5000
time[msec]

FIG.31

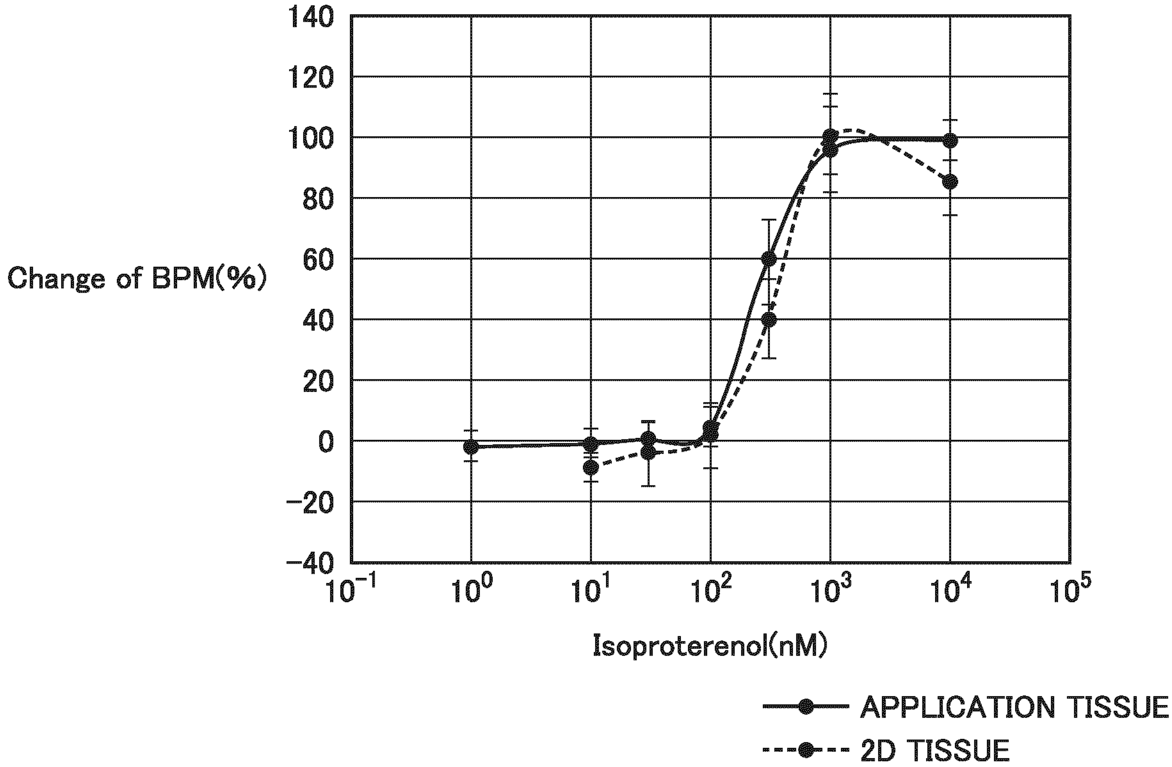
140
120
100
80
60
40
20
0
−20
−40
Change of BPM(%)
$10^{-1}$
$10^{0}$
$10^{1}$
$10^{2}$
$10^{3}$
$10^{4}$
$10^{5}$
Isoproterenol(nM)
APPLICATION TISSUE
2D TISSUE

FIG.32

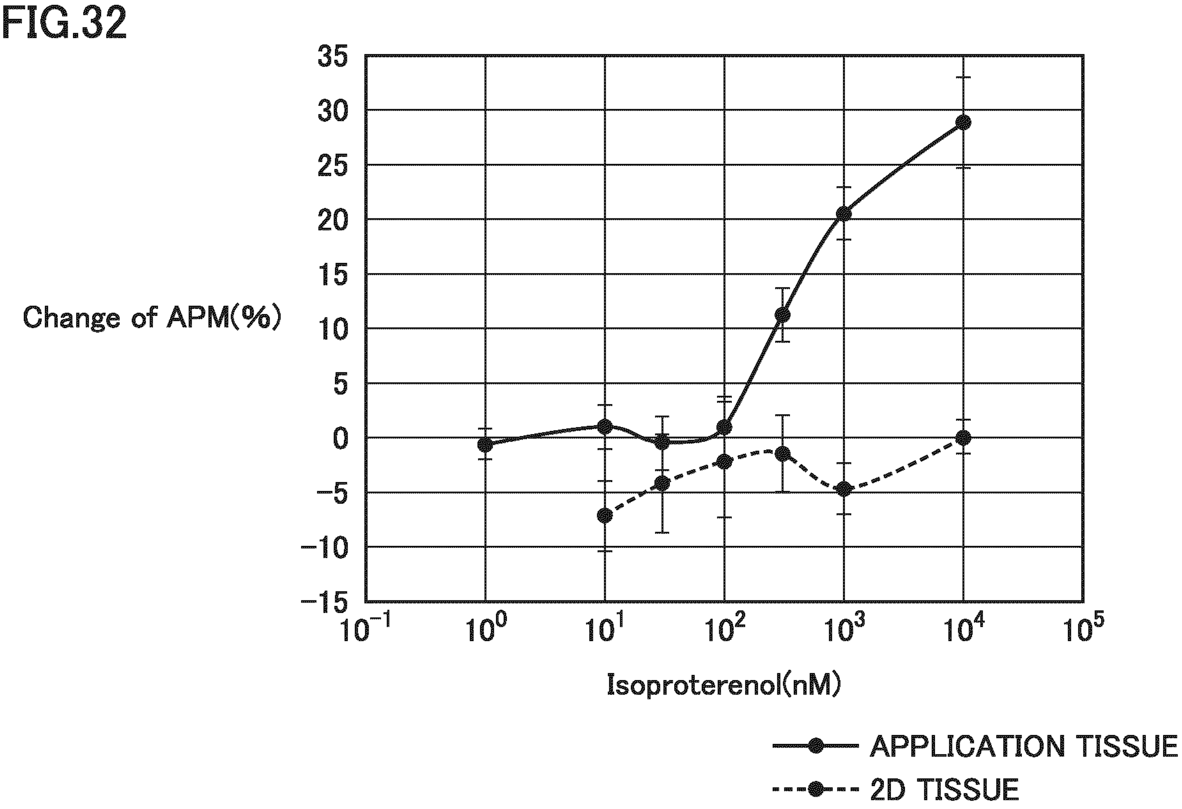
35
30
25
20
15
Change of APM(%)
10
5
0
−5
−10
−15
$10^{-1}$
$10^{0}$
$10^{1}$
$10^{2}$
$10^{3}$
$10^{4}$
$10^{5}$
Isoproterenol(nM)
APPLICATION TISSUE
2D TISSUE

FIG.33

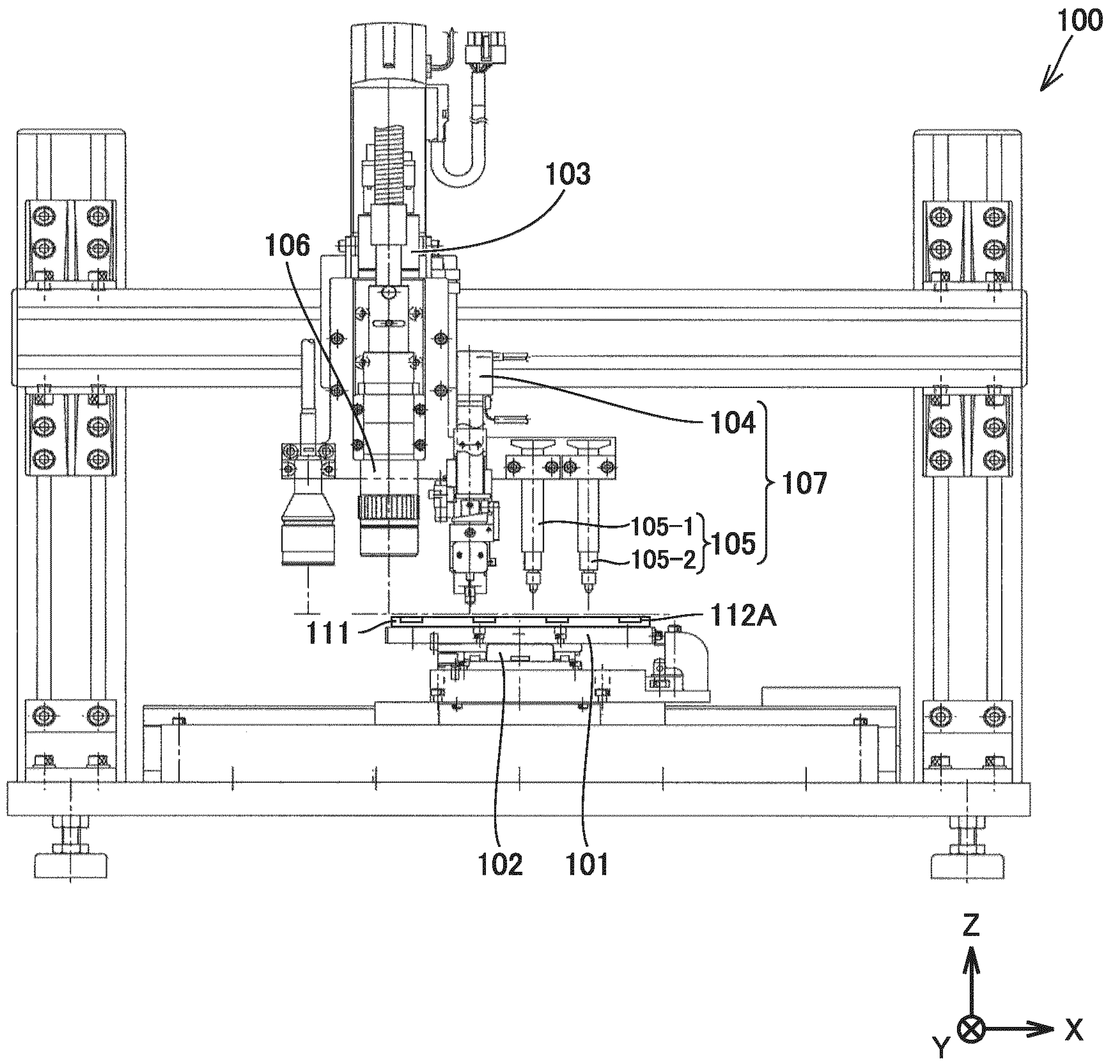

FIG.34

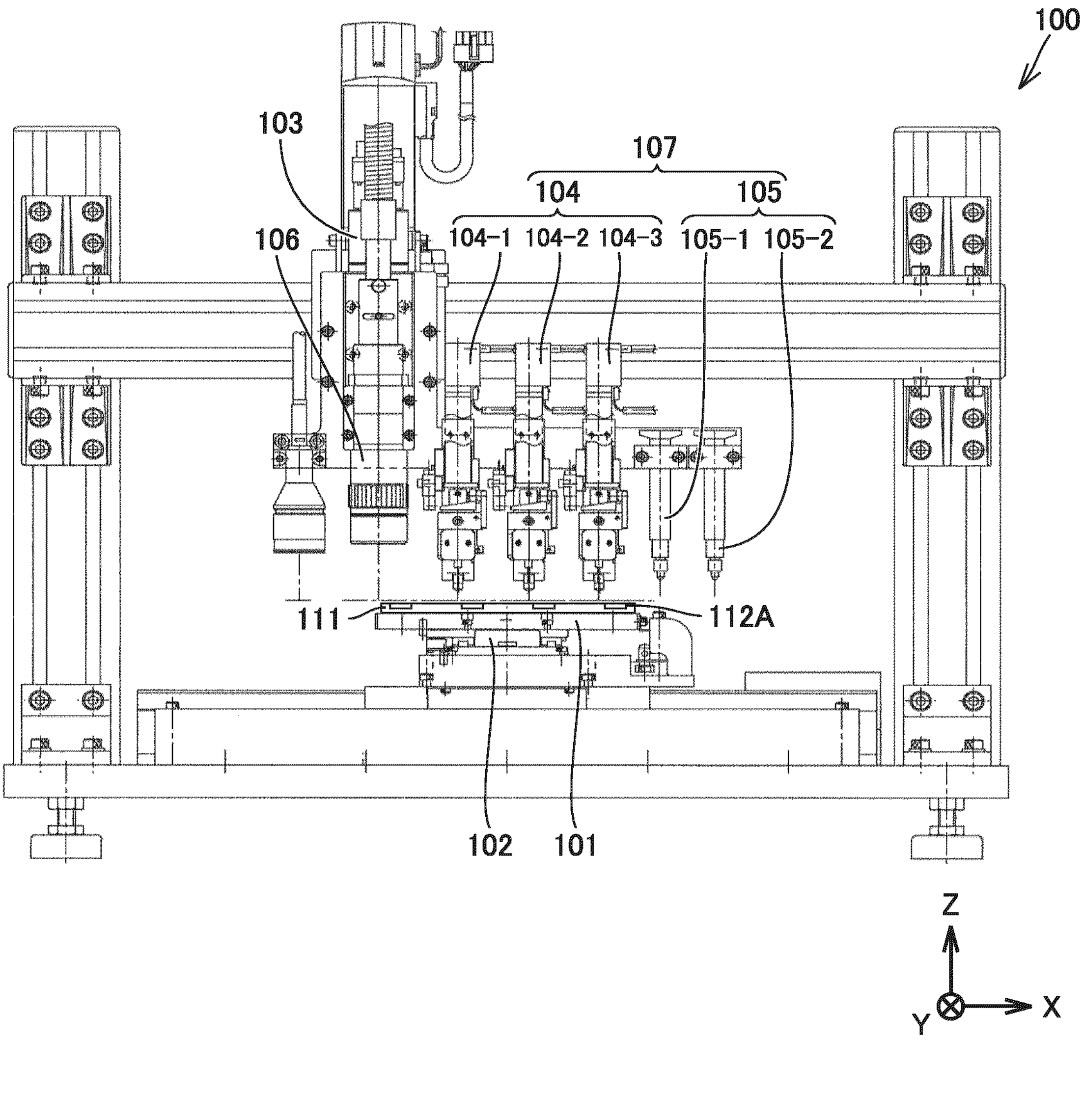
100
103
107
104
105
106
104-1
104-2
104-3
105-1
105-2
111
112A
102
101
Z
Y
X

FIG.35

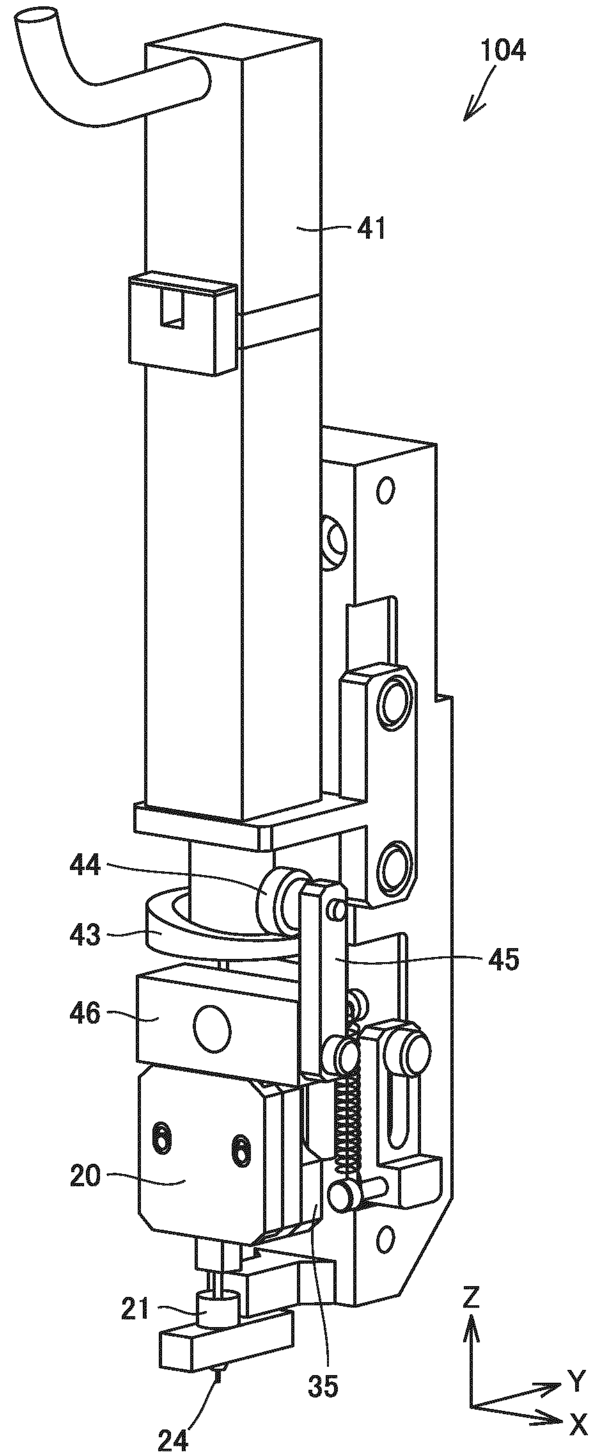

FIG.36

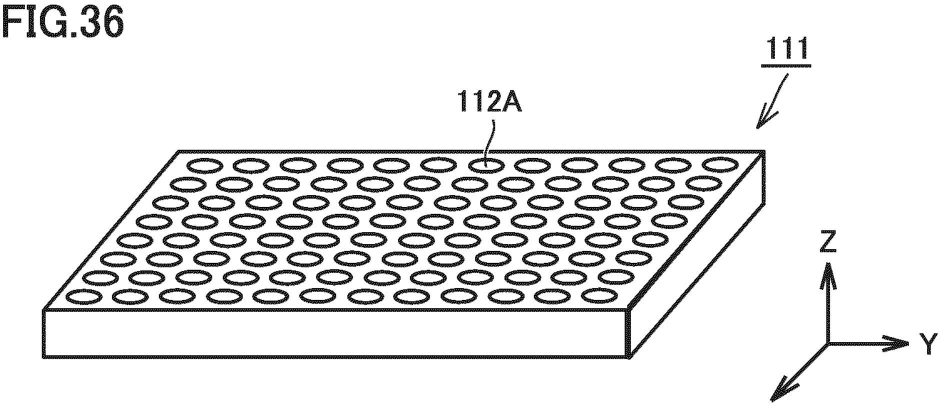

111
112A
Z
Y


FIG.37

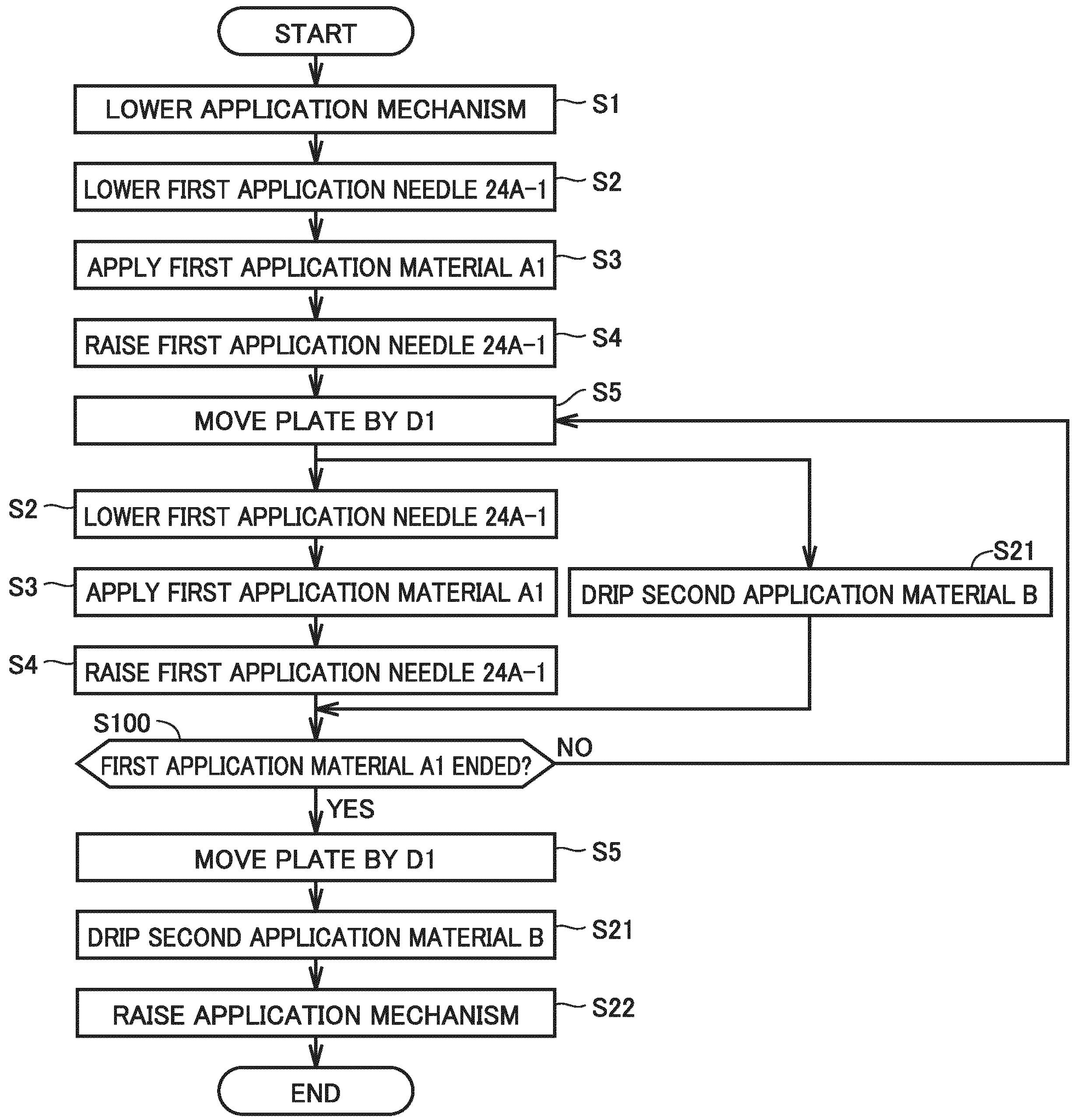

START
LOWER APPLICATION MECHANISM
S1
LOWER FIRST APPLICATION NEEDLE 24A-1
S2
APPLY FIRST APPLICATION MATERIAL A1
S3
RAISE FIRST APPLICATION NEEDLE 24A-1
S4
MOVE PLATE BY D1
S5
S2
LOWER FIRST APPLICATION NEEDLE 24A-1
S3
APPLY FIRST APPLICATION MATERIAL A1
S21
DRIP SECOND APPLICATION MATERIAL B
S4
RAISE FIRST APPLICATION NEEDLE 24A-1
S100
FIRST APPLICATION MATERIAL A1 ENDED?
NO
YES
MOVE PLATE BY D1
S5
DRIP SECOND APPLICATION MATERIAL B
S21
RAISE APPLICATION MECHANISM
S22
END

FIG.38

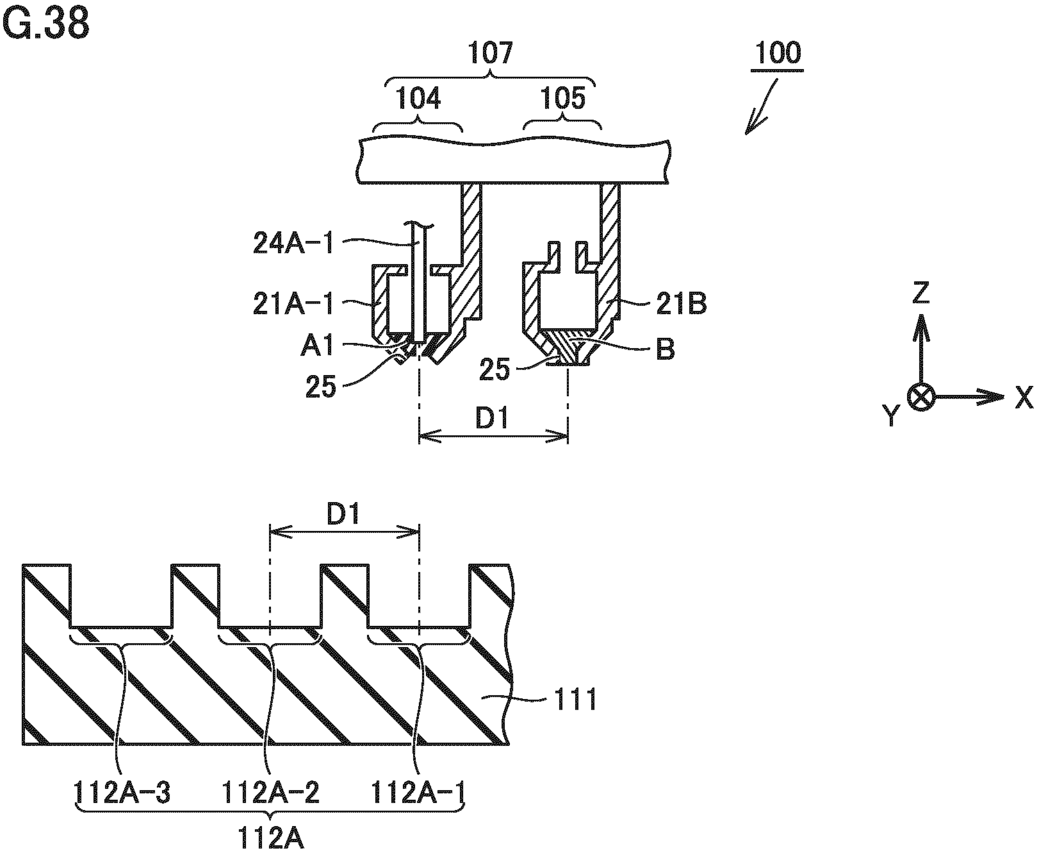
107
100
104
105
24A-1
21A-1
21B
A1
B
25
25
D1
D1
Z
Y
X
111
112A-3
112A-2
112A-1
112A

FIG.39

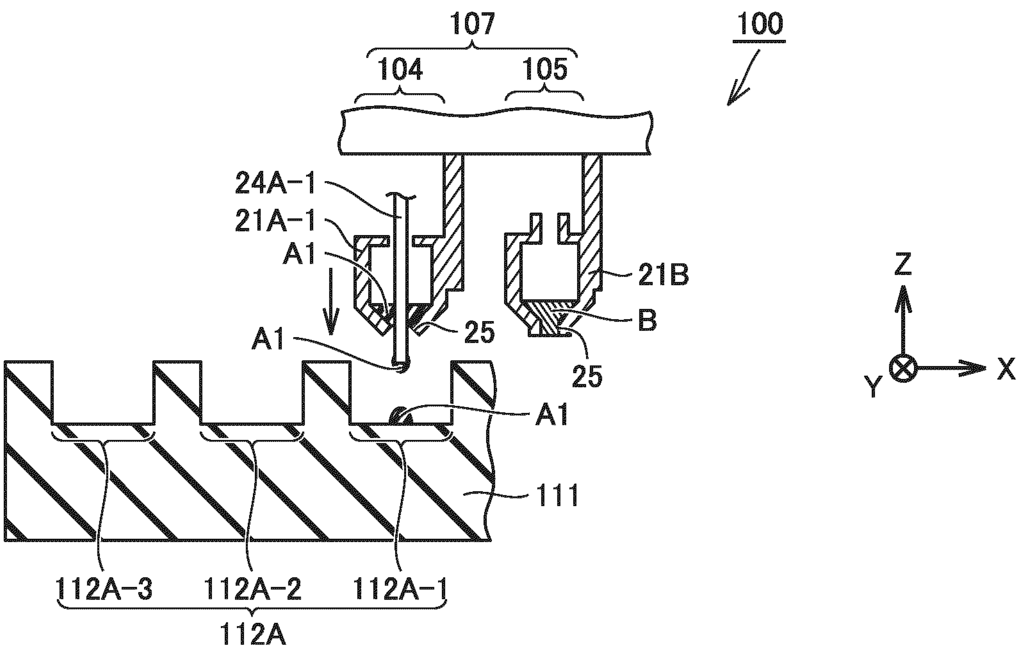
107
100
104
105
24A-1
21A-1
A1
21B
25
B
A1
25
A1
Z
Y
X
111
112A-3
112A-2
112A-1
112A

FIG.40

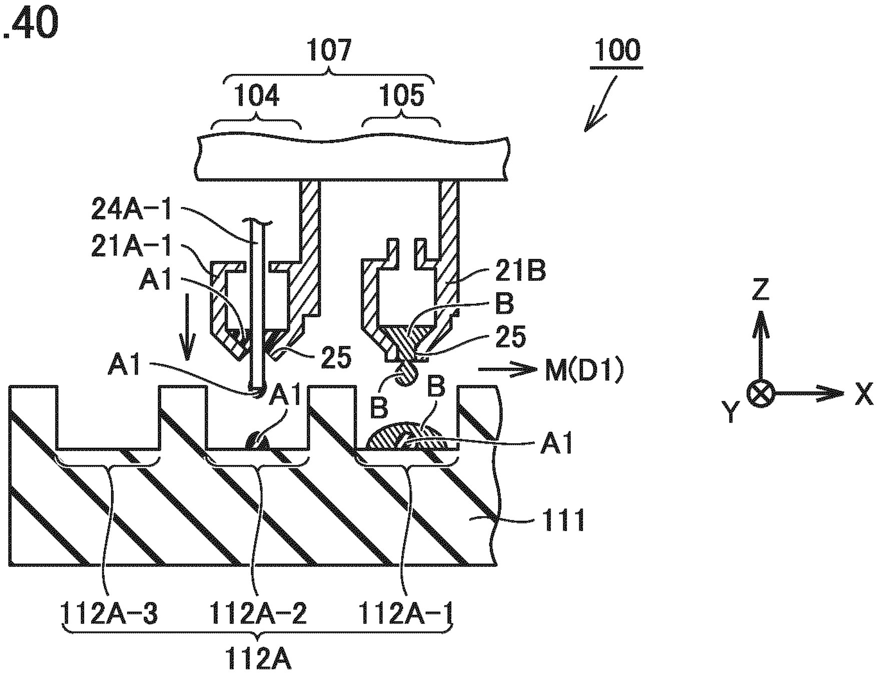
107
104
105
100
24A-1
21A-1
A1
A1
A1
25
21B
B
25
M(D1)
B
B
A1
Z
Y
X
111
112A-3
112A-2
112A-1
112A

FIG.41

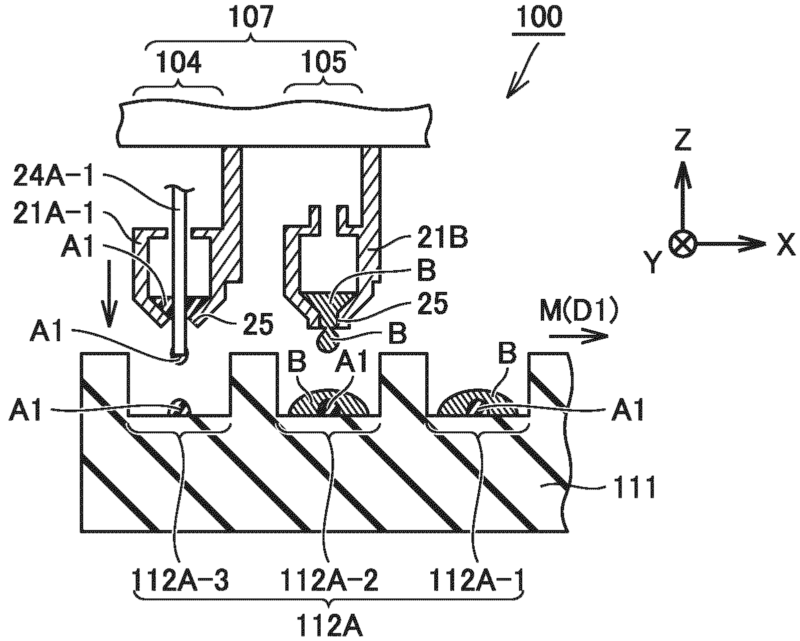
107
104
105
100
24A-1
21A-1
A1
A1
25
21B
B
25
B
M(D1)
B
A1
B
A1
A1
Z
Y
X
111
112A-3
112A-2
112A-1
112A

FIG.42

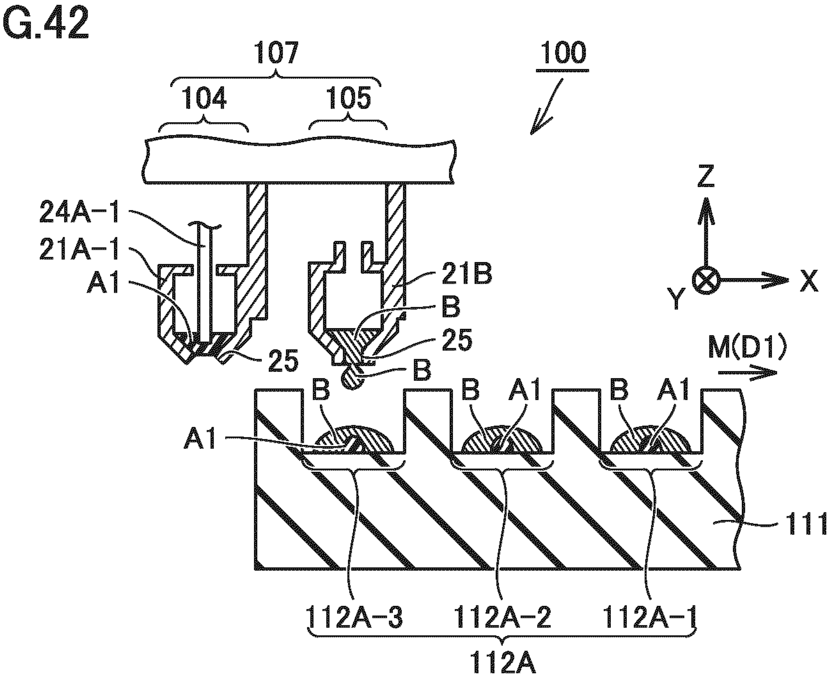
107
104
105
100
24A-1
21A-1
A1
25
21B
B
25
B
Z
Y
X
M(D1)
B
A1
B
A1
B
A1
111
112A-3
112A-2
112A-1
112A

FIG.43

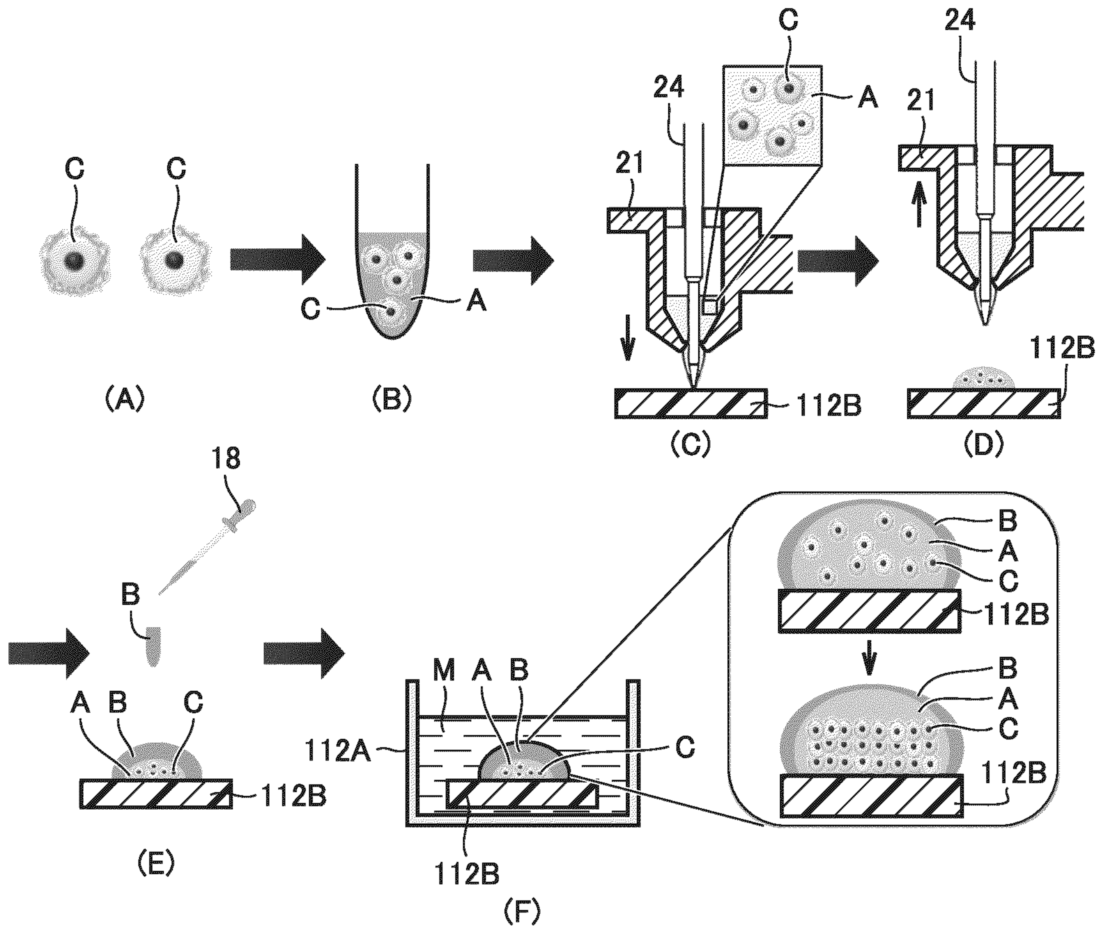
C
C
(A)
C
A
(B)
C
24
A
21
112B
(C)
24
21
112B
(D)
18
B
A B C
112B
(E)
M A B
112A
C
112B
(F)
B
A
C
112B
B
A
C
112B

FIG.44

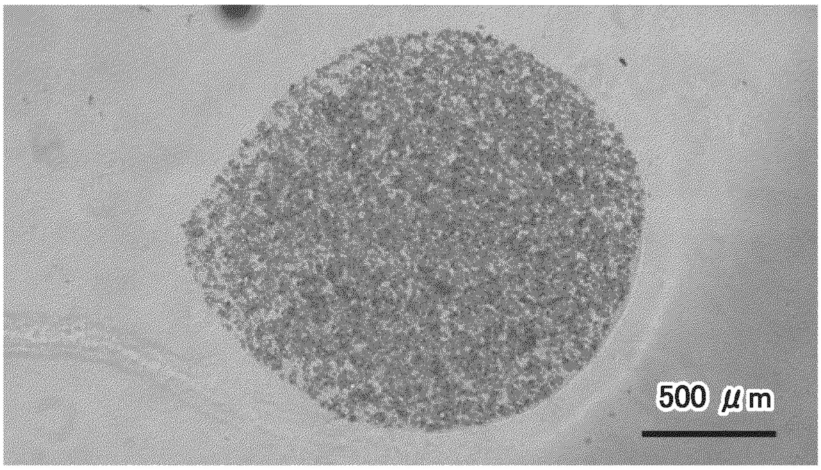


FIG.45

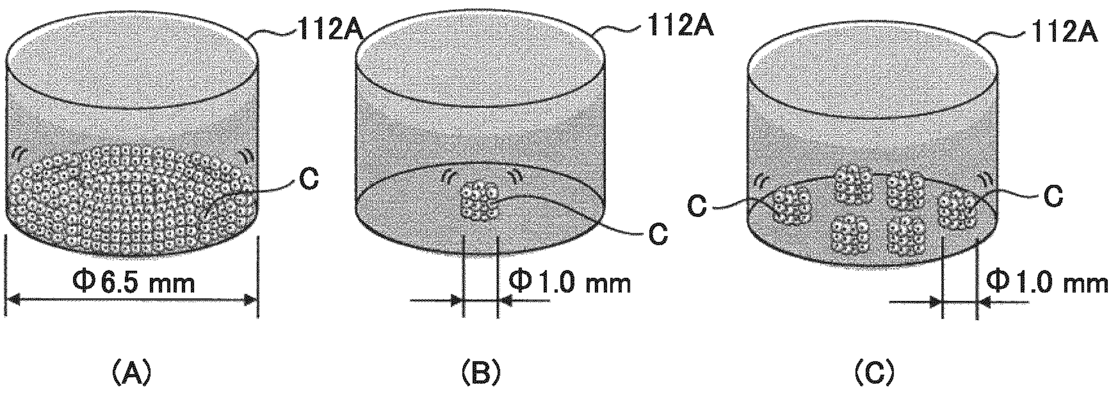

FIG.46

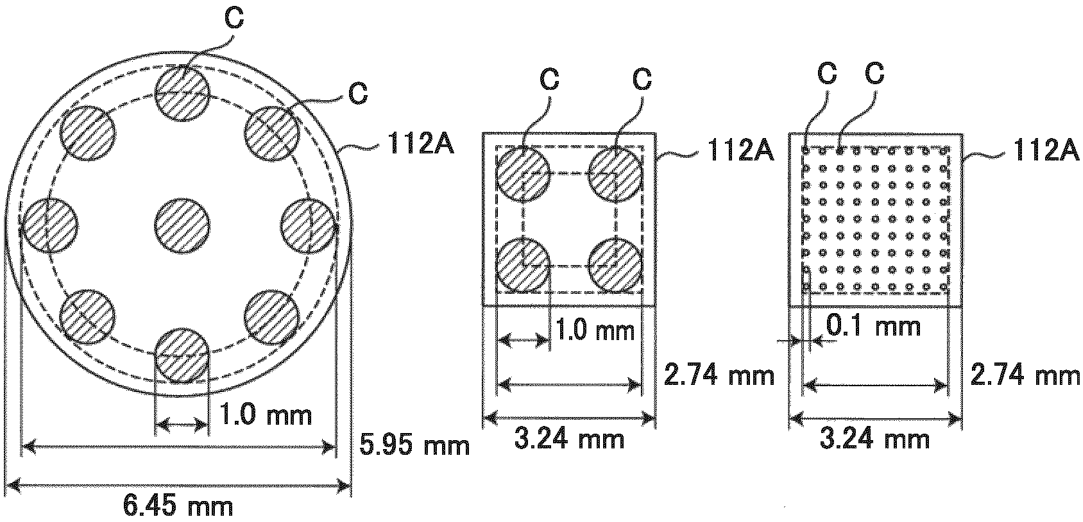
C
C
112A
1.0 mm
5.95 mm
6.45 mm
C
C
112A
1.0 mm
2.74 mm
3.24 mm
C C
112A
0.1 mm
2.74 mm
3.24 mm

(A) (B) (C)

FIG.47

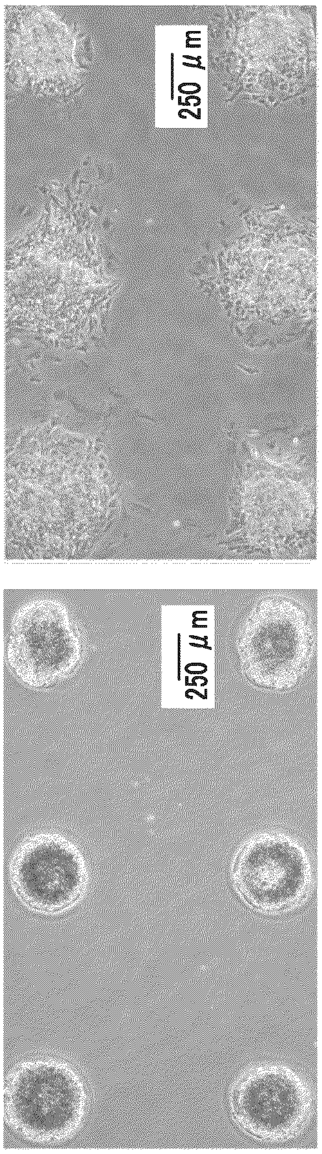


(A) (B)

FIG.48

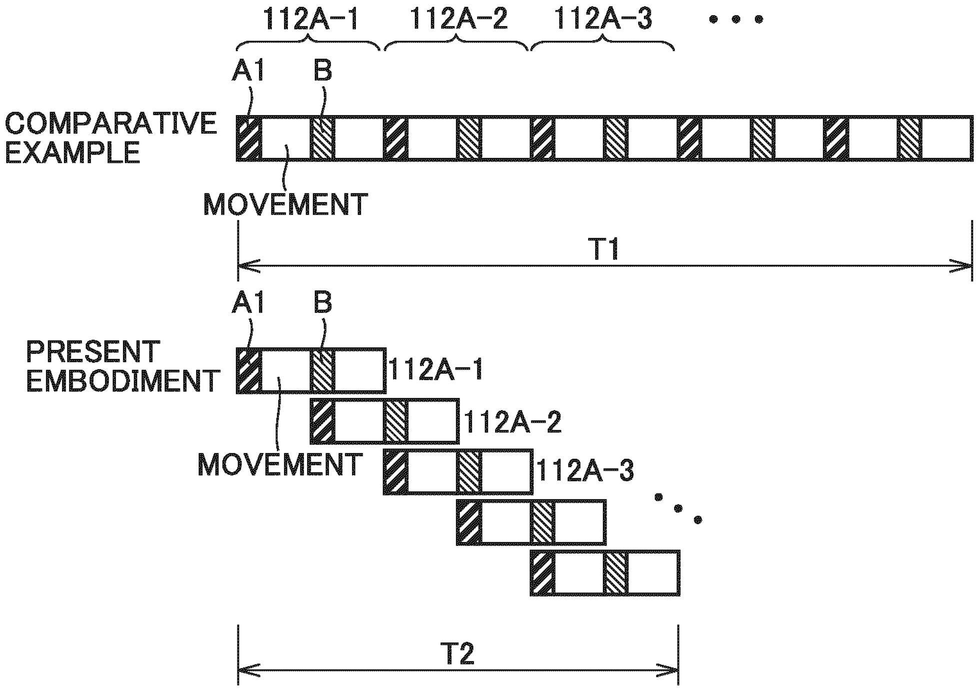
112A-1
112A-2
112A-3
• • •
A1
B
COMPARATIVE EXAMPLE
MOVEMENT
T1
A1
B
PRESENT EMBODIMENT
112A-1
112A-2
MOVEMENT
112A-3
• • •
T2

FIG.49

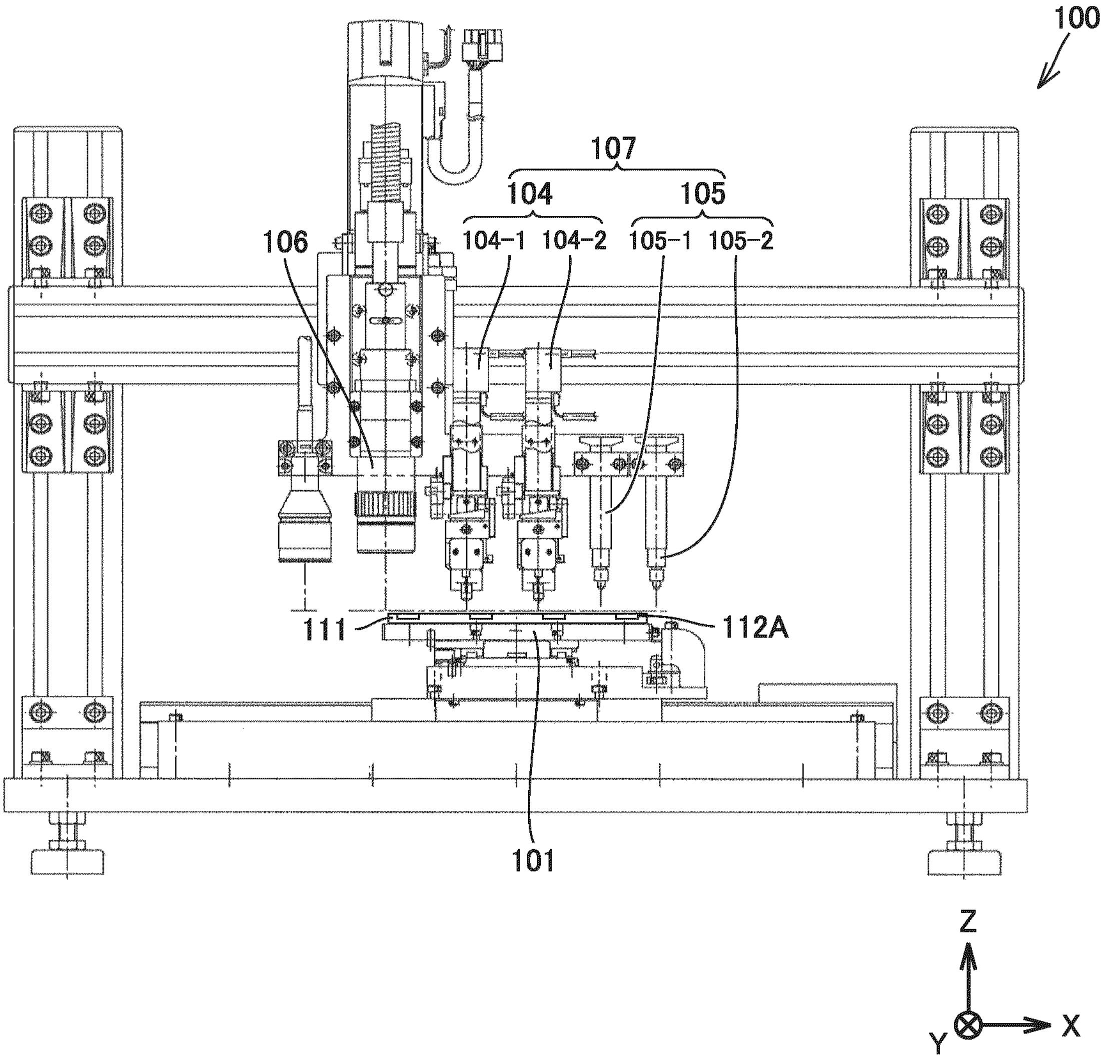

FIG.50

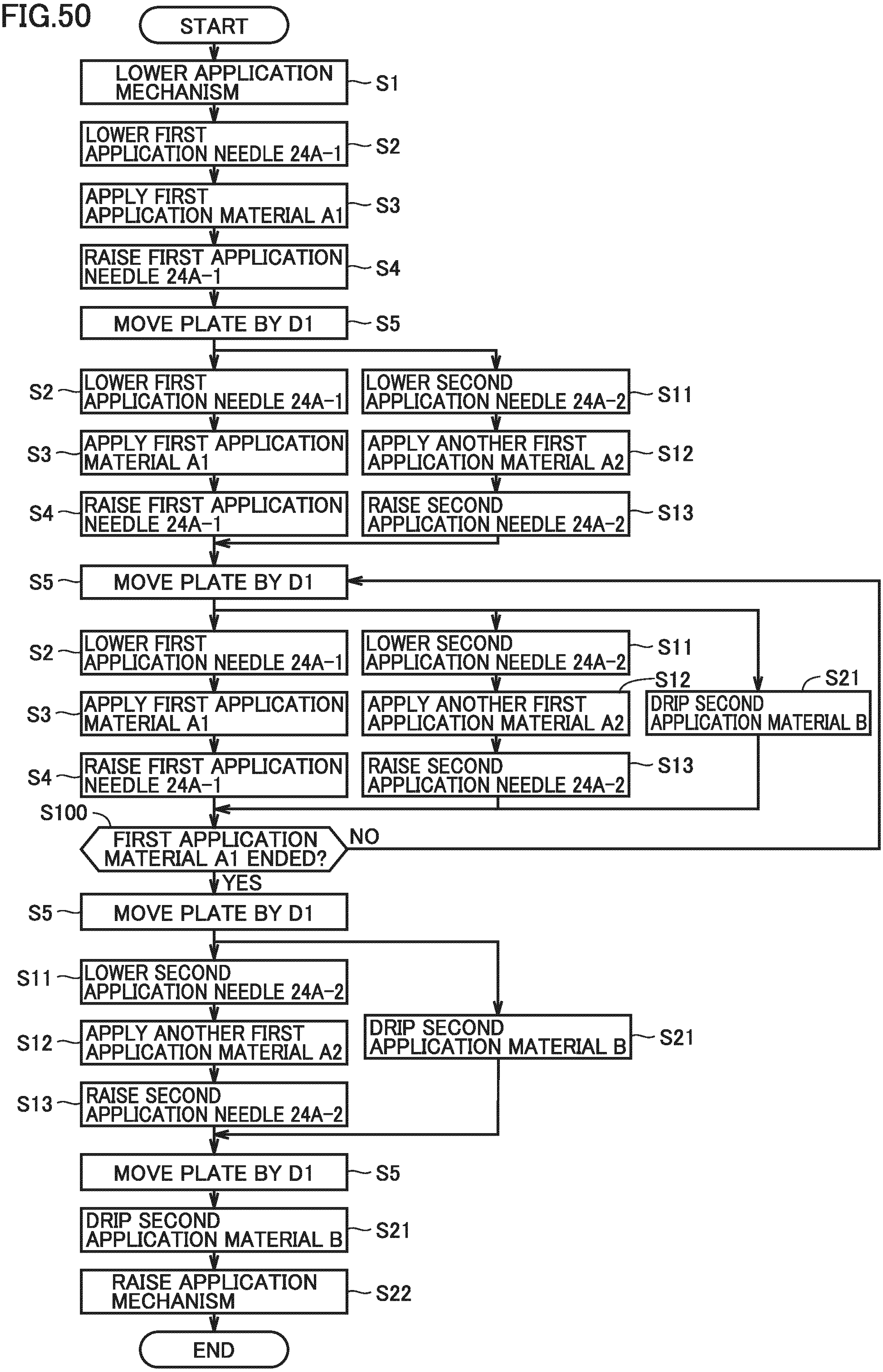
START
LOWER APPLICATION MECHANISM
S1
LOWER FIRST APPLICATION NEEDLE 24A-1
S2
APPLY FIRST APPLICATION MATERIAL A1
S3
RAISE FIRST APPLICATION NEEDLE 24A-1
S4
MOVE PLATE BY D1
S5
LOWER SECOND APPLICATION NEEDLE 24A-2
S11
APPLY ANOTHER FIRST APPLICATION MATERIAL A2
S12
RAISE SECOND APPLICATION NEEDLE 24A-2
S13
DRIP SECOND APPLICATION MATERIAL B
S21
S100
FIRST APPLICATION MATERIAL A1 ENDED?
NO
YES
RAISE APPLICATION MECHANISM
S22
END

FIG.51

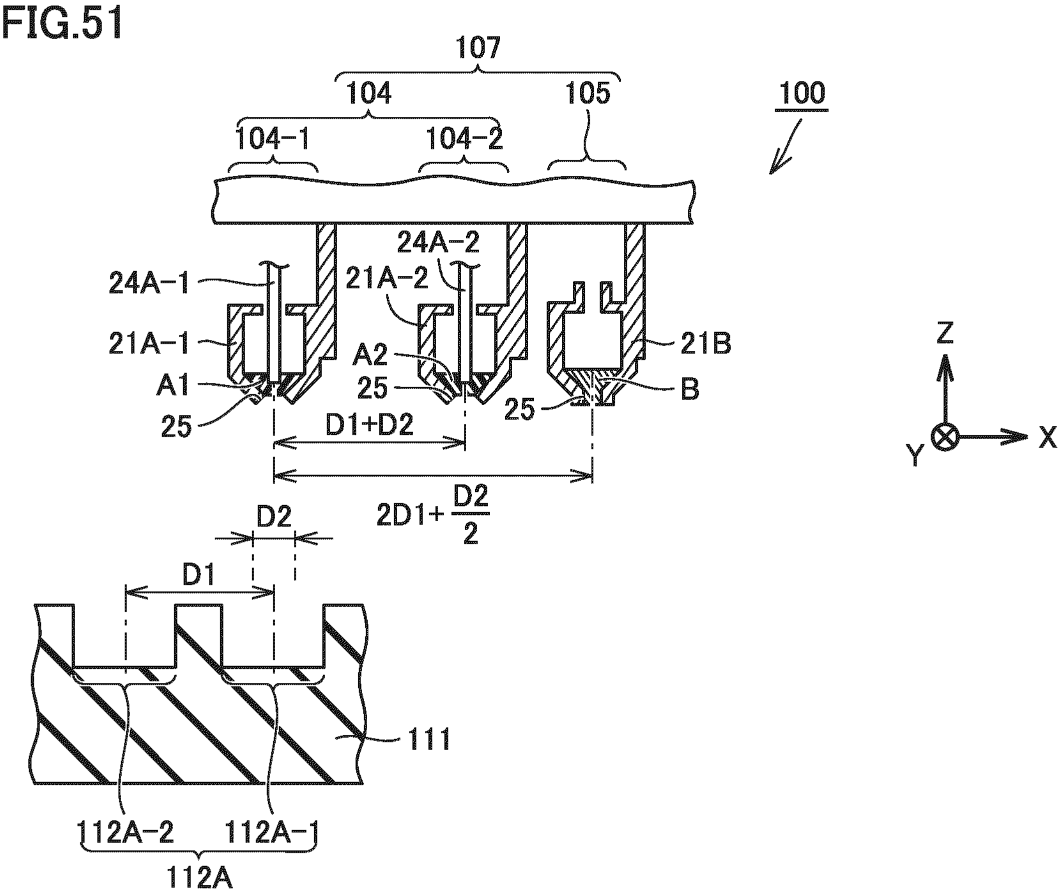
107
104
105
100
104-1
104-2
24A-1
24A-2
21A-2
21A-1
A1
A2
25
21B
B
D1+D2
2D1+ D2/2
D2
D1
111
112A-2
112A-1
112A
Z
Y
X

FIG.52

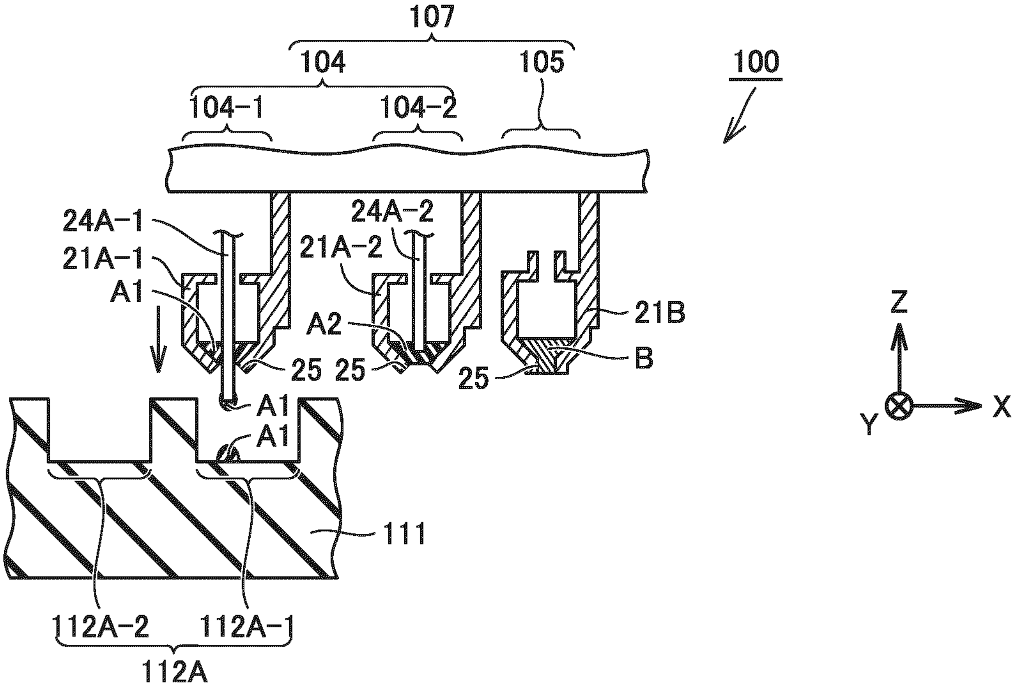
107
104
105
100
104-1
104-2
24A-1
24A-2
21A-1
21A-2
A1
A2
25
21B
B
111
112A-2
112A-1
112A
Z
Y
X

FIG.53

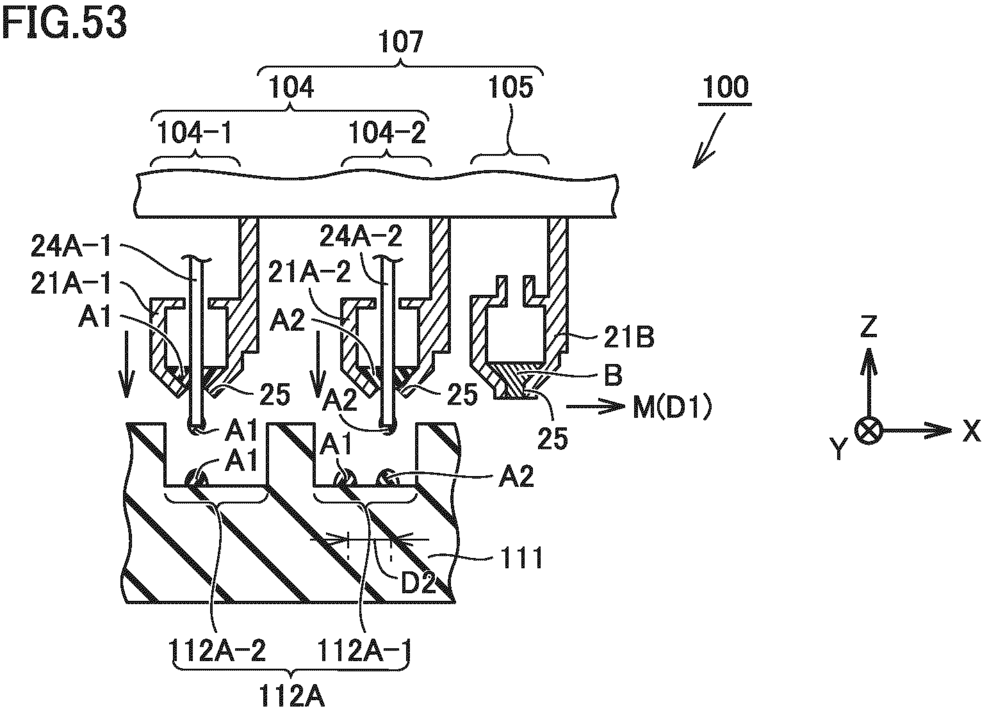
107
104
105
100
104-1
104-2
24A-1
24A-2
21A-1
21A-2
A1
A2
25
21B
B
M(D1)
Z
X
Y
111
D2
112A-2
112A-1
112A

FIG.54

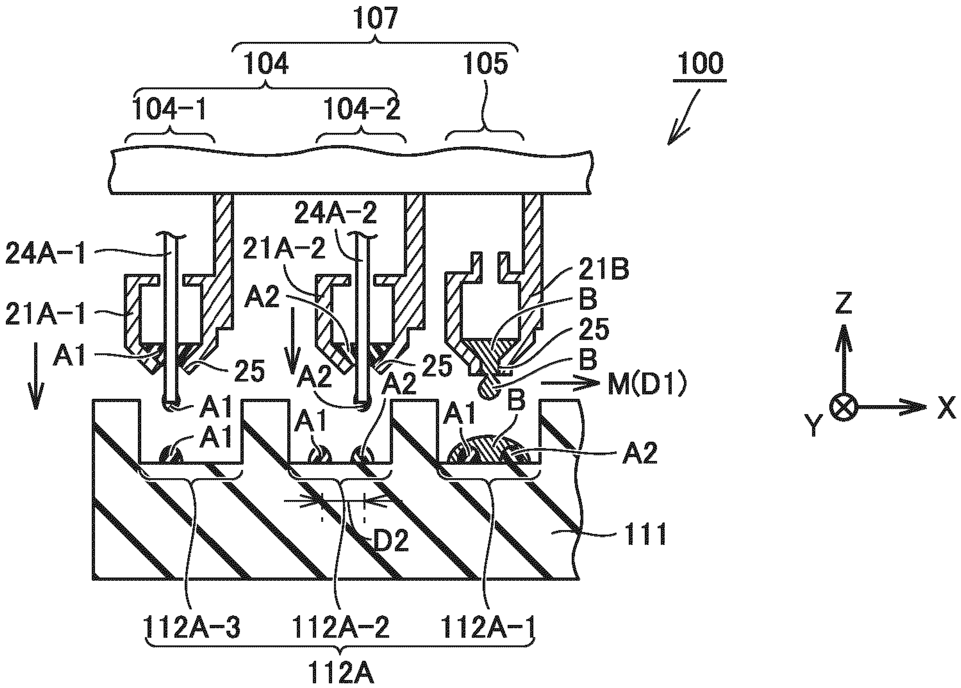
107
104
105
100
104-1
104-2
24A-1
24A-2
21A-1
21A-2
A1
A2
25
21B
B
M(D1)
Z
X
Y
111
D2
112A-3
112A-2
112A-1
112A

FIG.55

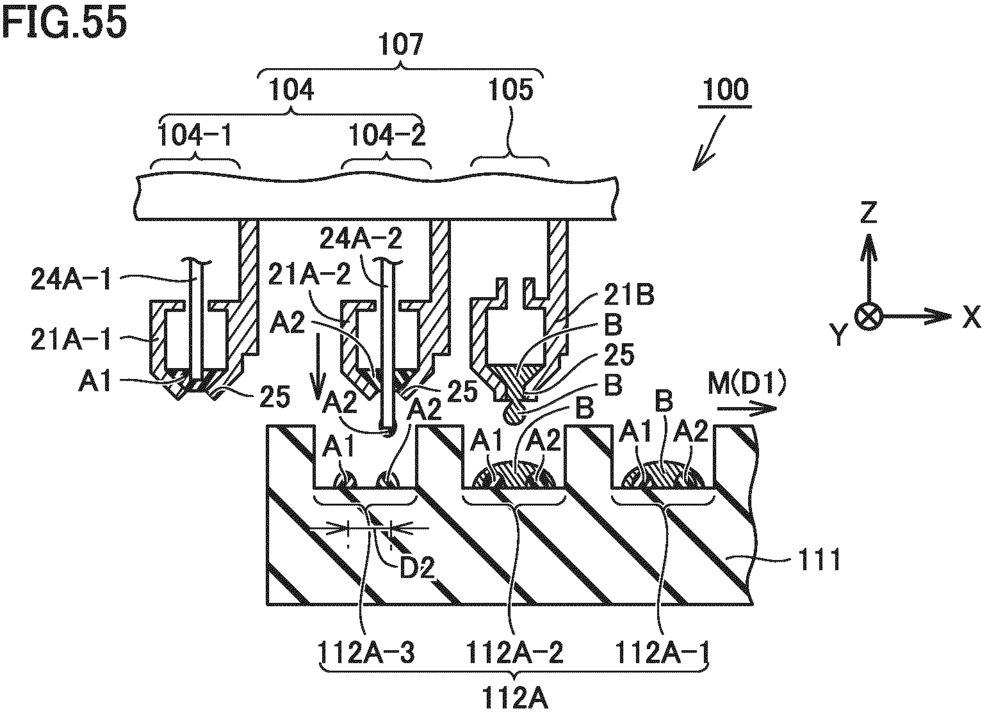
107
104
105
100
104-1
104-2
24A-2
24A-1
21A-2
21B
21A-1
A2
B
25
A1
25
A2
A2
25
B
M(D1)
B
B
A1
A2
A1
A2
A1
A2
D2
111
112A-3
112A-2
112A-1
112A
Z
Y
X

FIG.56

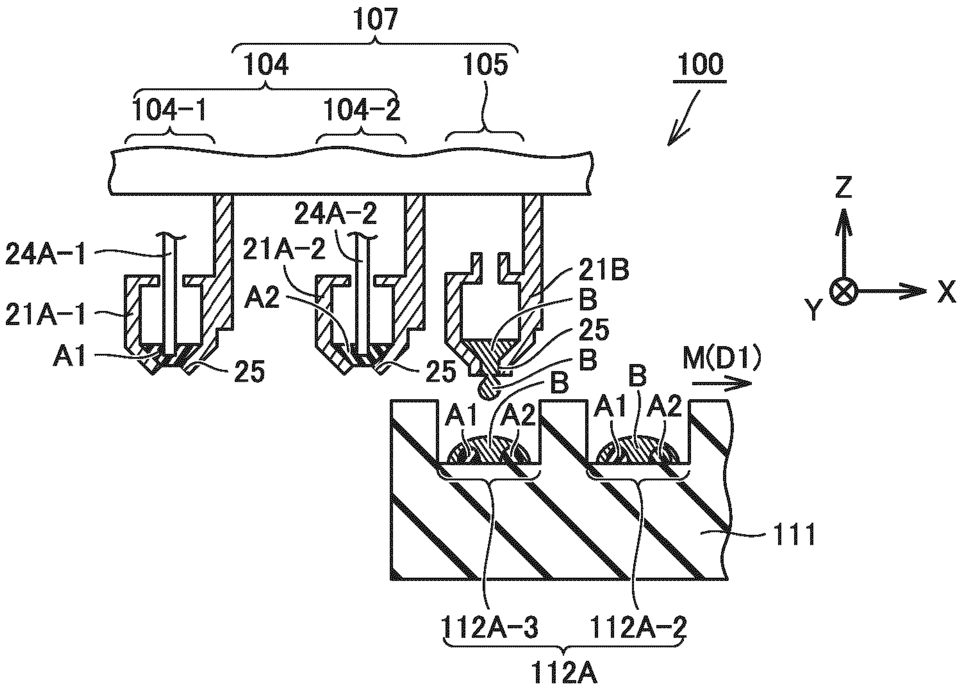
107
104
105
100
104-1
104-2
24A-2
24A-1
21A-2
21B
21A-1
A2
B
25
A1
25
25
B
M(D1)
B
B
A1
A2
A1
A2
111
112A-3
112A-2
112A
Z
Y
X

FIG.57

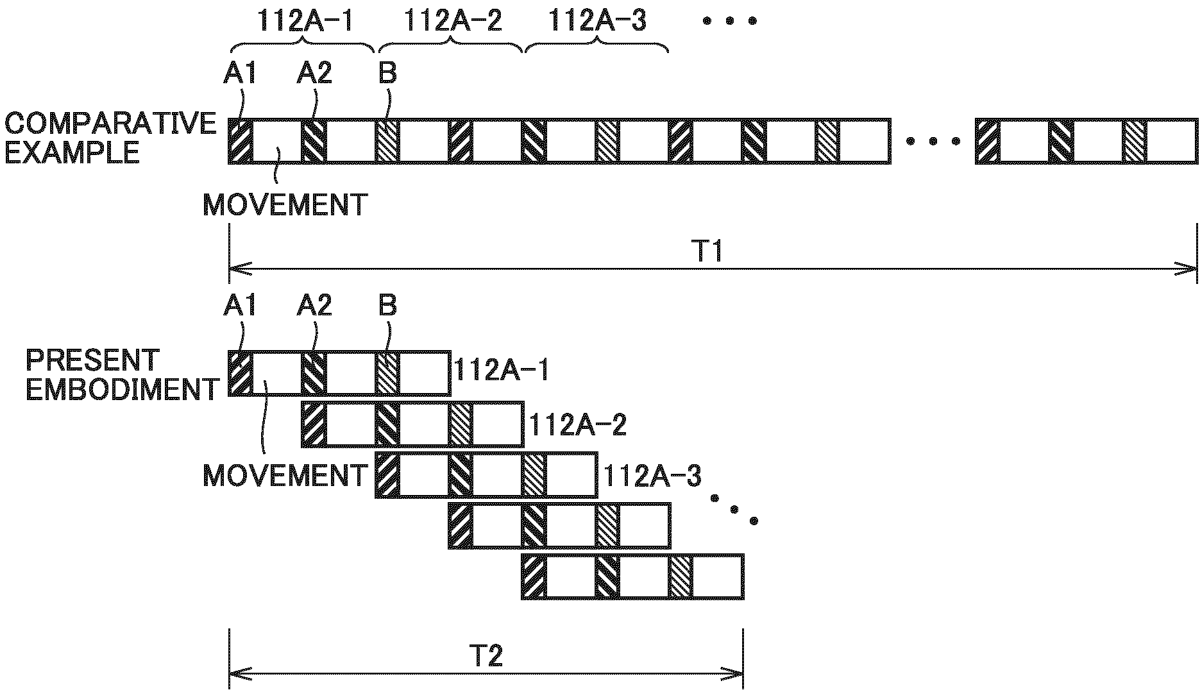
112A-1
112A-2
112A-3
A1
A2
B
COMPARATIVE EXAMPLE
MOVEMENT
T1
A1
A2
B
PRESENT EMBODIMENT
112A-1
112A-2
MOVEMENT
112A-3
T2

FIG.58

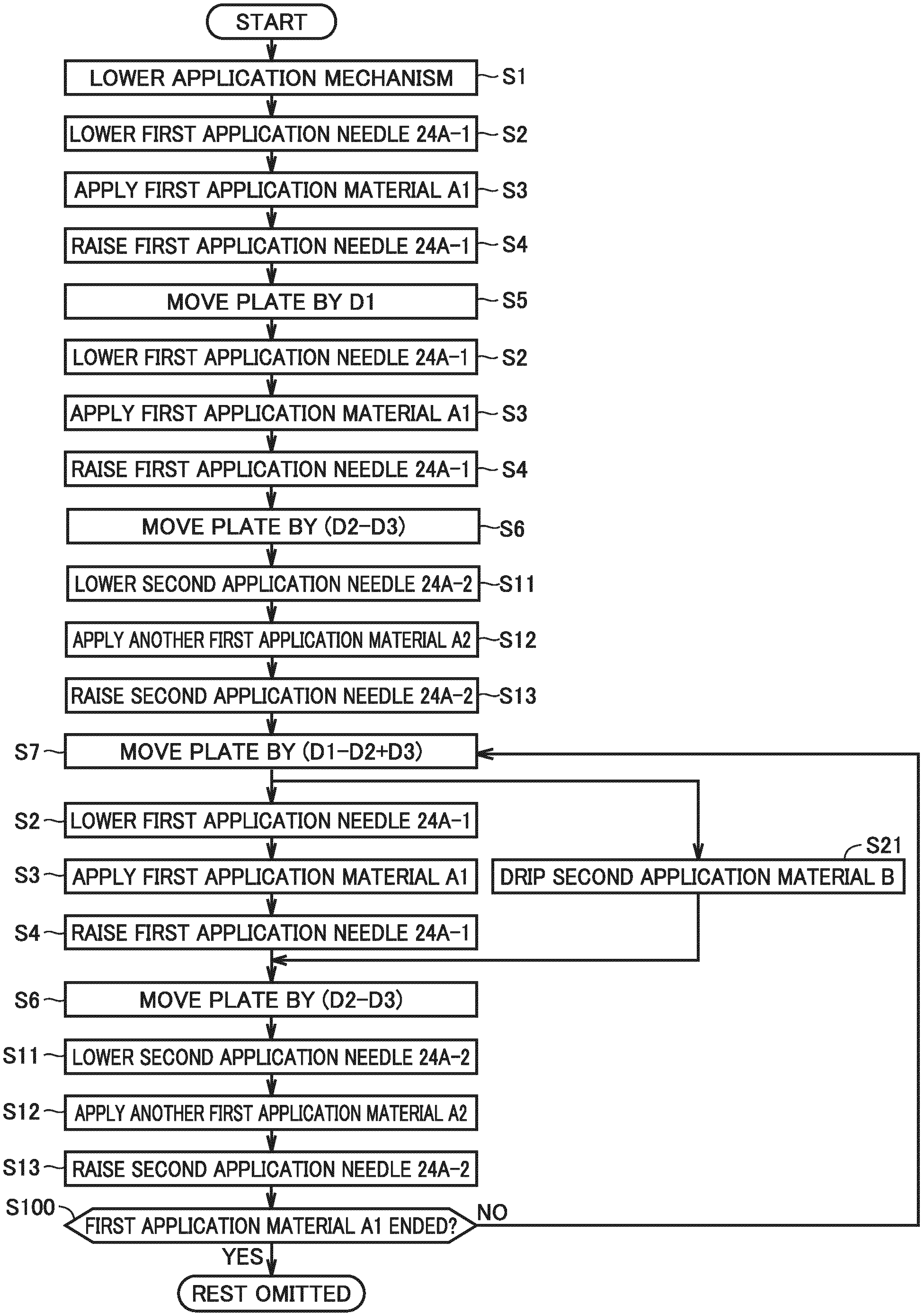
START
LOWER APPLICATION MECHANISM
S1
LOWER FIRST APPLICATION NEEDLE 24A-1
S2
APPLY FIRST APPLICATION MATERIAL A1
S3
RAISE FIRST APPLICATION NEEDLE 24A-1
S4
MOVE PLATE BY D1
S5
LOWER FIRST APPLICATION NEEDLE 24A-1
S2
APPLY FIRST APPLICATION MATERIAL A1
S3
RAISE FIRST APPLICATION NEEDLE 24A-1
S4
MOVE PLATE BY (D2-D3)
S6
LOWER SECOND APPLICATION NEEDLE 24A-2
S11
APPLY ANOTHER FIRST APPLICATION MATERIAL A2
S12
RAISE SECOND APPLICATION NEEDLE 24A-2
S13
S7
MOVE PLATE BY (D1-D2+D3)
S2
LOWER FIRST APPLICATION NEEDLE 24A-1
S21
DRIP SECOND APPLICATION MATERIAL B
S3
APPLY FIRST APPLICATION MATERIAL A1
S4
RAISE FIRST APPLICATION NEEDLE 24A-1
S6
MOVE PLATE BY (D2-D3)
S11
LOWER SECOND APPLICATION NEEDLE 24A-2
S12
APPLY ANOTHER FIRST APPLICATION MATERIAL A2
S13
RAISE SECOND APPLICATION NEEDLE 24A-2
S100
FIRST APPLICATION MATERIAL A1 ENDED?
NO
YES
REST OMITTED

FIG.59

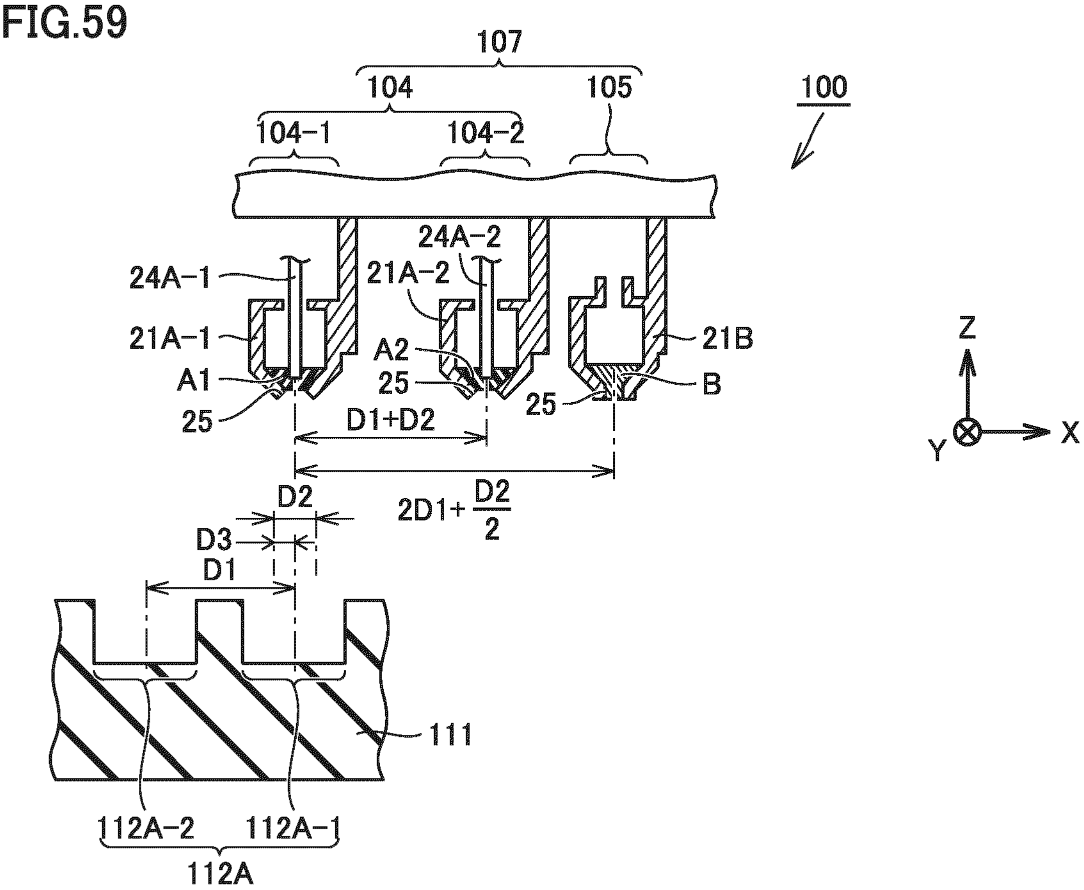
107
104
105
100
104-1
104-2
24A-2
24A-1
21A-2
21A-1
A2
21B
A1
25
25
B
25
Z
D1+D2
Y
X
2D1+ D2/2
D2
D3
D1
111
112A-2
112A-1
112A

FIG.60

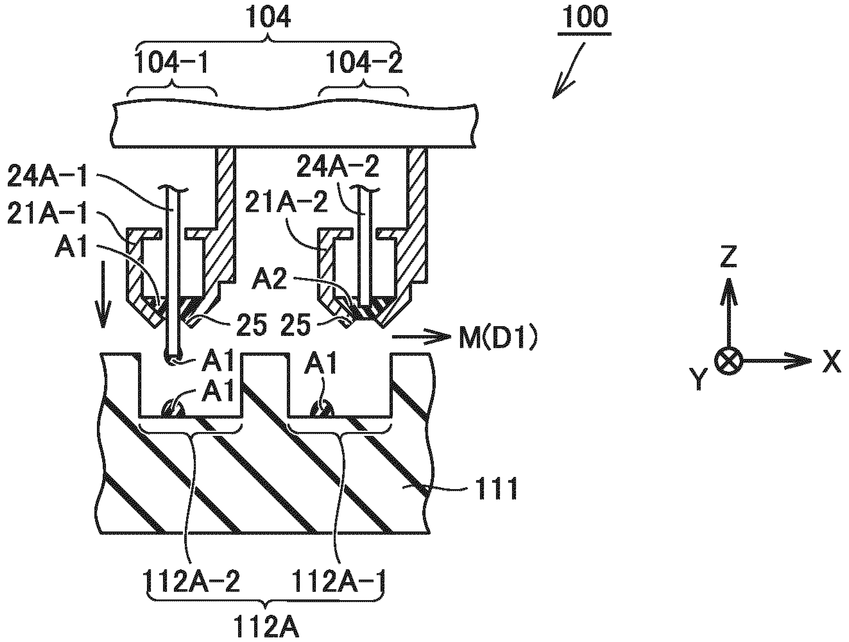
104
104-1
104-2
100
24A-1
24A-2
21A-1
21A-2
A1
A2
25
25
M(D1)
A1
A1
A1
Z
Y
X
111
112A-2
112A-1
112A

FIG.61

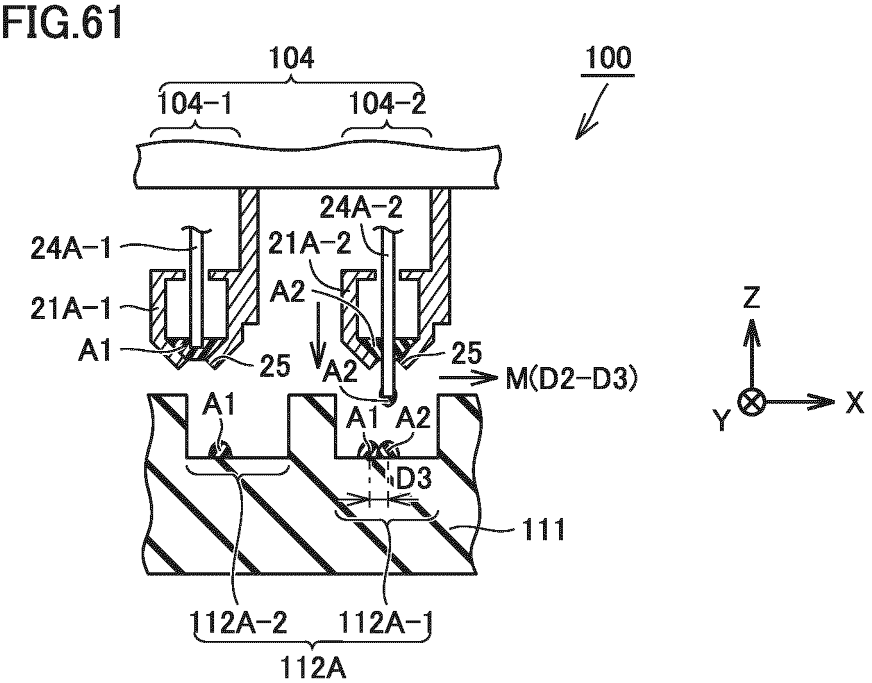
104
104-1
104-2
100
24A-2
24A-1
21A-2
A2
21A-1
A1
25
25
A2
M(D2-D3)
A1
A1
A2
D3
Z
Y
X
111
112A-2
112A-1
112A

FIG.62

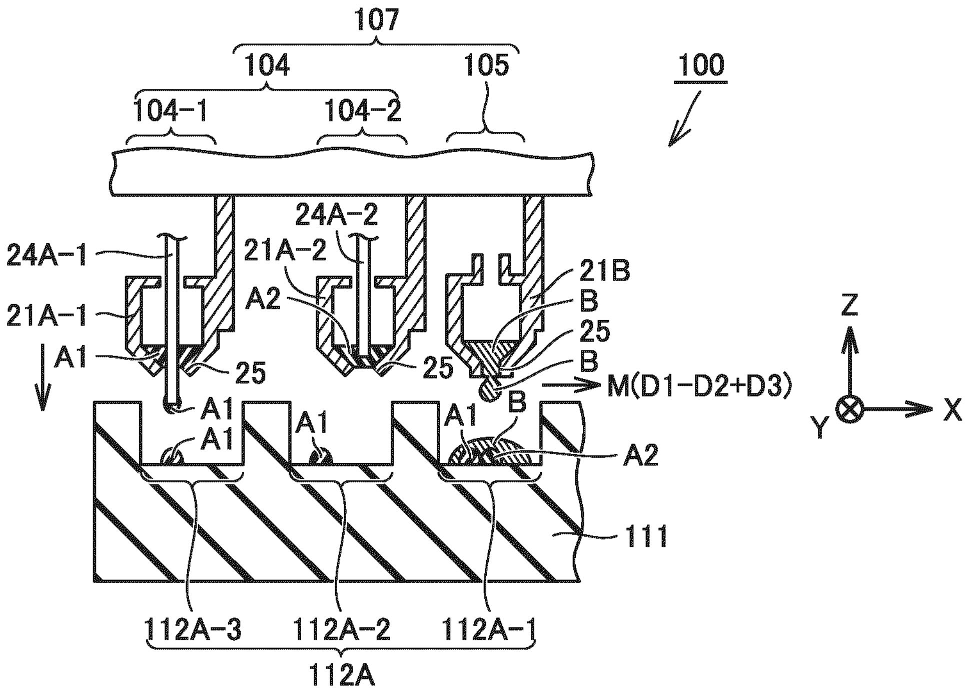
107
104
105
100
104-1
104-2
24A-2
24A-1
21A-2
21B
A2
B
21A-1
25
Z
A1
25
25
B
M(D1-D2+D3)
A1
B
Y
X
A1
A1
A1
A2
111
112A-3
112A-2
112A-1
112A

FIG.63

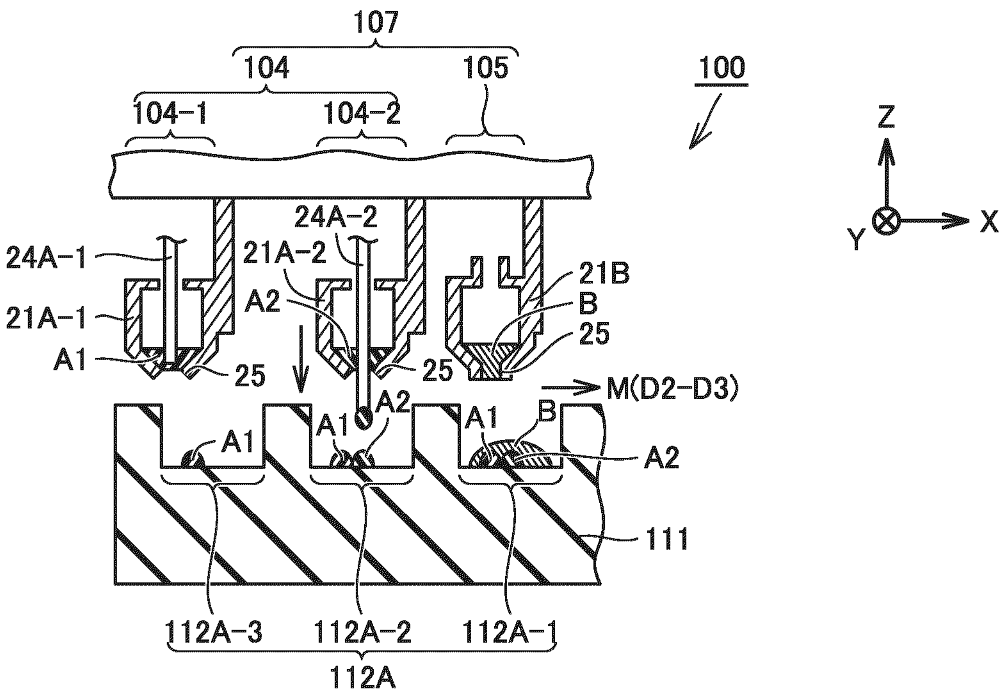
107
104
105
100
104-1
104-2
Z
24A-2
24A-1
21A-2
Y
X
21B
A2
B
21A-1
25
A1
25
25
M(D2-D3)
A2
A1
A1
B
A1
A2
111
112A-3
112A-2
112A-1
112A

FIG.64

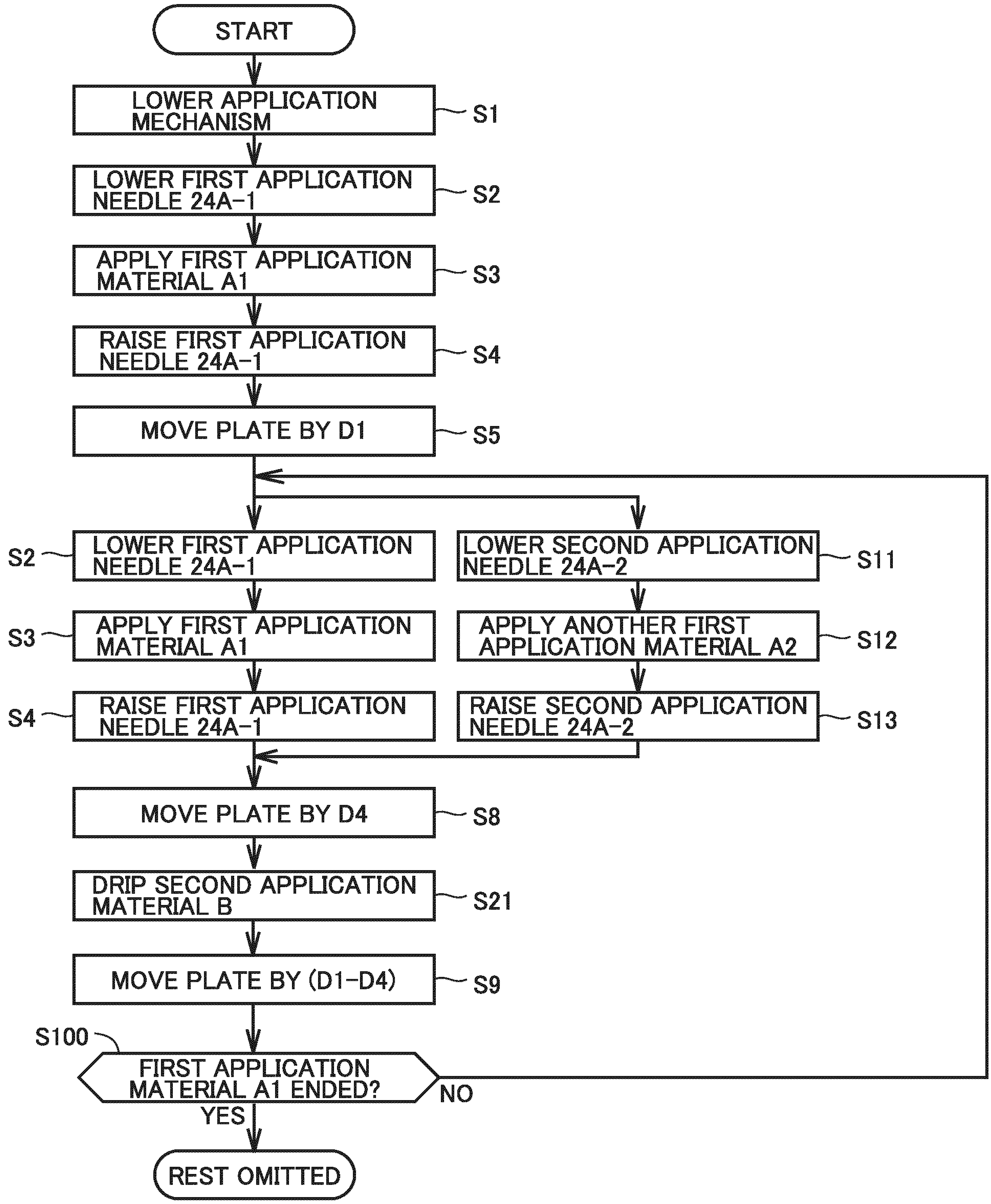
START
LOWER APPLICATION MECHANISM
S1
LOWER FIRST APPLICATION NEEDLE 24A-1
S2
APPLY FIRST APPLICATION MATERIAL A1
S3
RAISE FIRST APPLICATION NEEDLE 24A-1
S4
MOVE PLATE BY D1
S5
S2
LOWER FIRST APPLICATION NEEDLE 24A-1
LOWER SECOND APPLICATION NEEDLE 24A-2
S11
S3
APPLY FIRST APPLICATION MATERIAL A1
APPLY ANOTHER FIRST APPLICATION MATERIAL A2
S12
S4
RAISE FIRST APPLICATION NEEDLE 24A-1
RAISE SECOND APPLICATION NEEDLE 24A-2
S13
MOVE PLATE BY D4
S8
DRIP SECOND APPLICATION MATERIAL B
S21
MOVE PLATE BY (D1-D4)
S9
S100
FIRST APPLICATION MATERIAL A1 ENDED?
NO
YES
REST OMITTED

FIG.65

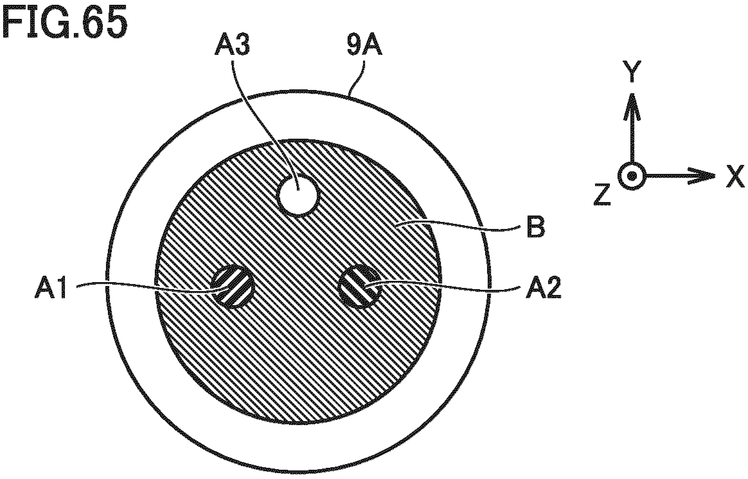
A3
9A
Y
Z
X
B
A1
A2

FIG.66

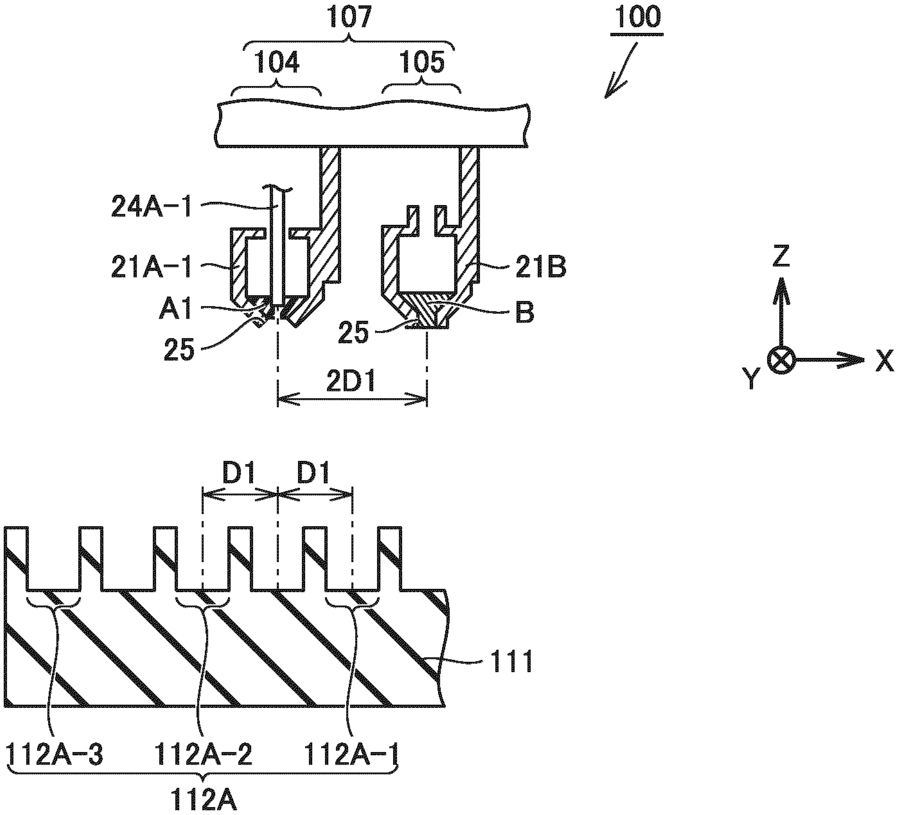
107
100
104
105
24A-1
21A-1
A1
25
21B
B
25
2D1
Z
Y
X
D1
D1
111
112A-3
112A-2
112A-1
112A

FIG.67

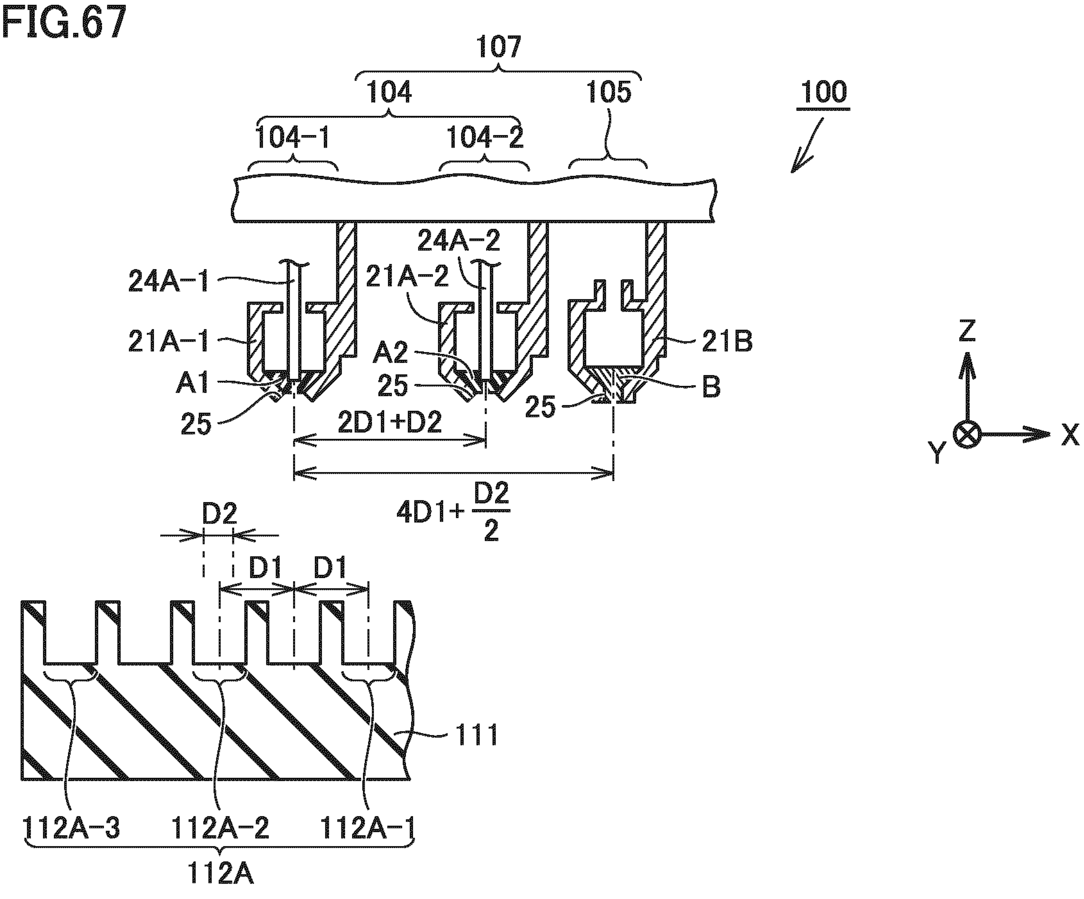
107
104
105
100
104-1
104-2
24A-2
24A-1
21A-2
21A-1
A2
21B
A1
25
25
B
25
2D1+D2
25
4D1+ D2/2
Z
Y
X
D2
D1
D1
111
112A-3
112A-2
112A-1
112A

FIG.68

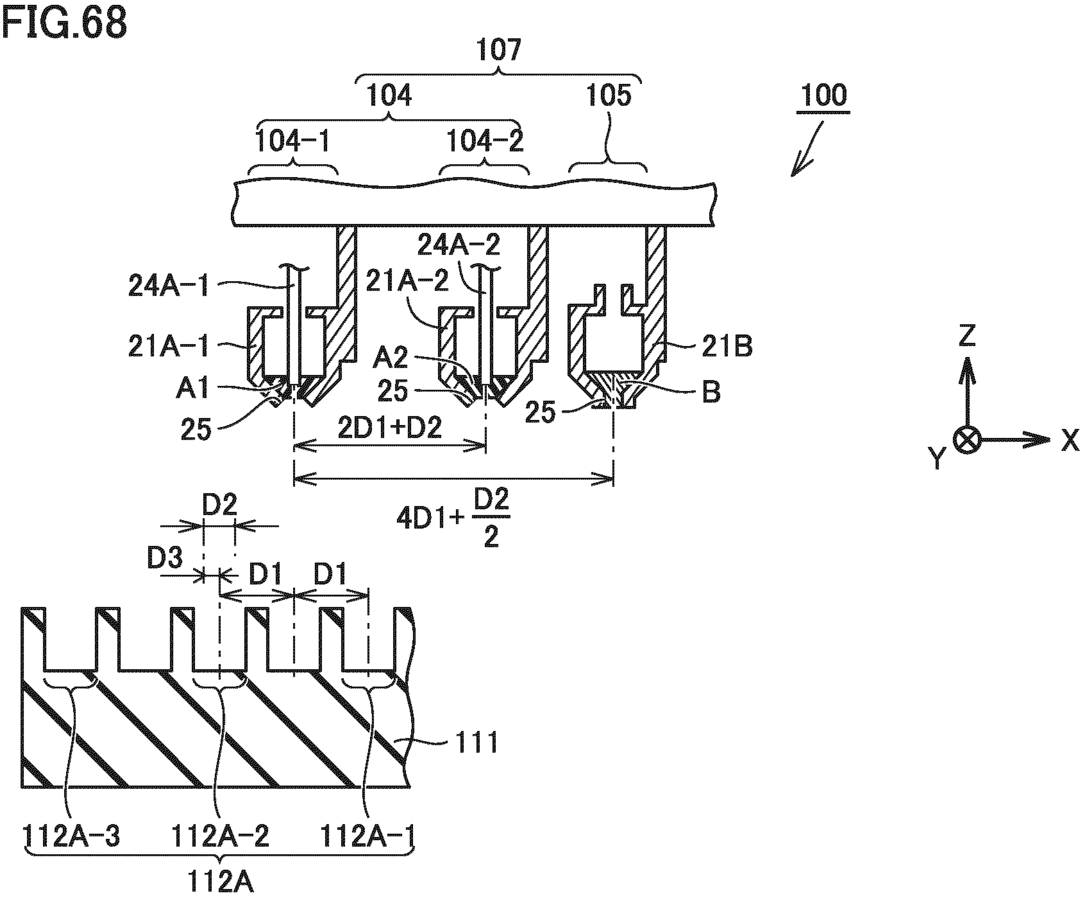
107
104
105
100
104-1
104-2
24A-2
24A-1
21A-2
21A-1
A2
21B
A1
25
25
B
25
25
2D1+D2
Z
Y
X
4D1+ D2/2
D2
D3
D1
D1
111
112A-3
112A-2
112A-1
112A

**INTERNATIONAL SEARCH REPORT**

International application No. **PCT/JP2023/032319**

**A. CLASSIFICATION OF SUBJECT MATTER**

***C12N 5/071***(2010.01)i; ***B05C 1/02***(2006.01)i; ***B05C 5/00***(2006.01)i; ***B05C 11/10***(2006.01)i; ***B05D 1/26***(2006.01)i; ***B05D 1/36***(2006.01)i; ***B05D 3/00***(2006.01)i; ***B05D 7/00***(2006.01)i; ***C12N 5/077***(2010.01)i; ***C12N 5/10***(2006.01)i; ***C12N 15/09***(2006.01)i

FI: C12N5/071; B05D1/36 Z; C12N5/10; C12N15/09 Z; B05C1/02 101; B05C5/00 101; B05C1/02 104; B05C11/10; B05D1/26 Z; B05D3/00 C; B05D7/00 N; C12N5/077

According to International Patent Classification (IPC) or to both national classification and IPC

**B. FIELDS SEARCHED**

Minimum documentation searched (classification system followed by classification symbols)

C12N5/071; B05C1/02; B05C5/00; B05C11/10; B05D1/26; B05D1/36; B05D3/00; B05D7/00; C12N5/077; C12N5/10; C12N15/09

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/179929 A1 (OSAKA UNIVERSITY) 10 September 2020 (2020-09-10) fig. 18, 19, 32, 33, paragraphs [0231]-[0254] | 21-30 |
| A | | 1-20 |
| X | JP 2019-209301 A (OSAKA UNIVERSITY) 12 December 2019 (2019-12-12) paragraphs [0013], [0057]-[0067], fig. 1 | 21-30 |

☐ Further documents are listed in the continuation of Box C. ☑ See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **01 November 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/032319**

| Patent document cited in search report | Publication date (day/month/year) | Patent family member(s) | Publication date (day/month/year) |
|---|---|---|---|
| WO 2020/179929 A1 | 10 September 2020 | EP 3936238 A1<br>fig. 18, 19, 32, 33, paragraphs [0246]-[0268]<br>CN 113474092 A | |
| JP 2019-209301 A | 12 December 2019 | WO 2019/235513 A1<br>paragraphs [0014], [0073]-[0084], fig. 1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020178612 A
- WO 2019088224 A
- JP 2020141599 A